(19)

Europäisches
Patentamt

European
Patent Office

Office européen
des brevets

(11) EP 4 273 249 A2

(12) **EUROPEAN PATENT APPLICATION**

(43) Date of publication:
08.11.2023 Bulletin 2023/45

(51) International Patent Classification (IPC):
**C12N 15/63** (2006.01)

(21) Application number: **23184251.9**

(22) Date of filing: **07.07.2023**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Novozymes A/S
2880 Bagsvaerd (DK)**

(72) Inventor: **Kallehauge, Thomas Krogh
2880 Bagsvaerd (DK)**

(74) Representative: **NZ EPO Representatives
Krogshoejvej 36
2880 Bagsvaerd (DK)**

Remarks:
The complete document including Reference
Table(s) and the Sequence Listing(s) can be
downloaded from the EPO website

(54) **IMPROVED EXPRESSION OF RECOMBINANT PROTEINS**

(57) The present invention relates to signal peptides, signal peptide-linkers, fusion polypeptides comprising signal peptide-linker, and polynucleotides encoding the signal peptides, signal peptide-linkers, and fusion polypeptides, and to nucleic acid constructs, vectors, and host cells comprising the polynucleotides as well as methods of producing the fusion polypeptides, and methods for increasing secretion of a polypeptide of interest.

EP 4 273 249 A2

Figure 1

**Description**

**Reference to a Sequence Listing**

This application contains a Sequence Listing in computer readable form, which is incorporated herein by reference.

**Field of the Invention**

The present invention relates to signal peptides, signal peptide-linkers, fusion polypeptides comprising signal peptide-linker, and polynucleotides encoding the signal peptides, signal peptide-linkers, and fusion polypeptides, and to nucleic acid constructs, vectors, and host cells comprising the polynucleotides as well as methods of producing the fusion polypeptides, and methods for increasing secretion of a polypeptide of interest.

**Description of the Related Art**

Product development in industrial biotechnology includes a continuous challenge to increase enzyme yields at large scale to reduce costs. Two major approaches have been used for this purpose in the last decades. The first one is based on classical mutagenesis and screening. Here, the specific genetic modification is not predefined, and the main requirement is a screening assay that is sensitive to detect increments in yield. High-throughput screening enables large numbers of mutants to be screened in search for the desired phenotype, i.e., higher enzyme yields. The second approach includes numerous strategies ranging from the use of stronger promoters, and multi-copy strains to ensure high expression of the gene of interest, to the use of codon-optimized gene sequences to aid translation. However, high-level production of a given protein may in turn trigger several bottlenecks in the cellular machinery for secretion of the enzyme of interest into the medium, emphasizing the need for further optimization strategies.

For cells to function, proteins must be targeted to their proper locations. To direct a protein (e.g., to an intracellular compartment or organelle, or for secretion), organisms often encode instructions in a leading short peptide sequence (typically 15-30 amino acids), called a signal peptide (SP). SPs are present in the amino terminus (N-terminus) of many newly synthesized polypeptides that target these polypeptides into or across cellular membranes, thereby aiding maturation and secretion. The amino acid sequence of the SP influences secretion efficiency and thereby the yield of the polypeptide manufacturing process. SPs have been engineered for a variety of industrial and therapeutic purposes, including increased export for recombinant protein production and increasing the therapeutic levels of proteins secreted from industrial production hosts.

A large degree of redundancy in the amino acid sequence of SPs makes it difficult to predict the efficiency of any given SP for production of enzymes at industrial scale. Hence, SP selection is an important step for manufacturing of recombinant proteins, but the optimal combination of signal peptide and mature protein is very context dependent and not easy to predict.

Also, the selection of a SP sequence for the expression of a certain class of molecule (e.g. enzymes, such as an amylase enzyme) is often limited to SP sequences derived from this certain class of molecule, such as using an amylase SP-sequence from a first organism for the expression of an amylase or amylase variant from the same organism or from a second organism.

During the maturation process, the SP is typically cleaved off by a signal peptidase (SPase), resulting in a matured protein of interest. The SPase cleavage site is located between the SP sequence and the N-terminal end of the amino acid sequence of the protein of interest. SPases have been identified in all orders of life. In eukaryotes, SPase systems are located in the endoplasmic reticulum (ER), the mitochondria, and chloroplasts. In prokaryotes, SPases are classified into three groups: SPase I, II, and IV. SPase II and IV are required for cleaving signal peptides from lipoproteins and prepilin proteins, respectively. While researchers have attempted to generalize the understanding of SP-protein pairs by developing general SP design guidelines, those guidelines are heuristics at best and are limited to SP sequence designs upstream of the cleavage site for the signal peptidases.

The object of the present invention is the provision of host cells with increased secretion of recombinant protein.

**Summary of the Invention**

The inventors of the present invention surprisingly found that polypeptide secretion can be enhanced by providing an additional linker (herein named "signal peptide linker", or "SP-linker") located between the signal peptide and the polypeptide of interest. The SP-linker is comprising or consisting of the N-terminal amino acids of a donor polypeptide which is heterologous to the polypeptide of interest. Without being bound by theory, it is presently thought that the SP-linker facilitates more effective cleavage of the signal peptide during the maturation process of the polypeptide of interest.

Thereby, an increased amount of polypeptide of interest is secreted by the host cell relative to the secretion of the same polypeptide of interest only comprising a signal peptide but not comprising a SP-linker.

Suitable combinations of signal peptides and SP-linkers can be identified using the herein disclosed methods. During the screening for optimal combinations of signal peptides and SP-linkers, novel signal peptide sequences were identified which show promising expression yields.

As can be seen throughout the examples, expression of recombinant protein is increased up to 230% when a SP-linker is fused between the signal peptide and the polypeptide of interest. Furthermore surprisingly, the inventors observed that a SP-linker can maintain increased protein expression even after being fused with other signal peptides.

Since protein secretion and signal peptide cleavage are highly conversed mechanisms throughout the majority of organisms, the invention may be suitable for all organisms which are capable of protein secretion and signal peptide cleavage. The present invention may also be suitable for optimized expression of any secreted polypeptide, since a SP - SP-linker construct may be identified for any secreted polypeptide, or be transferred from one polypeptide of interest to another polypeptide of interest.

In a 1st aspect 1A, the present invention relates to a nucleic acid construct comprising or consisting of:

a) a first polynucleotide encoding a signal peptide (SP),
b) a second polynucleotide located downstream of the first polynucleotide and encoding a SP-linker consisting of 2 to 15 amino acids, wherein the SP-linker amino acid sequence consists of the N-terminal amino acid sequence of a second donor polypeptide; and
at least one third polynucleotide located downstream of the second polynucleotide and encoding a polypeptide of interest;

wherein the first polynucleotide and the second polynucleotide are heterologous to the third polynucleotide, and wherein the first polynucleotide, the second polynucleotide, and the third polynucleotide are operably linked in translational fusion.

In a 1st aspect 1B, the invention also relates to a nucleic acid construct comprising:

a) a first polynucleotide encoding a signal peptide from a bacterial yckD polypeptide; and
b) a second polynucleotide encoding a polypeptide of interest;

wherein the first polynucleotide and the second polynucleotide are operably linked in translational fusion.

In a 1st aspect 1C, the invention also relates to a nucleic acid construct comprising:

a) a first polynucleotide encoding a signal peptide from a fungal prolyl dipeptidyl peptidase; and

b) a second polynucleotide encoding a polypeptide of interest;

wherein the first polynucleotide and the second polynucleotide are operably linked in translational fusion.

In a 2nd aspect, the invention relates to an expression vector comprising a nucleic acid construct according to the 1st aspect 1A , the 1st aspect 1B, or the 1st aspect 1C.

In a 3rd aspect 3A, the invention relates to a host cell producing a polypeptide of interest and comprising in its genome:

a) a nucleic acid construct according to the 1st aspect 1A; and/or
b) an expression vector comprising a nucleic acid construct according to the 1st aspect 1A.

In a 3rd aspect 3B, the invention relates to a bacterial host cell producing a polypeptide of interest and comprising in its genome:

a) a nucleic acid construct according to the 1st aspect 1B; and/or
b) an expression vector comprising ca nucleic acid construct according to the 1st aspect 1B.

In a 3rd aspect 3C, the invention relates to a fungal host cell producing a polypeptide of interest and comprising in its genome:

a) a nucleic acid construct according to the 1st aspect 1C; and/or
b) an expression vector comprising ca nucleic acid construct according to the 1st aspect 1C.

In a 4th aspect, the invention relates to a method of producing a polypeptide of interest, the method comprising:

a) cultivating a host cell according to the 3rd aspects 3A and/or 3B and/or 3C under conditions conducive for production of the polypeptide of interest; and optionally

b) recovering the polypeptide of interest.

In a 5th aspect, the present invention relates to a transgenic plant, plant part or plant cell transformed with the nucleic acid construct according to the 1st aspects.

In a 6th aspect, the invention relates to a method of producing a polypeptide of interest, comprising cultivating the transgenic plant or plant cell of the 5th aspect under conditions conducive for production of the polypeptide.

In a 7th aspect, the invention relates to a fusion polypeptide, comprising a polypeptide of interest and a SP-linker consisting of 2 to 15 amino acids, wherein the SP-linker is located at the N-terminal end of the polypeptide of interest and is heterologous to the polypeptide of interest.

In an 8th aspect the invention relates to an extended fusion polypeptide comprising the fusion polypeptide of the 7th aspect and a second polypeptide of interest.

In a 9th aspect the invention relates to a hybrid polypeptide comprising the fusion polypeptide of the 7th aspect.

In a 10th aspect 10A, the invention also relates to a polypeptide, comprising a polypeptide of interest, and a signal peptide derived from a bacterial yckD polypeptide.

In a 10th aspect 10B, the invention relates to a polypeptide, comprising a polypeptide of interest, and a signal peptide derived from a fungal prolyl dipeptidyl peptidase.

In an 11th aspect 11A, the invention relates to a fusion polypeptide comprising the polypeptide of the 10th aspect 10A and a second polypeptide of interest.

In an 11th aspect 11B, the invention relates to a fusion polypeptide comprising the polypeptide of the 10th aspect 10B and a second polypeptide of interest.

In a 12th aspect 12A, the invention relates to a hybrid polypeptide comprising the polypeptide of the 10th aspect 10B.

In a 12th aspect 12B, the invention relates to a hybrid polypeptide comprising the polypeptide of the 10th aspect 10B.

In a 13th aspect, the invention relates to a method for increasing secretion of a polypeptide of interest by a host cell, the method comprising the steps of

a) **providing** a plurality of first polynucleotide sequences, each first polynucleotide encoding a signal peptide;

b) **expressing** a first polypeptide with a host cell, wherein the polynucleotide encoding the first polypeptide is linked in translational fusion with a first polynucleotide;

c) **measuring** the amount of first polypeptide for each first polynucleotide sequence;

d) **ranking** each first polynucleotide based on the amount of secreted first polypeptide;

e) **selecting** one or more of the ranked first polynucleotides;

f) **fusing** the selected one or more first polynucleotide with a second polynucleotide encoding a SP-linker comprising or consisting of 2-15 N-terminal amino acids from the secreted first polypeptide expressed in step b), wherein the second polynucleotide is located downstream of the first polynucleotide, and

g) **expressing,** in a host cell, the polypeptide of interest in translational fusion with the fusion construct generated in step f), wherein the SP-linker is located upstream of the polypeptide of interest.

**Brief Description of the Drawings**

**Figure 1** shows a schematic overview of the *Bacillus* expression cassettes and fusion proteins according to the invention.

**Figure 2** shows amylase expression with a signal peptide (BT11054), and amylase expression with a signal peptide and SP-linker (BT11055).

**Figure 3** shows amylase expression with a signal peptide (BT11078), and amylase expression with a signal peptide and SP-linker (BT11079).

**Figure 4** shows amylase expression across the strains comprising a signal peptide only (BT11054 and BT1 1078), and the strains comprising a signal peptide with a SP-linker (BT11055 and BT11079)

**Figure 5** shows protease expression with a the *yckD* signal peptide (BT11063) compared to protease expression with the *aprL* signal peptide (BT11032).

**Figure 6** shows varying protein expression depending on SP-linker length.

**Figure 7** shows amylase expression with and without SP-linker.
**Figure 8** shows amylase expression using the same SP-linker in combination with different SP sequences.
**Figure 9** shows a schematic overview of the *Aspergillus* expression cassettes and fusion proteins according to the invention.
**Figure 10** shows phytase expression with a signal peptide (AT6552, AT6553), and phytase expression with a signal peptide and SP-linker (AT6502, AT6503).
**Figure 11** shows phytase expression with a signal peptide (AT6552, AT6553), and phytase expression with a signal peptide and SP-linker (AT6502, AT6503).

## Definitions

In accordance with this detailed description, the following definitions apply. Note that the singular forms "a," "an," and "the" include plural references unless the context clearly dictates otherwise.

Unless defined otherwise or clearly indicated by context, all technical and scientific terms used herein have the same meaning as commonly understood by one of ordinary skill in the art to which this invention belongs.

**Protease:** The term "protease", "protease 1", or "protease 2" means a serine endopeptidase, such as a subtilisin (EC 3.4.21.62), that catalyzes the hydrolysis of proteins with broad specificity for peptide bonds. For purposes of the present invention, protease activity is determined according to the procedure described in the Examples. In one aspect, the protease polypeptides or the fusion protease polypeptides of the present invention have at least 20%, *e.g.*, at least 40%, at least 50%, at least 60%, at least 70%, at least 80%, at least 90%, at least 95%, or at least 100% of the protease activity of the mature polypeptide with SEQ ID NO: 16 (protease 1) or SEQ ID NO: 17 (protease 2).

**Amylase:** The term "amylase" means a glycosylase (EC 3.2), more specifically a glycosidase (EC 3.2.1) or an alpha-amylase, such as an 1,4- alpha-D-glucan glucano-hydrolase (EC 3.2.1.1), that catalyzes the endohydrolysis of (1→4)-alpha-D-glucosidic linkages in polysaccharides containing three or more (1→4)-alpha-linked D-glucose units. For purposes of the present invention, amylase activity is determined according to the procedure described in the Examples. In one aspect, the amylase polypeptides or the fusion amylase polypeptides of the present invention have at least 20%, e.g., at least 40%, at least 50%, at least 60%, at least 70%, at least 80%, at least 90%, at least 95%, or at least 100% of the amylase activity of the mature polypeptide encoded by SEQ ID NO: 13.

**Additional cleavage site:** The term "additional cleavage site" and/or, when used in relation to the fusion polypeptide of the invention the term "cleavage site", relates to a cleavage site located between the SP-linker and the amino acid sequence of the polypeptide of interest. Additionally or alternatively, when the polypeptide of interest comprises a pro-peptide, the additional cleavage site may be located between the SP-linker and the amino acid sequence of the pro-peptide of the polypeptide of interest. The additional cleavage site allows for removal of the SP-linker from the polypeptide of interest by a maturation process including a peptidase which recognizes the additional cleavage site, and removes the SP-linker by cleavage. A non-limiting example for amino acid sequences of an additional cleavage site and respective peptidase is the cleavage site "Lys-Arg" or "Arg-Arg" cleaved by Kexin (KEX2/ KEXB). The skilled person is aware of further readily available cleavage sites and peptidases which can be inserted between the SP-linker and polypeptide of interest. Examples of cleavage sites include, but are not limited to, the sites disclosed in Martin et al., 2003, J. Ind. Microbiol. Biotechnol. 3: 568-576; Svetina et al., 2000, J. Biotechnol. 76: 245-251; Rasmussen-Wilson et al., 1997, Appl. Environ. Microbiol. 63: 3488-3493; Ward et al., 1995, Biotechnology 13: 498-503; and Contreras et al., 1991, Biotechnology 9: 378-381; Eaton et al., 1986, Biochemistry 25: 505-512; Collins-Racie et al., 1995, Biotechnology 13: 982-987; Carter et al., 1989, Proteins: Structure, Function, and Genetics 6: 240-248; and Stevens, 2003, Drug Discovery World 4: 35-48.

**Biological function**: The term "biological function" means a biological function carried out by a polypeptide, or associated with a polypeptide. The function can be described as enzymatic activity, enzymatic specificity, structural function, metabolic function, e.g. catalysis of a reaction, degradation of a substrate, or binding of a substrate. Non-limiting examples for polypeptides having different biological functions are polypeptides catalyzing different reactions, binding to different substrates, having less than 60% amino acid sequence identity, or having at least 10% kDa difference in molecular weight.

**Catalytic domain:** The term "catalytic domain" means the region of an enzyme containing the catalytic machinery of the enzyme.

**cDNA:** The term "cDNA" means a DNA molecule that can be prepared by reverse transcription from a mature, spliced, mRNA molecule obtained from a eukaryotic or prokaryotic cell. cDNA lacks intron sequences that may be present in the corresponding genomic DNA. The initial, primary RNA transcript is a precursor to mRNA that is processed through a series of steps, including splicing, before appearing as mature spliced mRNA.

**Coding sequence**: The term "coding sequence" means a polynucleotide, which directly specifies the amino acid sequence of a polypeptide. The boundaries of the coding sequence are generally determined by an open reading frame, which begins with a start codon, such as ATG, GTG, or TTG, and ends with a stop codon, such as TAA, TAG, or TGA. The coding sequence may be a genomic DNA, cDNA, synthetic DNA, or a combination thereof.

**Control sequences**: The term "control sequences" means nucleic acid sequences involved in regulation of expression of a polynucleotide in a specific organism or *in vitro.* Each control sequence may be native (*i.e.,* from the same gene) or heterologous (*i.e.,* from a different gene) to the polynucleotide encoding the polypeptide, and native or heterologous to each other. Such control sequences include, but are not limited to leader, polyadenylation, prepropeptide, propeptide, signal peptide, promoter, terminator, enhancer, and transcription or translation initiator and terminator sequences. At a minimum, the control sequences include a promoter, and transcriptional and translational stop signals. The control sequences may be provided with linkers for the purpose of introducing specific restriction sites facilitating ligation of the control sequences with the coding region of the polynucleotide encoding a polypeptide.

**Expression**: The term "expression" means any step involved in the production of a polypeptide including, but not limited to, transcription, post-transcriptional modification, translation, post-translational modification, and secretion.

**Expression vector**: An "expression vector" refers to a linear or circular DNA construct comprising a DNA sequence encoding a polypeptide, which coding sequence is operably linked to a suitable control sequence capable of effecting expression of the DNA in a suitable host. Such control sequences may include a promoter to effect transcription, an optional operator sequence to control transcription, a sequence encoding suitable ribosome binding sites on the mRNA, enhancers and sequences which control termination of transcription and translation.

**Extension:** The term "extension" means an addition of one or more amino acids to the amino and/or carboxyl terminus of a polypeptide, e.g. the SP-linker or the polypeptide of interest. When the "extended" polypeptide is the polypeptide of interest, the extended polypeptide has substantially the same enzyme activity as the non-extended polypeptide. When the "extended" polypeptide is the SP-linker, the extended SP-linker contributes to increased secretion of the polypeptide of interest, compared to the secretion of the polypeptide of interest without the SP-linker.

**First polynucleotide:** The term "first polynucleotide" means a polynucleotide encoding a signal peptide. According to the present invention, the first polynucleotide is located upstream (at the 5' end) of the second polynucleotide encoding the SP-linker. The signal peptide encoded by the first polynucleotide comprises a signal peptidase cleavage site located at the C-terminal end of the signal peptide. The first polynucleotide is isolated or derived from a native polynucleotide encoding a signal peptide operably linked in translational fusion with a first donor polypeptide. Thus, the first polynucleotide can be cloned from the gene encoding the first donor polypeptide, to generate a recombinant gene where said signal peptide is operably linked in translational fusion with a polypeptide of interest, and optionally with a SP-linker.

**First donor polypeptide:** The term "first donor" or "first donor polypeptide" means a polypeptide which in nature is operably linked in translational fusion with the signal peptide encoded by the first polynucleotide. Typically, the first donor polypeptide is a secreted polypeptide, which in its mature form does no longer comprise the signal peptide, since the signal peptide is cleaved off during the maturation process, leaving a mature first donor polypeptide. For the purpose of the present invention, the first polynucleotide is identical to or derived from the polynucleotide sequence encoding the native signal peptide of the first donor polypeptide.

**Fragment:** With reference to a SP-linker, the term "fragment" means a SP-linker polypeptide having one or more amino acids absent from the amino and/or carboxyl terminus of the SP-linker, wherein the fragment results in increased secretion of the polypeptide of interest relative to the secretion of the polypeptide of interest expressed only with a signal peptide and without a SP-linker. With reference to the fusion polypeptide of the invention, the term "fragment" means a fusion polypeptide having one or more amino acids absent from the amino and/or carboxyl terminus of the mature fusion polypeptide, wherein the fragment has substantially the same enzyme activity when compared to the enzyme activity of non-fragmented fusion polypeptide.

**Fusion polypeptide:** The term "fusion polypeptide" is a polypeptide in which one SP-linker polypeptide is fused at the N-terminus of a polypeptide of interest. A fusion polypeptide is produced by fusing a polynucleotide encoding another polypeptide to a polynucleotide of the present invention, or by fusing two or more polynucleotides of the present invention together. In one aspect of the invention, the fusion polypeptide comprises a polypeptide of interest and a SP-linker consisting of 2 to 15 amino acids, wherein the SP-linker is located at the N-terminal end of the polypeptide of interest and is heterologous to the polypeptide of interest.

Techniques for producing fusion polypeptides are known in the art, and include ligating the coding sequences encoding the polypeptides so that they are in frame and that expression of the fusion polypeptide is under control of the same promoter(s) and terminator. Fusion polypeptides may also be constructed using intein technology in which fusion polypeptides are created post-translationally (Cooper et al., 1993, EMBO J. 12: 2575-2583; Dawson et al., 1994, Science 266: 776-779). A fusion polypeptide can further comprise a cleavage site between the two polypeptides. Upon secretion of the fusion protein, the site is cleaved releasing the two polypeptides. Examples of cleavage sites include, but are not limited to, the sites disclosed in Martin et al., 2003, J. Ind. Microbiol. Biotechnol. 3: 568-576; Svetina et al., 2000, J. Biotechnol. 76: 245-251; Rasmussen-Wilson et al., 1997, Appl. Environ. Microbiol. 63: 3488-3493; Ward et al., 1995, Biotechnology 13: 498-503; and Contreras et al., 1991, Biotechnology 9: 378-381; Eaton et al., 1986, Biochemistry 25: 505-512; Collins-Racie et al., 1995, Biotechnology 13: 982-987; Carter et al., 1989, Proteins: Structure, Function, and Genetics 6: 240-248; and Stevens, 2003, Drug Discovery World 4: 35-48.

**Heterologous:** The term "heterologous" means, with respect to a host cell, that a polypeptide or nucleic acid does not naturally occur in the host cell. The term "heterologous" means, with respect to a polypeptide or nucleic acid, that a control sequence, *e.g.*, promoter, of a polypeptide or nucleic acid is not naturally associated with the polypeptide or nucleic acid, *i.e.*, the control sequence is from a gene other than the gene encoding the mature polypeptide. The term "heterologous" means, with respect to the polypeptide of interest or the third polynucleotide, that an amino acid sequence from a first or second donor polypeptide and the corresponding polynucleotide sequence encoding said amino acid sequence, e.g., a signal peptide sequence or a N-terminal amino acid sequence, is not naturally associated with the polypeptide of interest or the third polynucleotide, *i.e.*, the signal peptide encoded by the first polynucleotide and derived from the first donor, and the SP-linker encoded by the second polynucleotide and derived from the second donor are from a gene/genes other than the gene encoding the polypeptide of interest. A synthetic polynucleotide or synthetic polypeptide, with respect to a naturally and/or non-synthetic polynucleotide or polypeptide, respectively, is considered to be encompassed by the term "heterologous".

**Host Strain or Host Cell:** A "host strain" or "host cell" is an organism into which an expression vector, phage, virus, or other DNA construct, including a polynucleotide encoding a polypeptide of the present invention has been introduced. Exemplary host strains are microorganism cells (e.g., bacteria, filamentous fungi, and yeast) capable of expressing the polypeptide of interest and/or fermenting saccharides. The term "host cell" includes protoplasts created from cells.

**Hybrid polypeptide:** The term "hybrid polypeptide" means a polypeptide comprising domains from two or more polypeptides, e.g., a binding module from one polypeptide and a catalytic domain from another polypeptide. The domains may be fused at the N-terminus or the C-terminus.

**Introduced:** The term "introduced" in the context of inserting a nucleic acid sequence into a cell, means "transfection", "transformation" or "transduction," as known in the art.

**Isolated:** The term "isolated" means a polypeptide, nucleic acid, cell, or other specified material or component that has been separated from at least one other material or component, including but not limited to, other proteins, nucleic acids, cells, etc. An isolated polypeptide, nucleic acid, cell or other material is thus in a form that does not occur in nature. An isolated polypeptide includes, but is not limited to, a culture broth containing the secreted polypeptide expressed in a host cell.

**Mature polypeptide:** The term "mature polypeptide" or "mature polypeptide of interest" means a polypeptide in its mature form following N-terminal and/or C-terminal processing, such as the removal of the signal peptide from the SP-linker by a signal peptidase, resulting in a matured polypeptide of interest comprising the SP-linker at the N-terminal end of the amino acid sequence. Additionally or alternatively, the N- and/or C-terminal processing may include cleavage by a further peptidase when the polypeptide of interest comprises an additional cleavage site, e.g. a cleavage site located between the SP-linker and the amino acid sequence of the mature polypeptide of interest, resulting in a matured polypeptide of interest not comprising the SP-linker at the N-terminal end of the amino acid sequence. Additionally or alternatively, when the polypeptide of interest comprises a pro-peptide, the N- and/or C-terminal processing may include cleavage by a further peptidase when the polypeptide of interest comprises an additional cleavage site, e.g. a cleavage site located between the pro-peptide and the amino acid sequence of the mature polypeptide of interest, resulting in a matured polypeptide of interest lacking the SP-linker and the pro-peptide at the N-terminal end of the amino acid sequence.

**Native:** The term "native" means a nucleic acid or polypeptide naturally occurring in a host cell.

**Nucleic acid:** The term "nucleic acid" encompasses DNA, RNA, heteroduplexes, and synthetic molecules capable of encoding a polypeptide. Nucleic acids may be single stranded or double stranded, and may be chemical modifications. The terms "nucleic acid" and "polynucleotide" are used interchangeably. Because the genetic code is degenerate, more than one codon may be used to encode a particular amino acid, and the present compositions and methods encompass nucleotide sequences that encode a particular amino acid sequence. Unless otherwise indicated, nucleic acid sequences are presented in 5'-to-3' orientation.

**Nucleic acid construct:** The term "nucleic acid construct" means a nucleic acid molecule, either single- or double-stranded, which is isolated from a naturally occurring gene or is modified to contain segments of nucleic acids in a manner that would not otherwise exist in nature or which is synthetic, and which comprises one or more control sequences operably linked to the nucleic acid sequence.

**Obtained polypeptide/peptide/polynucleotide**: The term "obtained" or "derived" when used in reference to a polynucleotide sequence, polypeptide sequence, SP-linker sequence, variant sequence or signal peptide sequence, means that the molecule originally has been isolated from the given source and that the molecule can either be utilized in its native sequence or that the molecule is modified by methods known to the skilled person.

**Operably linked:** The term "operably linked" means that specified components are in a relationship (including but not limited to juxtaposition) permitting them to function in an intended manner. For example, a regulatory sequence is operably linked to a coding sequence such that expression of the coding sequence is under control of the regulatory sequence.

**Phytase**: For purposes of the present invention, phytase activity is determined according to the procedure described in the Examples.

**Pro-peptide:** The term "pro-peptide" means a part of a polypeptide which is cleaved off during maturation or activation. Once cleaved, a pro-peptide generally has no independent biological function. Non-limiting examples of polypeptides comprising a pro-peptide are a deamidase, a protease, a laccase, an alpha-factor, a collagen or procollagen, a proNGF (nervth growth factor), a myostatin, or a hormone or prohormone such as an insulin or proinsulin. In one aspect, the SP-linker of the invention is fused upstream to the pro-peptide of the polypeptide of interest. After maturation or activation, the SP-linker is then together with the pro-peptide cleaved off from the polypeptide of interest, resulting in a mature polypeptide of interest.

**Purified:** The term "purified" means a nucleic acid, polypeptide or cell that is substantially free from other components as determined by analytical techniques well known in the art (*e.g.*, a purified polypeptide or nucleic acid may form a discrete band in an electrophoretic gel, chromatographic eluate, and/or a media subjected to density gradient centrifugation). A purified nucleic acid or polypeptide is at least about 50% pure, usually at least about 60%, about 65%, about 70%, about 75%, about 80%, about 85%, about 90%, about 91%, about 92%, about 93%, about 94%, about 95%, about 96%, about 97%, about 98%, about 99%, about 99.5%, about 99.6%, about 99.7%, about 99.8% or more pure (*e.g.*, percent by weight or on a molar basis). In a related sense, a composition is enriched for a molecule when there is a substantial increase in the concentration of the molecule after application of a purification or enrichment technique. The term "enriched" refers to a compound, polypeptide, cell, nucleic acid, amino acid, or other specified material or component that is present in a composition at a relative or absolute concentration that is higher than a starting composition.

In one aspect, the term "purified" as used herein refers to the polypeptide or cell being essentially free from components (especially insoluble components) from the production organism. In other aspects. the term "purified" refers to the polypeptide being essentially free of insoluble components (especially insoluble components) from the native organism from which it is obtained. In one aspect, the polypeptide is separated from some of the soluble components of the organism and culture medium from which it is recovered. The polypeptide may be purified (*i.e.*, separated) by one or more of the unit operations filtration, precipitation, or chromatography.

Accordingly, the polypeptide may be purified such that only minor amounts of other proteins, in particular, other polypeptides, are present. The term "purified" as used herein may refer to removal of other components, particularly other proteins and most particularly other enzymes present in the cell of origin of the polypeptide. The polypeptide may be "substantially pure", *i.e.*, free from other components from the organism in which it is produced, *e.g.*, a host organism for recombinantly produced polypeptide. In one aspect, the polypeptide is at least 40% pure by weight of the total polypeptide material present in the preparation. In one aspect, the polypeptide is at least 50%, 60%, 70%, 80% or 90% pure by weight of the total polypeptide material present in the preparation. As used herein. a "substantially pure polypeptide" may denote a polypeptide preparation that contains at most 10%, preferably at most 8%, more preferably at most 6%, more preferably at most 5%, more preferably at most 4%, more preferably at most 3%, even more preferably at most 2%, most preferably at most 1%, and even most preferably at most 0.5% by weight of other polypeptide material with which the polypeptide is natively or recombinantly associated.

Therefore, it is preferred that the substantially pure polypeptide is at least 92% pure, preferably at least 94% pure, more preferably at least 95% pure, more preferably at least 96% pure, more preferably at least 97% pure, more preferably at least 98% pure, even more preferably at least 99% pure, most preferably at least 99.5% pure by weight of the total polypeptide material present in the preparation. The polypeptide of the present invention is preferably in a substantially pure form (i.e., the preparation is essentially free of other polypeptide material with which it is natively or recombinantly associated). This can be accomplished, for example by preparing the polypeptide by well-known recombinant methods or by classical purification methods.

**Recombinant:** The term "recombinant" is used in its conventional meaning to refer to the manipulation, *e.g.*, cutting and rejoining, of nucleic acid sequences to form constellations different from those found in nature. The term recombinant refers to a cell, nucleic acid, polypeptide or vector that has been modified from its native state. Thus, for example, recombinant cells express genes that are not found within the native (non-recombinant) form of the cell, or express native genes at different levels or under different conditions than found in nature. The term "recombinant" is synonymous with "genetically modified" and "transgenic".

**Recover:** The terms "recover" or "recovery" means the removal of a polypeptide from at least one fermentation broth component selected from the list of a cell, a nucleic acid, or other specified material, *e.g.*, recovery of the polypeptide from the whole fermentation broth, or from the cell-free fermentation broth, by polypeptide crystal harvest, by filtration, *e.g.*, depth filtration (by use of filter aids or packed filter medias, cloth filtration in chamber filters, rotary-drum filtration, drum filtration, rotary vacuum-drum filters, candle filters, horizontal leaf filters or similar, using sheed or pad filtration in framed or modular setups) or membrane filtration (using sheet filtration, module filtration, candle filtration, microfiltration, ultrafiltration in either cross flow, dynamic cross flow or dead end operation), or by centrifugation (using decanter centrifuges, disc stack centrifuges, hyrdo cyclones or similar), or by precipitating the polypeptide and using relevant solid-liquid separation methods to harvest the polypeptide from the broth media by use of classification separation by particle sizes. Recovery encompasses isolation and/or purification of the polypeptide.

**Second polynucleotide:** The term "second polynucleotide" means a polynucleotide encoding a SP-linker, i.e. N-terminal amino acids derived from a second donor polypeptide. Typically, the second donor polypeptide is secreted by its native host. According to the present invention, the second polynucleotide is located upstream (at the 5' end) of the third polynucleotide encoding the polypeptide of interest. The SP-linker encoded by the second polynucleotide comprises a signal peptidase cleavage site located at the N-terminal end of the SP-linker. The second polynucleotide is isolated or derived from a native polynucleotide encoding a second donor polypeptide. Typically, the second polynucleotide is isolated or derived from a native polynucleotide encoding the first 2-15 N-terminal amino acids of the mature form of the second donor polypeptide. Thus, the second polynucleotide can be cloned from the gene encoding the second donor polypeptide, to generate a recombinant gene where a signal peptide is operably linked in translational fusion with a SP-linker and the polypeptide of interest.

**Second donor polypeptide:** The term "second donor" or "second donor polypeptide" means a polypeptide which in nature is operably linked in translational fusion with a signal peptide. Typically, the second donor polypeptide is a secreted polypeptide, which in its mature form does no longer comprise the signal peptide. For the purpose of the present invention, the second polynucleotide is identical to or derived from the polynucleotide sequence encoding the first 2-15 N-terminal amino acids of the matured second donor polypeptide. The N-terminal amino acid sequence of the second donor polypeptide is defined as 2-15 amino acids at the N-terminus of the mature second donor polypeptide. Typically, the first amino acid of the N-terminus is the first amino acid downstream of the native signal peptide of the second donor polypeptide. Importantly, the N-terminal amino acid sequence of the second donor polypeptide does not comprise the native SP sequence of the second donor polypeptide.

**Sequence identity:** The relatedness between two amino acid sequences or between two nucleotide sequences is described by the parameter "sequence identity".

For purposes of the present invention, the sequence identity between two amino acid sequences is determined as the output of "longest identity" using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 6.6.0 or later. The parameters used are a gap open penalty of 10, a gap extension penalty of 0.5, and the EBLOSUM62 (EMBOSS version of BLOSUM62) substitution matrix. In order for the Needle program to report the longest identity, the -nobrief option must be specified in the command line. The output of Needle labeled "longest identity" is calculated as follows:

$$\text{(Identical Residues x 100)/(Length of Alignment – Total Number of Gaps in Alignment)}$$

For purposes of the present invention, the sequence identity between two polynucleotide sequences is determined as the output of "longest identity" using the Needleman-Wunsch algorithm (Needleman and Wunsch, 1970, supra) as implemented in the Needle program of the EMBOSS package (EMBOSS: The European Molecular Biology Open Software Suite, Rice et al., 2000, supra), preferably version 6.6.0 or later. The parameters used are a gap open penalty of 10, a gap extension penalty of 0.5, and the EDNAFULL (EMBOSS version of NCBI NUC4.4) substitution matrix. In order for the Needle program to report the longest identity, the nobrief option must be specified in the command line. The output of Needle labeled "longest identity" is calculated as follows:

$$\text{(Identical Deoxyribonucleotides x 100)/(Length of Alignment – Total Number of Gaps in Alignment)}$$

**Secreted polypeptide:** The term "secreted" means that the one or more polypeptide of interest, first donor polypeptide, second donor polypeptide or secreted polypeptide is processed and released into an extracellular environment, such as a growth medium. In one embodiment, the one or more polypeptide of interest, first donor polypeptide, second donor polypeptide or secreted polypeptide is processed and released via the conventional secretion pathway comprising the endoplasmatic reticulum, Golgi apparatus, and secretory vesicles. In another embodiment, the one or more polypeptide of interest, first donor polypeptide, second donor polypeptide or secreted polypeptide is processed and released via the unconventional secretion pathway (Rabouille, Trends in Cell Biology 2017, vol. 27, pp. 230-240; Kim et al., Journal of Cell Science 2018, vol. 131 , jcs213686). Additionally or alternatively, a polypeptis also secreted when a polypeptide is transported across the cell membrane without being released into the medium, but instead being integrated into the cell membrane and/or anchored in the cell membrane.

**Signal Peptide:** A "signal peptide" is a sequence of amino acids attached to the N-terminal portion of a protein, which facilitates the secretion of the protein outside the cell. The mature form of an extracellular protein lacks the signal peptide, which is cleaved off during the secretion process.

**Signal peptidase cleavage site:** The term "signal peptidase cleavage site" means a site in a polypeptide sequence which is subject for cleavage by a signal pepditase (SPase). According to the present invention, the signal peptidase cleavage site is located inbetween the signal peptide, and the SP-linker. Additionally or alternatively, secreted polypeptides without a SP-linker have a signal peptidase cleavage site located at the N-terminus of the signal peptide, upstream of the C-terminal end of the polypeptide. Signal peptidases, such as signal peptidase I (SPase I), are critical for the release of translocated preproteins from the membrane as they are transported from a cytoplasmic site of synthesis to extracytoplasmic locations. These proteins are synthesized with an amino-terminal extension, the signal peptide, which directs the preprotein to the Sec- or Tat-translocation pathway in bacteria. Recent evidence indicates that the SPase I cleaves preproteins as they emerge from either pathway, though the steps involved are unclear.

Non-limiting examples for SPases are the SPases with the polypeptide sequences of SEQ ID NO: 21, 22, 23, 49, 50, 51, or 98.

Additional non-limiting examples for SPases are SppA from *Bacillus licheniformis* as described by Cai D. et al., Microb Cell Fact 16, 70 (2017) (https://doi.org/10.1186/s12934-017-0688-7), and signal peptidases sipS, sipT, sipV, sipW described in Cai D. et al., Journal of Appl Microbiology 121, 3 (2016), 704-712.

**SP-linker:** The term "SP-linker" or "signal peptide linker" means a polypeptide comprising 2-15 amino acids which are derived from 2-15 N-terminal amino acids from a second donor polypeptide. The SP-linker is expressed in translational fusion together with a signal peptide (located upstream from the SP-linker, at the N-terminal end of the SP-linker) and together with a polypeptide of interest (located downstream from the SP-linker, at the C-terminal end of the SP-linker). Thereby the SP-linker is linking the signal peptide with the polypeptide of interest. Typically, the SP-linker is heterologous to the polypeptide of interest. Additionally, or alternatively, the SP-linker is heterologous, or homologous to the signal peptide. A SP-linker suitable for expression of a polypeptide of interest with a signal peptide can be identified by screening signal peptide sequences for increased secretion/yield of a chosen secreted polypeptide (second donor polypeptide) to then clone the 2-15 N-terminal amino acids from the chosen secreted polypeptide as a SP-linker and fuse the SP-linker to the C-terminal end of the signal peptide sequence, in which case the SP-linker is heterologous to the signal peptide. A SP-linker suitable for expression of a polypeptide of interest with a signal peptide can be chosen from the native 2-15 N-terminal amino acids of the secreted and matured polypeptide (second donor polypeptide) to which the signal peptide is naturally associated with, in which case the SP-linker is homogenous to the signal peptide. In the context of the invention, the SP sequence is not part of a SP-linker, but instead the SP-linker sequence starts with first N-terminal AA of the mature sequence of the second donor polypeptide.

**Subsequence:** The term "subsequence" means a polynucleotide having one or more nucleotides absent from the 5' and/or 3' end of a mature polypeptide coding sequence, wherein the subsequence encodes a fragment having enzyme activity, such as amylase activity or protease activity.

**Synthetic polynucleotide:** The term "synthetic polynucleotide" means a polynucleotide which is not derived from a natural polynucleotide or gene, and/or is not present in any known wildtype organism. The term "synthetic polypeptide" means a polypeptide which is not derived from a natural or native polypeptide, and/or is not present in any known wildtype organism. Typically, a synthetic polynucleotide or synthetic polypeptide is designed in-silico. A synthetic polypeptide may be expressed using a synthetic polynucleotide.

**Third polynucleotide:** The term "third polynucleotide" means a polynucleotide encoding for a polypeptide of interest. The third polynucleotide may include a pro-peptide coding sequence and/or a nucleic acid sequence encoding an additional cleavage site which allows removal of the SP-linker, and optionally also the pro-peptide, from the polypeptide of interest.

**Variant:** With reference to a polypeptide of interest, the term "variant" means a polypeptide having amylase or protease activity comprising a man-made mutation, *i.e.*, a substitution, insertion (including extension), and/or deletion (*e.g.*, truncation), at one or more positions. With reference to a SP-linker, the term "variant" means a polypeptide derived from the N-terminus of a second donor polypeptide, comprising a man-made mutation, *i.e.*, a substitution, insertion (including extension), and/or deletion (*e.g.*, truncation), at one or more positions.

A substitution means replacement of the amino acid occupying a position with a different amino acid; a deletion means removal of the amino acid occupying a position; and an insertion means adding 1-5 amino acids (*e.g.*, 1-3 amino acids, in particular, 1 amino acid) adjacent to and immediately following the amino acid occupying a position.

**Wild-type:** The term "wild-type" in reference to an amino acid sequence or nucleic acid sequence means that the amino acid sequence or nucleic acid sequence is a native or naturally occurring sequence. As used herein, the term "naturally-occurring" refers to anything (*e.g.*, proteins, amino acids, or nucleic acid sequences) that is found in nature. Conversely, the term "non-naturally occurring" refers to anything that is not found in nature (*e.g.*, recombinant nucleic acids and protein sequences produced in the laboratory or modification of the wild-type sequence).

**yckD polypeptide**: The term "yckD protein" or "yckD polypeptide" means a secreted protein of unknown function (DUF2680) derived from a bacterial host cell, such as *Bacillus subtilis, Bacillus licheniformis* or *Bacillus pumilus.* A non-limiting example for an yckD polypeptide is the polypeptide with SEQ ID NO: 19 derived from *Bacillus pumilus.* The gene encoding the yckD polypeptide also encodes a yckD signal peptide which is fused to the C-terminal end of the yckD polypeptide, and cleaved off during the maturation and secretion process. A non-limiting example for a yckD signal peptide is the signal peptide with the amino acid sequence of SEQ ID NO: 4.

**Fungal prolyl dipeptidyl peptidase**: The term "fungal prolyl dipeptidyl peptidase" means a secreted protein encoded by a fungal cell, preferably a filamentous fungal cell, e.g. *Aspergillus oryzae.* A non-limiting example for an fungal prolyl didpeptidyl peptidase is the *A. oryzae* peptidase which is secreted with its native signal peptide comprising the sequence of SEQ ID NO: 172.

## Detailed Description of the Invention

### Polynucleotides

In one aspect, the present invention relates to polynucleotides encoding a signal peptdie, a SP-linker, or a fusion polypeptide comprising a SP-linker and a polypeptide of interest, as described herein.

The polynucleotide may be a genomic DNA, a cDNA, a synthetic DNA, a synthetic RNA, a mRNA, or a combination thereof. The polynucleotide may be cloned from a strain of *Bacillus*, or a related organism and thus, for example, may be a polynucleotide sequence encoding a variant of the fusion polypeptide, signal peptide or SP-linker of the invention.

In one embodiment, the polynucleotide encoding the SP-linker is a subsequence encoding a N-terminal fragment of a secreted second donor polypeptide of the present invention. In an aspect, the subsequence contains at least 6 nucleotides (*e.g.*, nucleotides 1 to 6 of SEQ ID NO: 1 or SEQ ID NO: 2), at least 12 nucleotides (*e.g.*, nucleotides 1 to 12 of SEQ ID NO: 1 or SEQ ID NO: 2), or at least 21 nucleotides (*e.g.*, nucleotides 1 to 21 of SEQ ID NO: 1, SEQ ID NO: 2, SEQ ID NO: 175, or SEQ ID NO: 176).

In one embodiment the polynucleotide encoding the SP-linker of the present invention is isolated from a *Bacillus* cell.

In one embodiment the polynucleotide encoding the SP-linker of the present invention is isolated from an *Aspergillus* cell.

The polynucleotide may also be mutated by introduction of nucleotide substitutions that do not result in a change in the amino acid sequence of the polypeptide, but which correspond to the codon usage of the host organism intended for production of the enzyme, or by introduction of nucleotide substitutions that may give rise to a different amino acid sequence. For a general description of nucleotide substitution, see, *e.g.*, Ford et al., 1991, Protein Expression and Purification 2: 95-107.

### Nucleic Acid Constructs

The present invention also relates to nucleic acid constructs comprising a polynucleotide of the present invention, wherein the polynucleotide is operably linked to one or more control sequences that direct the expression of the coding sequence in a suitable host cell under conditions compatible with the control sequences.

The polynucleotide may be manipulated in a variety of ways to provide for expression of the polypeptide. Manipulation of the polynucleotide prior to its insertion into a vector may be desirable or necessary depending on the expression vector. Techniques for modifying polynucleotides utilizing recombinant DNA methods are well known in the art.

In a first aspect 1A, the present invention relates to a nucleic acid construct comprising or consisting of:

a) a first polynucleotide encoding a signal peptide (SP),
b) a second polynucleotide located downstream of the first polynucleotide and encoding a SP-linker consisting of 2 to 15 amino acids, wherein the SP-linker amino acid sequence consists of the N-terminal amino acid sequence of a second donor polypeptide; and
c) at least one third polynucleotide located downstream of the second polynucleotide and encoding a polypeptide of interest;

wherein the first polynucleotide and the second polynucleotide are heterologous to the third polynucleotide, and wherein the first polynucleotide, the second polynucleotide, and the third polynucleotide are operably linked in translational fusion.

During the maturation process, the SP will typically be cleaved off the polypeptide of interest, leaving a mature polypeptide of interest which may comprise the SP-linker, i.e. 2-15 additional amino acids, at the N-terminal end of the polypeptide of interest. When the polypeptide of interest comprises a pro-peptide at the N-terminal of the polypeptide of interest, the SP-linker may together with the pro-peptide be removed during the maturation process, resulting in a matured polypeptide of interest which is essentially free of SP-linker and/or pro-peptide sequence.

In one embodiment, the first polynucleotide encoding the signal peptide is derived from a polynucleotide encoding a first donor polypeptide. Typically, the first donor polypeptide is a secreted polypeptide. The second donor polypeptide may comprise a pro-peptide, which may be removed during the maturation process. In one embodiment the first donor polypeptide is a matured first donor polypeptide.

In another embodiment, the second polynucleotide encoding the SP-linker is derived from a polynucleotide encoding a second donor polypeptide. In one embodiment, the second donor polypeptide is a matured second donor polypeptide. In one embodiment, the polypeptide of interest is a matured polypeptide of interest.

Preferably the second donor polypeptide is heterologous to the polypeptide of interest.

In one embodiment the SP-linker consists of 2 to 10 amino acids, preferably consists of 2 to 9 amino acids, 2 to 8 amino acids, 3 to 8 amino acids, 3 to 9 amino acids, 2 to 7 amino acids, 3 to 7 amino acids, 2 to 6 amino acids, or 3 to 6 amino acids, 4 to 10 amino acids, 5 to 10 amino acids, 4 to 9 amino acids, 5 to 9 amino acids, 4 to 8 amino acids, or 5 to 8 amino acids. The optimal length of the SP-linker may depend on signal peptidases encoded by the host cell in which the nucleic acid construct is expressed in. Additionally or alternatively, the optimal length of the SP-linker may vary for different second donor polypeptides, and may also depend on the signal peptide it is fused with. The skilled person in the art will easily recognize ways to identify an optimal length of a specific SP-linker, or a class of SP-linkers derived from heterologous second donor polypeptides. For example, SP-linkers of different lenght can be ranked against another relative to the expression levels of a polypeptide of interest to which the SP-linker is fused to, wherein an optimal SP-linker length will rank amongst the SP-linkers with the highest polypeptide secretion and/or yield.

In one embodiment, the first polynucleotide is endogenous to the second polynucleotide encoding the SP-linker, and/or endogenous to the polynucleotide encoding the second donor polypeptide. When the SP and the SP-linker are endogenous to another, the polynucleotide encoding the SP - SP-linker can directly be cloned from the gene encoding the donor polypeptide. In one embodiment the donor polypeptide is the *aspartate phosphatase* from *B. pumilus* (SEQ ID NO: 18). In another embodiment the donor polypeptide is the *yckD* protein from *B. pumilus* (SEQ ID NO: 19). In another embodiment the donor polypeptide is the xylanase from *Thermomyces lanuginosus.* In another embodiment the donor polypeptide is the endoglucanase from *Thermothielavioides terrestris.* Using such endogenous SP- SP-linker constructs, the native cleavage site between the SP and the SP-linker will be transferred to the polypeptide of interest.

In one embodiment, the amino acid sequence of the matured first donor polypeptide has at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% sequence identity to the amino acid sequence of the matured second donor polypeptide. The first donor and the second donor may be homologs of another, derived from the same species or derived from different species.

In one embodiment the matured first donor polypeptide and the matured second donor polypeptide have a different biological function. In one embodiment the matured second donor polypeptide and the matured polypeptide of interest have a different biological function.

In a preferred embodiment, a signal peptidase cleavage site is located downstream of the signal peptide encoded by the first polynucleotide. In another embodiment, a signal peptidase cleavage site is located upstream of the SP-linker encoded by the second polynucleotide. The signal peptidase cleavage site allows maturation of the polypeptide of interest, and removal of the SP sequence from the matured polypeptide of interest.

In one embodiment, the signal peptide is derived from a first donor polypeptide selected from the list consisting of a structural polypeptide, a receptor, a secreted polypeptide, a hormone, or a secreted enzyme, such as a hydrolase, isomerase, ligase, lyase, lysozyme, oxidoreductase, or transferase, e.g., an aminopeptidase, amylase, carbohydrase, carboxypeptidase, catalase, cellobiohydrolase, cellulase, chitinase, cutinase, cyclodextrin glycosyltransferase, deoxyribonuclease, endoglucanase, esterase, alpha-galactosidase, beta-galactosidase, glucoamylase, alpha-glucosidase, beta-glucosidase, invertase, laccase, lipase, mannosidase, mutanase, nuclease, oxidase, pectinolytic enzyme, peroxidase, phosphodiesterase, phytase, polyphenoloxidase, proteolytic enzyme, ribonuclease, transglutaminase, xylanase, aspartate phosphatase, or beta-xylosidase.

In another embodiment, the signal peptide is derived from a first donor polypeptide comprising or consisting of an aspartate phosphatase, preferably an aspartate phosphatase from *Bacillus,* such as *Bacillus pumilus.*

In another embodiment, the signal peptide is derived from a first donor polypeptide comprising or consisting of a peptidase, such as an polyl dipeptidyl peptidase, preferably a peptidase from *Aspergillus,* such as *Aspergilly oryzae.*

In another embodiment, the signal peptide is derived from a first donor polypeptide comprising or consisting of a lipase, such as an triacylglycerol lipase tglA, preferably a lipase from *Aspergillus*, such as *Aspergilly oryzae.*

In one embodiment, the signal peptide is derived from a bacterial cell or a eukaryotic cell.

In one embodiment, the signal peptide is derived from a bacterial cell, e.g., a Gram-positive cell selected from the group consisting of *Bacillus*, *Clostridium*, *Enterococcus*, *Geobacillus*, *Lactobacillus*, *Lactococcus*, *Oceanobacillus*, *Staphylococcus*, *Streptococcus*, or *Streptomyces* cells, or a Gram-negative bacteria selected from the group consisting of *Campylobacter*, *E. coli*, *Flavobacterium*, *Fusobacterium*, *Helicobacter*, *Ilyobacter*, *Neisseria*, *Pseudomonas*, *Salmonella*, and *Ureaplasma* cells, such as *Bacillus alkalophilus*, *Bacillus amyloliquefaciens*, *Bacillus brevis*, *Bacillus circulans*,

*Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis, Bacillus thuringiensis, Streptococcus equisimilis, Streptococcus pyogenes, Streptococcus uberis,* and *Streptococcus equi* subsp. *Zooepidemicus, Streptomyces achromogenes, Streptomyces avermitilis, Streptomyces coelicolor, Streptomyces griseus,* and *Streptomyces lividans* cells.

In a particular embodiment, the signal peptide is derived from a *Bacillus* cell, such as from a *Bacillus subtilis* cell, a *Bacillus licheniformis* cell, or a *Bacillus pumilus* cell.

In one embodiment, the signal peptide is derived from a *Bacillus pumilus* cell.

In another embodiment, the signal peptide is derived from a first donor polypeptide comprising or consisting of an amino acid sequence having at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% sequence identity to the amino acid sequence of SEQ ID NO: 18 or 19.

In one embodiment, the signal peptide is derived from a eukaryotic cell selected from the list of a fungal cell, a filamentous fungal cell, a yeast cell, and a mammalian cell.

In one embodiment, the signal peptide is derived from a yeast cell, *e.g.* a *Candida, Hansenula, Kluyveromyces, Pichia, Saccharomyces, Schizosaccharomyces,* or *Yarrowia* cell, such as a *Kluyveromyces lactis, Saccharomyces carlsbergensis, Saccharomyces cerevisiae, Saccharomyces diastaticus, Saccharomyces douglasii, Saccharomyces kluyveri, Saccharomyces norbensis, Saccharomyces oviformis,* or *Yarrowia lipolytica* cell.

In another embodiment, the signal peptide is derived from a filamentous fungal recombinant host cell, *e.g.*, an *Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes,* or *Trichoderma* cell, in particular, an *Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Talaromyces emersonii, Thielavia terrestris, Trametes villosa, Trametes versicolor, Trichoderma harzianum, Trichoderma koningii, Trichoderma longibrachiatum, Trichoderma reesei,* or *Trichoderma viride* cell.

In one embodiment, the signal peptide is derived from a *Pichia pastoris,* a *Trichoderma reesei,* a *Aspergillus niger,* or a *Aspergillus oryzae* cell.

In one embodiment, the signal peptide is derived from an *Aspergillus oryzae* cell.

In one embodiment the signal peptide is derived from an *Aspergillus oryzae* polyl dipeptidyl peptidase.

In one embodiment the signal peptide is derived from an *Aspergillus oryzae* triacylglycerol lipase *tglA.*

In another embodiment, the SP-linker is derived from a second donor polypeptide selected from the list consisting of a structural polypeptide, a receptor, a secreted polypeptide, a hormone, or a secreted enzyme, such as a hydrolase, isomerase, ligase, lyase, lysozyme, oxidoreductase, or transferase, e.g., an aminopeptidase, amylase, carbohydrase, carboxypeptidase, catalase, cellobiohydrolase, cellulase, chitinase, cutinase, cyclodextrin glycosyltransferase, deoxyribonuclease, endoglucanase, esterase, alpha-galactosidase, beta-galactosidase, glucoamylase, alpha-glucosidase, beta-glucosidase, invertase, laccase, lipase, mannosidase, mutanase, nuclease, oxidase, pectinolytic enzyme, peroxidase, phosphodiesterase, phytase, polyphenoloxidase, proteolytic enzyme, ribonuclease, transglutaminase, xylanase, aspartate phosphatase, or beta-xylosidase.

In a particular embodiment, the SP-linker is derived from a second donor polypeptide comprising or consisting of a protease. The protease may comprise a pro-peptide at the N-terminal end, thus the SP-linker may be derived from the pro-peptide.

In one embodiment the SP-linker is derived from a second donor polypeptide comprising or consisting of a xylanase.

In one embodiment the SP-linker is derived from a second donor polypeptide comprising or consisting of a endoglucanase.

In one embodiment, the SP-linker of the second donor polypeptide is derived from a bacterial cell or a eukaryotic cell.

In one embodiment, the SP-linker of the second donor polypeptide is derived from a bacterial cell, *e.g.,* a Gram-positive cell selected from the group consisting of *Bacillus, Clostridium, Enterococcus, Geobacillus, Lactobacillus, Lac-*

*tococcus, Oceanobacillus, Staphylococcus, Streptococcus,* or *Streptomyces* cells, or a Gram-negative bacteria selected from the group consisting of *Campylobacter, E. coli, Flavobacterium, Fusobacterium, Helicobacter, Ilyobacter, Neisseria, Pseudomonas, Salmonella,* and *Ureaplasma* cells, such as *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis, Bacillus thuringiensis, Streptococcus equisimilis, Streptococcus pyogenes, Streptococcus uberis,* and *Streptococcus equi* subsp. *Zooepidemicus, Streptomyces achromogenes, Streptomyces avermitilis, Streptomyces coelicolor, Streptomyces griseus,* and *Streptomyces lividans* cells.

In a particular embodiment the SP-linker of the second donor polypeptide is derived from a *Bacillus* cell, such as from a *Bacillus subtilis* cell, a *Bacillus licheniformis* cell, or a *Bacillus pumilus* cell.

In one embodiment, the SP-linker of the second donor polypeptide is derived from a *Bacillus* cell or a *Nocardiopsis* cell, preferably from a *Bacillus clausii* cell, or a *Nocardiopsis prasina* cell.

In one embodiment, the SP-linker is derived from a second donor polypeptide comprising or consisting of an amino acid sequence having at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% sequence identity to the amino acid sequence of SEQ ID NO: 16 or 17.

In one embodiment, the SP-linker of the second donor polypeptide is derived from a eukaryotic cell selected from the list of a fungal cell, a filamentous fungal cell, a yeast cell, and a mammalian cell.

In another embodiment, the SP-linker of the second donor polypeptide is derived from a yeast cell, *e.g.* a *Candida, Hansenula, Kluyveromyces, Pichia, Saccharomyces, Schizosaccharomyces,* or *Yarrowia* cell, such as a *Kluyveromyces lactis, Saccharomyces carlsbergensis, Saccharomyces cerevisiae, Saccharomyces diastaticus, Saccharomyces douglasii, Saccharomyces kluyveri, Saccharomyces norbensis, Saccharomyces oviformis,* or *Yarrowia lipolytica* cell.

In one embodiment the SP-linker of the second donor polypeptide is derived from a filamentous fungal recombinant host cell, *e.g.,* an *Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes,* or *Trichoderma* cell, in particular, an *Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Talaromyces emersonii, Thielavia terrestris, Trametes villosa, Trametes versicolor, Trichoderma harzianum, Trichoderma koningii, Trichoderma longibrachiatum, Trichoderma reesei,* or *Trichoderma viride* cell.

In one embodiment, the SP-linker of the second donor polypeptide is derived from a *Pichia pastoris,* a *Trichoderma reesei,* a *Aspergillus niger,* or a *Aspergillus oryzae* cell.

In one embodiment, the SP-linker of the second donor polypeptide is derived from *Thermomyces lanuginosus.*

In one embodiment, the SP-linker of the second donor polypeptide is derived from *Thermothielavioides terrestris.*

In one embodiment, the first polynucleotide and/or the second polynucleotide is a synthetic polynucleotide. A synthetic first polynucleotide encoding a synthetic SP may be combined with a native, non-synthetic or with a synthetic second polynucleotide encoding a SP-linker to achieve increased expression and/or secretion of the polypeptide of interest. Additionally or alternatively, a synthetic second polynucleotide encoding a synthetic SP-linker may be combined with a native, non-synthetic or with a synthetic first polynucleotide encoding a SP to achieve increased expression and/or secretion of the polypeptide of interest.

In one embodiment, the nucleic acid construct further comprises a heterologous promoter operably linked to the first polynucleotide. In a particular embodiment, the heterologous promoter comprises or consists of a nucleotide sequence having at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% sequence identity to the polynucleotide sequence of SEQ ID NO: 20.

In one embodiment, the second donor polypeptide has protease activity. In a particular embodiment, the second donor polypeptide comprises a pro-peptide at the N-terminal end of the amino acid sequence. When the second donor comprises a pro-peptide at the N-terminal end, the SP-linker may be derived from the pro-peptide of the second donor polypeptide.

In one embodiment, the first polynucleotide sequence comprises or consists of a nucleic acid sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the nucleic acid sequence of SEQ ID NO: 1.

In a particular embodiment, the first polynucleotide sequence comprises or consists of a nucleic acid sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the nucleic acid sequence of SEQ ID NO: 2.

In a particular embodiment, the first polynucleotide sequence comprises or consists of a nucleic acid sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the nucleic acid sequence of SEQ ID NO: 177.

In a particular embodiment, the first polynucleotide sequence comprises or consists of a nucleic acid sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the nucleic acid sequence of SEQ ID NO: 178.

In one particular embodiment, the first polynucleotide sequence encodes a signal peptide comprising or consisting of an amino acid sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the amino acid sequence of SEQ ID NO: 3 "MKFKTLSVVFVMISVISVVSYFTSEVTA".

In another embodiment, the first polynucleotide sequence encodes a signal peptide comprising or consisting of an amino acid sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the amino acid sequence of SEQ ID NO: 4 "MMKKVSGLIILSMMLLSGFYMETAYGA".

In another embodiment, the first polynucleotide sequence encodes a signal peptide comprising or consisting of an amino acid sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the amino acid sequence of SEQ ID NO: 171 "MHLAIKSLFVSLLGASVLASPLPSNALVERA".

In another embodiment, the first polynucleotide sequence encodes a signal peptide comprising or consisting of an amino acid sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the amino acid sequence of SEQ ID NO: 172 "MKYSKLLLLLVSVVQA".

In one embodiment, the second polynucleotide comprises or consists of a polynucleotide sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the polynucleotide sequence of SEQ ID NO: 5.

In another embodiment, the second polynucleotide comprises or consists of a polynucleotide sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the polynucleotide sequence of SEQ ID NO: 6.

In one embodiment, the second polynucleotide comprises or consists of a polynucleotide sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the polynucleotide sequence of SEQ ID NO: 175.

In another embodiment, the second polynucleotide comprises or consists of a polynucleotide sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the polynucleotide sequence of SEQ ID NO: 176.

In one embodiment, the second polynucleotide sequence encodes a SP-linker comprising or consisting of an amino acid sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the amino acid sequence of SEQ ID NO: 7 "TGALPQS". In one embodiment, the first and the second polynucleotides encode a SP with SP-linker comprising or consisting of an amino acid sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the amino acid sequence of SEQ ID NO: 11 "MKFKTLSVVFVMISVISVVSYFTSEVTATGALPQS", SP-linker sequence in bold letters.

In another embodiment, the second polynucleotide sequence encodes a SP-linker comprising or consisting of an amino acid sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the amino acid sequence of SEQ ID NO: 8 "AEEAKEK". In one embodiment, the first and the second polynucleotides encode a SP with a SP-linker comprising or consisting of an amino acid sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the amino acid sequence of SEQ ID NO: 12 "MMKKVSGLIILSMMLLSGFYMETAYGAAEEAKEK", SP-linker sequence in bold letters.

In another embodiment, the second polynucleotide sequence encodes a SP-linker comprising or consisting of an amino acid sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the amino acid sequence of SEQ ID NO: 179 "FPAGNAT". In one embodiment, the first and the second polynucleotides encode a SP with a SP-linker comprising or consisting of an amino acid sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the amino acid sequence of SEQ ID NO: 173 "MHLAIKSLFVSLLGASVLASPLPSNALVERA**FPAGNAT**", SP-linker sequence in bold letters.

In another embodiment, the second polynucleotide sequence encodes a SP-linker comprising or consisting of an amino acid sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the amino acid sequence of SEQ ID NO: 180 "ASGSGQS". In one embodiment, the first and the second polynucleotides encode a SP with a SP-linker comprising or consisting of an amino acid sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the amino acid sequence of SEQ ID NO: 174 "MKYSKLLLLLVSVVQA**ASGSGQS**", SP-linker sequence in bold letters.

In one embodiment, the first and the second polynucleotides encode a SP with a SP-linker comprising or consisting of an amino acid sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the amino acid sequence of SEQ ID NO: 11, SEQ ID NO: 12, SEQ ID NO: 153- 167, SEQ ID NO: 170, SEQ ID NO: 173, or SEQ ID NO: 174.

In one embodiment, the second polynucleotide encodes a SP-linker comprising or consisting of an amino acid sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the amino acid sequence of "T", "TG", "TGA",

SEQ ID NO: 24 "TGAL",
SEQ ID NO: 25 "TGALP",
SEQ ID NO: 26 "TGALPQ",
SEQ ID NO: 27 "TGALPQS",
SEQ ID NO: 28 "TGALPQSP",
SEQ ID NO: 29 "TGALPQSPT",
SEQ ID NO: 30 "TGALPQSPTP",
SEQ ID NO: 31 "TGALPQSPTPE",
SEQ ID NO: 32 "TGALPQSPTPEA",
SEQ ID NO: 33 "TGALPQSPTPEAD",
SEQ ID NO: 34 "TGALPQSPTPEADA",
SEQ ID NO: 35 "TGALPQSPTPEADAV",
SEQ ID NO: 36 "TGALPQSPTPEADAVS",
SEQ ID NO: 37 "TGALPQSPTPEADAVSM",
SEQ ID NO: 38 "TGALPQSPTPEADAVSMQ",
SEQ ID NO: 39 "TGALPQSPTPEADAVSMQE",
SEQ ID NO: 40 "TGALPQSPTPEADAVSMQEA",
SEQ ID NO: 41 "TGALPQSPTPEADAVSMQEAL",

SEQ ID NO: 42 "TGALPQSPTPEADAVSMQEALQ",
SEQ ID NO: 43 "TGALPQSPTPEADAVSMQEALQR",
SEQ ID NO: 44 "TGALPQSPTPEADAVSMQEALQRD",
SEQ ID NO: 45 "TGALPQSPTPEADAVSMQEALQRDL",
SEQ ID NO: 46 "TGALPQSPTPEADAVSMQEALQRDLD",
SEQ ID NO: 47 "TGALPQSPTPEADAVSMQEALQRDLDL",
SEQ ID NO: 48 "TGALPQSPTPEADAVSMQEALQRDLDLT", "A", "AE", "AEE",
SEQ ID NO: 52 "AEEA",
SEQ ID NO: 53 "AEEAK",
SEQ ID NO: 54 "AEEAKE",
SEQ ID NO: 55 "AEEAKEK",
SEQ ID NO: 56 "AEEAKEKY",
SEQ ID NO: 57 "AEEAKEKYL",
SEQ ID NO: 58 "AEEAKEKYLI",
SEQ ID NO: 59 "AEEAKEKYLIG",
SEQ ID NO: 60 "AEEAKEKYLIGF",
SEQ ID NO: 61 "AEEAKEKYLIGFN",
SEQ ID NO: 62 "AEEAKEKYLIGFNE",
SEQ ID NO: 63 "AEEAKEKYLIGFNEQ",
SEQ ID NO: 64 "AEEAKEKYLIGFNEQE",
SEQ ID NO: 65 "AEEAKEKYLIGFNEQEA",
SEQ ID NO: 66 "AEEAKEKYLIGFNEQEAV",
SEQ ID NO: 67 "AEEAKEKYLIGFNEQEAVS",
SEQ ID NO: 68 "AEEAKEKYLIGFNEQEAVSE",
SEQ ID NO: 69 "AEEAKEKYLIGFNEQEAVSEF",
SEQ ID NO: 70 "AEEAKEKYLIGFNEQEAVSEFV",
SEQ ID NO: 71 "AEEAKEKYLIGFNEQEAVSEFVE",
SEQ ID NO: 72 "AEEAKEKYLIGFNEQEAVSEFVEQ",
SEQ ID NO: 73 "AEEAKEKYLIGFNEQEAVSEFVEQV",
SEQ ID NO: 74 "AEEAKEKYLIGFNEQEAVSEFVEQVE",
SEQ ID NO: 75 "AEEAKEKYLIGFNEQEAVSEFVEQVEA",
SEQ ID NO: 76 "AEEAKEKYLIGFNEQEAVSEFVEQVEAN", "S", "SH", "SHD",
SEQ ID NO: 79,
SEQ ID NO: 83 "SHDE",
SEQ ID NO: 84 "SHDEA",
SEQ ID NO: 85 "SHDEAR",
SEQ ID NO: 86 "SHDEARR",
SEQ ID NO: 87 "SHDEARRG",
SEQ ID NO: 88 "SHDEARRGH",
SEQ ID NO: 89 "SHDEARRGHT",
SEQ ID NO: 90 "SHDEARRGHTA",
SEQ ID NO: 91 "SHDEARRGHTAS",
SEQ ID NO: 92 "SHDEARRGHTASI",
SEQ ID NO: 93 "SHDEARRGHTASIG",
SEQ ID NO: 94 "SHDEARRGHTASIGY",
SEQ ID NO: 95 "SHDEARRGHTASIGYT",
SEQ ID NO: 96 "SHDEARRGHTASIGYTA", "T", "TP", "TPQ",
SEQ ID NO: 97 "TPQMEKR"
SEQ ID NO: 101 "TPQM",
SEQ ID NO: 102 "TPQME",
SEQ ID NO: 103 "TPQMEK",
SEQ ID NO: 104 "TPQMEKR",
SEQ ID NO: 105 "TPQMEKRP",
SEQ ID NO: 106 "TPQMEKRPV",
SEQ ID NO: 107 "TPQMEKRPVM",
SEQ ID NO: 108 "TPQMEKRPVMH",
SEQ ID NO: 109 "TPQMEKRPVMHQ",
SEQ ID NO: 110 "TPQMEKRPVMHQV",

SEQ ID NO: 111 "TPQMEKRPVMHQVK",
SEQ ID NO: 112 "TPQMEKRPVMHQVKL",
SEQ ID NO: 113 "TPQMEKRPVMHQVKLT",
SEQ ID NO: 114 "TPQMEKRPVMHQVKLTT",
SEQ ID NO: 115 "TPQMEKRPVMHQVKLTTE",
SEQ ID NO: 116 "TPQMEKRPVMHQVKLTTEQ",
SEQ ID NO: 117 "TPQMEKRPVMHQVKLTTEQQ",
SEQ ID NO: 118 "TPQMEKRPVMHQVKLTTEQQK",
SEQ ID NO: 119 "TPQMEKRPVMHQVKLTTEQQKQ",
SEQ ID NO: 120 "TPQMEKRPVMHQVKLTTEQQKQI",
SEQ ID NO: 121 "TPQMEKRPVMHQVKLTTEQQKQIE",
SEQ ID NO: 122 "TPQMEKRPVMHQVKLTTEQQKQIEM",
SEQ ID NO: 123 "TPQMEKRPVMHQVKLTTEQQKQIEML",
SEQ ID NO: 124 "TPQMEKRPVMHQVKLTTEQQKQIEMLE",
SEQ ID NO: 125 "TPQMEKRPVMHQVKLTTEQQKQIEMLEQ",
SEQ ID NO: 126 "TPQMEKRPVMHQVKLTTEQQKQIEMLEQQ", "FP", "FPA",
SEQ ID NO: 181 "FPAG",
SEQ ID NO: 182 "FPAGN",
SEQ ID NO: 183 "FPAGNA",
SEQ ID NO: 179 "FPAGNAT",
"AS", "ASG",
SEQ ID NO: 184 "ASGS",
SEQ ID NO: 185 "ASGSG",
SEQ ID NO: 186 "ASGSGQ", or
SEQ ID NO: 180 "ASGSGQS".

In one embodiment, the signal peptidase cleavage site is recognized by a signal peptidase selected from the list of a SPase I, SPase II, SPase III, and SPase IV. The signal peptidase recognizes the cleavage site, based on a recognition motif located at the C-terminal end of the SP sequence and/or a recognition motif located at the N-terminal end of the SP-linker. The presence of the SP-linker with an additional recognition motif for the signal peptidase at the N-terminal end of the SP-linker is thought to increase secretion and/or yield of the polypeptide of interest, relative to the secretion and/or yield of the polypeptide of interest expressed from a cassette lacking the SP-linker.

In one embodiment, the signal peptidase cleavage site is recognized by a signal peptidase derived from *Bacillus licheniformis*, preferably by a signal peptidase comprising or consisting of an amino acid sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the amino acid sequence of SEQ ID NO: 21, 22, 23, 49, 50, 51, or 98.

In another embodiment, the second polynucleotide encodes a SP-linker consisting of two amino acids, three amino acids, four amino acids, 5 amino acids, 6 amino acids, 7 amino acids, 8 amino acids, 9 amino acids, 10 amino acids, 11 amino acids, 12 amino acids, 13 amino acids, 14 amino acids, or 15 amino acids. In a particular embodiment, the second polynucleotide encodes a SP-linker consisting of 2 - 10 amino acids.

In one embodiment, the polypeptide of interest is selected from the list of an enzyme, a therapeutic polypeptide, and a food or feed polypeptide, such as an antibody, an antigen, an antimicrobial peptide, a growth factor, a hormone, an immunodilator, a neurotransmitter, a receptor, a reporter protein, a structural protein, a transcription factor, a heme-containing polypeptide, a brazzein, a casein, a patatin, an ovalbumin, an osteopontin, an ovotransferrin, an ovomucin, an ovomucoid, an ovostatin, a lactoferrin, an alpha-lactalbumin, a beta-lactalbumin, a glycomacropeptide, or a collagen. Any desired polypeptide of interest can be fused to the SP-linker to optimize secretion and/or yield thereof. A polypeptide of interest, which in nature is not secreted by its organism, may also be fused to a SP-linker of the present invention to enable secretion and recombinant production.

In one embodiment, the polypeptide of interest is selected from a hydrolase, isomerase, ligase, lyase, lysozyme, oxidoreductase, or transferase, e.g., an aminopeptidase, amylase, carbohydrase, carboxypeptidase, catalase, cellobiohydrolase, cellulase, chitinase, cutinase, cyclodextrin glycosyltransferase, deoxyribonuclease, endoglucanase, esterase, alpha-galactosidase, beta-galactosidase, glucoamylase, alpha-glucosidase, beta-glucosidase, invertase, laccase, lipase, mannosidase, mutanase, nuclease, oxidase, pectinolytic enzyme, peroxidase, phosphodiesterase, phytase, polyphenoloxidase, proteolytic enzyme, ribonuclease, transglutaminase, xylanase, aspartate phosphatase, or beta-xylosidase.

In one embodiment, the third polynucleotide sequence comprises or consists of a nucleotide sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the nucleotide sequence of SEQ ID NO: 13.

In one embodiment, the polypeptide of interest comprises a pro-peptide. Preferably, the pro-peptide is located at the N-terminal end of the amino acid sequence of the protein of interest. In one embodiment, the pro-peptide is located downstream of the SP-linker. The SP-linker and the pro-peptide may both be cleaved off the amino acid sequence of the matured protein of interest.

In one embodiment, the protein of interest is not secreted when expressed in its native form. In one embodiment, the protein of interest is selected from the list of an epimerase, a heme-containing polypeptide, a mannanase, an endoglucanase, an asparaginase, a xylanase, a glucoisomerase, a xyloseisomerase, and a protease.

In one embodiment the nucleic acid construct encodes an additional cleavage site located between the SP-linker and the polypeptide of interest, or between the SP-linker and the pro-peptide of the polypeptide of interest. The additional cleavage site allows removal of the SP-linker from the matured polypeptide of interest. In one embodiment, the additional cleavage site is a proteolytic cleavage site. In one embodment, the additional cleavage site is recognized by a suitable protease, preferably a KexB protease. The additional cleavage site may comprise or consist of a dibasic amino acid motif such as a Lys-Arg ("KR") or an Arg-Arg motif ("RR"). Thus, the additional cleavage site may be a KexB or Kex2 cleavage site.

In one embodiment, the nucleic acid construct is isolated.

In another embodiment, the nucleic acid construct is purified.

In a first aspect 1B, the invention also relates to a nucleic acid construct comprising:

    a) a first polynucleotide encoding a signal peptide from a bacterial yckD polypeptide; and
    b) a second polynucleotide encoding a polypeptide of interest;

wherein the first polynucleotide and the second polynucleotide are operably linked in translational fusion.

In one embodiment, the nucleic acid construct further comprises a heterologous promoter, and wherein said promoter, the first polynucleotide, and the second polynucleotide are operably linked. In one embodiment, the promoter is a P3 promoter or a P3-based promoter. In a particular embodiment, the promoter is a P3 promoter or a P3-based promoter, preferably the heterologous promoter is a tandem promoter comprising the P3 promoter or is a tandem promoter derived from the P3 promoter. In one embodiment, the promoter comprises or consists of a nucleic acid sequence having a sequence identity of least 80%, e.g. at least 85%, at least 90%, at least 91%, at least 92%, at least 93%, at least 94%, at least 95%, at least 96%, at least 97%, at least 98%, at least 99%, or 100%, to SEQ ID NO: 20, preferably the promoter comprises, consists essentially of, or consists of SEQ ID NO: 20.

In one embodiment, the signal peptide is a naturally occurring signal peptide, or a functional fragment or functional variant of a naturally occurring signal peptide.

In one embodiment, the signal peptide is obtained from a yckD polypeptide from a *Bacillus* species selected from the group consisting of *Bacillus alkalophilus*, *Bacillus amyloliquefaciens*, *Bacillus brevis*, *Bacillus circulans*, *Bacillus clausii*, *Bacillus coagulans*, *Bacillus firmus*, *Bacillus lautus*, *Bacillus lentus*, *Bacillus licheniformis*, *Bacillus megaterium*, *Bacillus pumilus*, *Bacillus stearothermophilus*, *Bacillus subtilis*, and *Bacillus thuringiensis* cells. In a particular embodiment, the signal peptide is obtained from a yckD polypeptide from *Bacillus licheniformis*, *Bacillus subtilis* or *Bacillus pumilus.* In one embodiment, the signal peptide is obtained from a yckD polypeptide from *Bacillus pumilus.*

In one embodiment, the signal peptide is obtained from a bacterial yckD polypeptide having a sequence identity of at least 80%, e.g. at least 85%, at least 90%, at least 91%, at least 92%, at least 93%, at least 94%, at least 95%, at least 96%, at least 97%, at least 98%, at least 99%, or 100%, to SEQ ID NO: 19, preferably the bacterial yckD polypeptide comprises, consists essentially of, or consists of SEQ ID NO: 19.

In one embodiment, the first polynucleotide encoding the signal peptide has a sequence identity of at least 80%, *e.g.* at least 85%, at least 90%, at least 91%, at least 92%, at least 93%, at least 94%, at least 95%, at least 96%, at least 97%, at least 98%, at least 99%, or 100%, to the mature polypeptide coding sequence of SEQ ID NO:2; most preferably the polynucleotide comprises, consists essentially of, or consists of the mature polypeptide coding sequence of SEQ ID NO: 2.

In one embodiment, the signal peptide has a sequence identity of at least 80%, *e.g.* at least 85%, at least 90%, at least 91%, at least 92%, at least 93%, at least 94%, at least 95%, at least 96%, at least 97%, at least 98%, at least 99%, or 100%, to SEQ ID NO: 4; preferably the signal peptide comprises, consists essentially of, or consists of SEQ ID NO: 4.

In a 1st aspect 1C, the invention also relates to a nucleic acid construct comprising:

    a) a first polynucleotide encoding a signal peptide from a fungal prolyl dipeptidyl peptidase; and

    b) a second polynucleotide encoding a polypeptide of interest;

wherein the first polynucleotide and the second polynucleotide are operably linked in translational fusion.

In one embodiment, the nucleic acid construct further comprises a heterologous promoter, and wherein said promoter, the first polynucleotide, and the second polynucleotide are operably linked.

In one embodiment, the signal peptide is a naturally occurring signal peptide, or a functional fragment or functional variant of a naturally occurring signal peptide.

In a particular embodiment, the signal peptide is obtained from a lipase from *Aspergillus oryzae*.

In one embodiment, the first polynucleotide encoding the signal peptide has a sequence identity of at least 80%, *e.g.* at least 85%, at least 90%, at least 91%, at least 92%, at least 93%, at least 94%, at least 95%, at least 96%, at least 97%, at least 98%, at least 99%, or 100%, to the mature polypeptide coding sequence of SEQ ID NO: 177; most preferably the polynucleotide comprises, consists essentially of, or consists of the mature polypeptide coding sequence of SEQ ID NO: 177.

In one embodiment, the signal peptide has a sequence identity of at least 80%, e.g. at least 85%, at least 90%, at least 91%, at least 92%, at least 93%, at least 94%, at least 95%, at least 96%, at least 97%, at least 98%, at least 99%, or 100%, to SEQ ID NO: 171; preferably the signal peptide comprises, consists essentially of, or consists of SEQ ID NO: 171.

In one embodiment, the signal peptide is obtained from a prolyl dipeptidyl peptidase from *Aspergillus oryzae.*

In one embodiment, the first polynucleotide encoding the signal peptide has a sequence identity of at least 80%, e.g. at least 85%, at least 90%, at least 91%, at least 92%, at least 93%, at least 94%, at least 95%, at least 96%, at least 97%, at least 98%, at least 99%, or 100%, to the mature polypeptide coding sequence of SEQ ID NO: 178; most preferably the polynucleotide comprises, consists essentially of, or consists of the mature polypeptide coding sequence of SEQ ID NO: 178.

In one embodiment, the signal peptide has a sequence identity of at least 80%, e.g. at least 85%, at least 90%, at least 91%, at least 92%, at least 93%, at least 94%, at least 95%, at least 96%, at least 97%, at least 98%, at least 99%, or 100%, to SEQ ID NO: 172; preferably the signal peptide comprises, consists essentially of, or consists of SEQ ID NO: 172.

In one embodiment, the N- and/or C-terminal end of the signal peptide has been extended by addition of one or more amino acids.

In one embodiment, the signal peptide is a fragment of the signal peptides of any of the previous embodiments of this aspect.

In one embodiment, the polypeptide of interest is selected from the list of a hydrolase, isomerase, ligase, lyase, lysozyme, oxidoreductase, or transferase, e.g., an aminopeptidase, amylase, carbohydrase, carboxypeptidase, catalase, cellobiohydrolase, cellulase, chitinase, cutinase, cyclodextrin glycosyltransferase, deoxyribonuclease, endoglucanase, esterase, alpha-galactosidase, beta-galactosidase, glucoamylase, alpha-glucosidase, beta-glucosidase, invertase, laccase, lipase, mannosidase, mutanase, nuclease, oxidase, pectinolytic enzyme, peroxidase, phosphodiesterase, phytase, polyphenoloxidase, proteolytic enzyme, ribonuclease, transglutaminase, xylanase, beta-xylosidase, or a protease having protease activity.

In one embodiment, the polypeptide of interest is a fungal polypeptide, preferably a filamentous fungal polypeptide.

In one embodiment, the polypeptide of interest is a endoglucanase.

In one embodiment, the polypetpdie of interest is a phytase.

In one embodiment, the polypeptide of interest is a microbial polypeptide; preferably a bacterial polypeptide.

In one embodiment, the polypeptide of interest is a protease having protease activity obtained from *Bacillus licheniformis*, *Bacillus subtilis*, *Nocardiopsis prasina or Bacillus clausii.* In a particular embodiment, the protease having protease activity is obtained from *Bacillus clausii.*

In one embodiment, the protease has a sequence identity of at least 80%, e.g. at least 85%, at least 90%, at least 91%, at least 92%, at least 93%, at least 94%, at least 95%, at least 96%, at least 97%, at least 98%, at least 99%, or 100%, to the mature polypeptide of SEQ ID NO: 16, preferably the protease comprises, consists essentially of, or consists of the mature polypeptide of SEQ ID NO: 16.

In another embodiment, the N- and/or C-terminal end of the polypeptide of interest and/or signal peptide has been extended by addition of one or more amino acids. In one embodiment, the protease or polypeptide of interest is a fragment of protease or polypeptide of interest according to any of the embodiments of this aspect, respectively.

Promoters

The control sequence may be a promoter, a polynucleotide that is recognized by a host cell for expression of a polynucleotide encoding a polypeptide of the present invention. The promoter contains transcriptional control sequences that mediate the expression of the polypeptide. The promoter may be any polynucleotide that shows transcriptional activity in the host cell including mutant, truncated, and hybrid promoters, and may be obtained from genes encoding extracellular or intracellular polypeptides either homologous or heterologous to the host cell.

Examples of suitable promoters for directing transcription of the polynucleotide of the present invention in a bacterial host cell are described in Sambrook et al., 1989, Molecular Cloning: A Laboratory Manual, Cold Spring Harbor Lab., NY, Davis *et al.*, 2012, *supra*, and Song et al., 2016, PLOS One 11(7): e0158447.

Examples of suitable promoters for directing transcription of the polynucleotide of the present invention in a filamentous fungal host cell are promoters obtained from *Aspergillus*, *Fusarium*, *Rhizomucor* and *Trichoderma* cells, such as the promoters described in Mukherjee et al., 2013, "Trichoderma: Biology and Applications", and by Schmoll and Dattenböck, 2016, "Gene Expression Systems in Fungi: Advancements and Applications", Fungal Biology.

For expression in a yeast host, examples of useful promoters are described by Smolke et al., 2018, "Synthetic Biology: Parts, Devices and Applications" (Chapter 6: Constitutive and Regulated Promoters in Yeast: How to Design and Make Use of Promoters in S. cerevisiae), and by Schmoll and Dattenböck, 2016, "Gene Expression Systems in Fungi: Advancements and Applications", Fungal Biology.

Examples of suitable promoters in mammlian cells are the CMV (cytomegalovirus) promoter, SV40 promoter, or promoters described in The FANTOM Consortium and the RIKEN PMI and CLST (DGT). Nature 507, 462-470 (2014). https://doi.org/10.1038/nature13182, or promoters described in Romanova N, Noll T. Biotechnol J. 2018 Mar;13(3):e1700232. doi: 10.1002/biot.201700232. Epub 2017 Dec 7. PMID: 29145694.

Terminators

The control sequence may also be a transcription terminator, which is recognized by a host cell to terminate transcription. The terminator is operably linked to the 3'-terminus of the polynucleotide encoding the polypeptide. Any terminator that is functional in the host cell may be used in the present invention.

Preferred terminators for bacterial host cells may be obtained from the genes for *Bacillus clausii* alkaline protease (*aprH*), *Bacillus licheniformis* alpha-amylase (*amyL*), and *Escherichia coli* ribosomal RNA (*rrnB*).

Preferred terminators for filamentous fungal host cells may be obtained from *Aspergillus* or *Trichoderma* species, such as obtained from the genes for *Aspergillus niger* glucoamylase, *Trichoderma reesei* beta-glucosidase, *Trichoderma reesei* cellobiohydrolase I, and *Trichoderma reesei* endoglucanase I, such as the terminators described in Mukherjee et al., 2013, "Trichoderma: Biology and Applications", and by Schmoll and Dattenböck, 2016, "Gene Expression Systems in Fungi: Advancements and Applications", Fungal Biology.

Preferred terminators for yeast host cells may be obtained from the genes for *Saccharomyces cerevisiae* enolase, *Saccharomyces cerevisiae* cytochrome C (CYC1), and *Saccharomyces cerevisiae* glyceraldehyde-3-phosphate dehydrogenase. Other useful terminators for yeast host cells are described by Romanos et al., 1992, Yeast 8: 423-488.

mRNA Stabilizers

The control sequence may also be an mRNA stabilizer region downstream of a promoter and upstream of the coding sequence of a gene which increases expression of the gene.

Examples of suitable mRNA stabilizer regions are obtained from a *Bacillus thuringiensis cryIIIA* gene (WO 94/25612) and a *Bacillus subtilis* SP82 gene (Hue et al., 1995, J. Bacteriol. 177: 3465-3471).

Examples of mRNA stabilizer regions for fungal cells are described in Geisberg et al., 2014, Cell 156(4): 812-824, and in Morozov et al., 2006, Eukaryotic Cell 5(11): 1838-1846.

Leader Sequences

The control sequence may also be a leader, a non-translated region of an mRNA that is important for translation by the host cell. The leader is operably linked to the 5'-terminus of the polynucleotide encoding the polypeptide. Any leader that is functional in the host cell may be used.

Suitable leaders for bacterial host cells are described by Hambraeus et al., 2000, Microbiology 146(12): 3051-3059, and by Kaberdin and Bläsi, 2006, FEMS Microbiol. Rev. 30(6): 967-979.

Preferred leaders for filamentous fungal host cells may be obtained from the genes for *Aspergillus oryzae* TAKA amylase and *Aspergillus nidulans* triose phosphate isomerase.

Suitable leaders for yeast host cells may be obtained from the genes for *Saccharomyces cerevisiae* enolase (ENO-1), *Saccharomyces cerevisiae* 3-phosphoglycerate kinase, *Saccharomyces cerevisiae* alpha-factor, and *Saccharomyces cerevisiae* alcohol dehydrogenase/glyceraldehyde-3-phosphate dehydrogenase (ADH2/GAP).

Polyadenylation Sequences

The control sequence may also be a polyadenylation sequence, a sequence operably linked to the 3'-terminus of the polynucleotide which, when transcribed, is recognized by the host cell as a signal to add polyadenosine residues to transcribed mRNA. Any polyadenylation sequence that is functional in the host cell may be used.

Preferred polyadenylation sequences for filamentous fungal host cells are obtained from the genes for *Aspergillus nidulans* anthranilate synthase, *Aspergillus niger* glucoamylase, *Aspergillus niger* alpha-glucosidase, *Aspergillus oryzae* TAKA amylase, and *Fusarium oxysporum* trypsin-like protease.

Useful polyadenylation sequences for yeast host cells are described by Guo and Sherman, 1995, Mol. Cellular Biol. 15: 5983-5990.

Signal Peptides

The control sequence may also be a signal peptide coding region that encodes a signal peptide linked to the N-terminus of the SP-linker, or the N-terminus of the polypeptide of interest and directs the polypeptide of interest into the cell's secretory pathway. The 5'-end of the coding sequence of the polynucleotide may inherently contain a signal peptide coding sequence naturally linked in translation reading frame with the segment of the coding sequence that encodes the polypeptide. Alternatively, the 5'-end of the coding sequence may contain a signal peptide coding sequence that is heterologous to the coding sequence. A heterologous signal peptide coding sequence may be required where the coding sequence does not naturally contain a signal peptide coding sequence. Alternatively, a heterologous signal peptide coding sequence may simply replace the natural signal peptide coding sequence to enhance secretion of the polypeptide. Any signal peptide coding sequence that directs the expressed polypeptide into the secretory pathway of a host cell may be used.

Effective signal peptide coding sequences for bacterial host cells are the signal peptide coding sequences obtained from the genes for *Bacillus* NCIB 11837 maltogenic amylase, *Bacillus licheniformis* subtilisin, *Bacillus licheniformis* beta-lactamase, *Bacillus stearothermophilus* alpha-amylase, *Bacillus stearothermophilus* neutral proteases (*nprT, nprS, nprM*), and *Bacillus subtilis prsA.* Further signal peptides are described by Freudl, 2018, Microbial Cell Factories 17: 52. Non-limiting examples for signal peptides are SEQ ID NO: 3 and SEQ ID NO: 4.

Effective signal peptide coding sequences for filamentous fungal host cells are the signal peptide coding sequences obtained from the genes for *Aspergillus niger* neutral amylase, *Aspergillus niger* glucoamylase, *Aspergillus oryzae* TAKA amylase, *Humicola insolens* cellulase, *Humicola insolens* endoglucanase V, *Humicola lanuginosa* lipase, and *Rhizomucor miehei* aspartic proteinase, such as the signal peptide described by Xu et al., 2018, Biotechnology Letters 40: 949-955. Non-limiting examples for signal peptides are SEQ ID NO: 171, and SEQ ID NO: 172.

Useful signal peptides for yeast host cells are obtained from the genes for *Saccharomyces cerevisiae* alpha-factor and *Saccharomyces cerevisiae* invertase. Other useful signal peptide coding sequences are described by Romanos *et al.*, 1992, *supra*.

Useful signal peptides for mammalian host cells are described in Kober, Lars & Zehe, Christoph & Bode, Juergen. (2013). Biotechnology and bioengineering. 110. 10.1002/bit.24776,

ATTALLAH, Carolina, et al. Protein Expression and Purification, 2017, 132: 27-33, and STERN, Beate, et al. Trends Cell Mol Biol, 2007, 2: 1-17.

Propeptides

The control sequence may also be a propeptide coding sequence that encodes a propeptide positioned at the N-terminus of a polypeptide of interest. The resultant polypeptide is known as a proenzyme or propolypeptide (or a zymogen in some cases). A propolypeptide is generally inactive and can be converted to an active polypeptide by catalytic or autocatalytic cleavage of the propeptide from the propolypeptide. The propeptide coding sequence may be obtained from the genes for *Bacillus subtilis* alkaline protease (*aprE*), *Bacillus subtilis* neutral protease (*nprT*), *Myceliophthora thermophila* laccase (WO 95/33836), *Rhizomucor miehei* aspartic proteinase, and *Saccharomyces cerevisiae* alpha-factor.

Where both signal peptide and propeptide sequences are present, the propeptide sequence is positioned next to the N-terminus of a polypeptide and the signal peptide sequence is positioned next to the N-terminus of the propeptide sequence. Additially or alternatively, where signal peptide, SP-linker and propeptide sequences are present, the propeptide sequence is positioned next to the N-terminus of a polypeptide, the SP-linker is positioned next to the N-terminus of the propeptide, and the signal peptide sequence is positioned next to the N-terminus of the SP-linker sequence. Additionally or alternatively, when both signal peptide and propeptide and/or SP-linker sequences are present, the polypeptide of interest may comprise only a part of the signal peptide sequence and/or only a part of the propeptide sequence and/or only a part of the SP-linker sequence. Alternatively, the final or isolated polypeptide of interest may comprise a mixture of mature polypeptides and polypeptides which comprise, either partly or in full length, a propeptide sequence and/or a signal peptide sequence and/or a SP-linker sequence.

Regulatory Sequences

It may also be desirable to add regulatory sequences that regulate expression of the polypeptide relative to the growth of the host cell. Examples of regulatory sequences are those that cause expression of the gene to be turned on or off in response to a chemical or physical stimulus, including the presence of a regulatory compound. Regulatory sequences in prokaryotic systems include the *lac*, *tac*, and *trp* operator systems. In yeast, the ADH2 system or GAL1 system may be used. In filamentous fungi, the *Aspergillus niger* glucoamylase promoter, *Aspergillus oryzae* TAKA alpha-amylase promoter, and *Aspergillus oryzae* glucoamylase promoter, *Trichoderma reesei* cellobiohydrolase I promoter, and *Trichoderma reesei* cellobiohydrolase II promoter may be used. Other examples of regulatory sequences are those that allow for gene amplification. In fungal systems, these regulatory sequences include the dihydrofolate reductase gene that is amplified in the presence of methotrexate, and the metallothionein genes that are amplified with heavy metals.

**Expression Vectors**

The present invention also relates to recombinant expression vectors comprising a polynucleotide of the present invention, a promoter, and transcriptional and translational stop signals. The various nucleotide and control sequences may be joined together to produce a recombinant expression vector that may include one or more convenient restriction sites to allow for insertion or substitution of the polynucleotide encoding the polypeptide at such sites. Alternatively, the polynucleotide may be expressed by inserting the polynucleotide or a nucleic acid construct comprising the polynucleotide into an appropriate vector for expression. In creating the expression vector, the coding sequence is located in the vector so that the coding sequence is operably linked with the appropriate control sequences for expression.

The recombinant expression vector may be any vector (e.g., a plasmid or virus) that can be conveniently subjected to recombinant DNA procedures and can bring about expression of the polynucleotide. The choice of the vector will typically depend on the compatibility of the vector with the host cell into which the vector is to be introduced. The vector may be a linear or closed circular plasmid.

The vector may be an autonomously replicating vector, *i.e.*, a vector that exists as an extrachromosomal entity, the replication of which is independent of chromosomal replication, *e.g.*, a plasmid, an extrachromosomal element, a minichromosome, or an artificial chromosome. The vector may contain any means for assuring self-replication. Alternatively, the vector may be one that, when introduced into the host cell, is integrated into the genome and replicated together with the chromosome(s) into which it has been integrated. Furthermore, a single vector or plasmid or two or more vectors or plasmids that together contain the total DNA to be introduced into the genome of the host cell, or a transposon, may be used.

The vector preferably contains one or more selectable markers that permit easy selection of transformed, transfected, transduced, or the like cells. A selectable marker is a gene the product of which provides for biocide or viral resistance, resistance to heavy metals, prototrophy to auxotrophs, and the like.

The vector preferably contains at least one element that permits integration of the vector into the host cell's genome or autonomous replication of the vector in the cell independent of the genome.

For integration into the host cell genome, the vector may rely on the polynucleotide's sequence encoding the polypeptide or any other element of the vector for integration into the genome by homologous recombination, such as homology-directed repair (HDR), or non-homologous recombination, such as non-homologous end-joining (NHEJ).

For autonomous replication, the vector may further comprise an origin of replication enabling the vector to replicate autonomously in the host cell in question. The origin of replication may be any plasmid replicator mediating autonomous replication that functions in a cell. The term "origin of replication" or "plasmid replicator" means a polynucleotide that enables a plasmid or vector to replicate *in vivo.*

More than one copy of a polynucleotide of the present invention may be inserted into a host cell to increase production of a polypeptide. For example, 2 or 3 or 4 or 5 or more copies are inserted into a host cell. An increase in the copy number of the polynucleotide can be obtained by integrating at least one additional copy of the sequence into the host cell genome or by including an amplifiable selectable marker gene with the polynucleotide where cells containing amplified copies of the selectable marker gene, and thereby additional copies of the polynucleotide, can be selected for by cultivating the cells in the presence of the appropriate selectable agent.

In a second aspect, the expression vector comprises a nucleic acid construct according to the first aspect 1A, or the first aspect 1B, or the first aspect 1C.

**Host Cells**

The present invention also relates to recombinant host cells, comprising a polynucleotide of the present invention operably linked to one or more control sequences that direct the production of a polypeptide of the present invention.

A construct or vector comprising a polynucleotide is introduced into a host cell so that the construct or vector is maintained as a chromosomal integrant or as a self-replicating extrachromosomal vector as described earlier. The choice of a host cell will to a large extent depend upon the gene encoding the polypeptide and its source.

For purposes of this invention, *Bacillus* classes/genera/species shall be defined as described in Patel and Gupta, 2020, Int. J. Syst. Evol. Microbiol. 70: 406-438.

Methods for introducing DNA into prokaryotic host cells are well-known in the art, and any suitable method can be used including but not limited to protoplast transformation, competent cell transformation, electroporation, conjugation, transduction, with DNA introduced as linearized or as circular polynucleotide. Persons skilled in the art will be readily able to identify a suitable method for introducing DNA into a given prokaryotic cell depending, *e.g.*, on the genus. Methods for introducing DNA into prokaryotic host cells are for example described in Heinze et al., 2018, BMC Microbiology 18:56, Burke et al., 2001, Proc. Natl. Acad. Sci. USA 98: 6289-6294, Choi et al., 2006, J. Microbiol. Methods 64: 391-397, and Donald et al., 2013, J. Bacteriol. 195(11): 2612-2620. Methods for introducing DNA into mammalian cells are described in DEROUAZI, Madiha, et al. Biotechnology and bioengineering, 2004, 87.4: 537-545, and PHAM, Phuong Lan; KAMEN, Amine; DUROCHER, Yves. Molecular biotechnology, 2006, 34.2: 225-237.

The host cell may be a fungal cell. "Fungi" as used herein includes the phyla Ascomycota, Basidiomycota, Chytridiomycota, and Zygomycota as well as the Oomycota and all mitosporic fungi (as defined by Hawksworth et al., In, Ainsworth and Bisby's Dictionary of The Fungi, 8th edition, 1995, CAB International, University Press, Cambridge, UK).

Fungal cells may be transformed by a process involving protoplast-mediated transformation, Agrobacterium-mediated transformation, electroporation, biolistic method and shock-wave-mediated transformation as reviewed by Li et al., 2017, Microbial Cell Factories 16: 168 and procedures described in EP 238023, Yelton et al., 1984, Proc. Natl. Acad. Sci. USA 81: 1470-1474, Christensen et al., 1988, Bio/Technology 6: 1419-1422, and Lubertozzi and Keasling, 2009, Biotechn. Advances 27: 53-75. However, any method known in the art for introducing DNA into a fungal host cell can be used, and the DNA can be introduced as linearized or as circular polynucleotide.

The fungal host cell may be a yeast cell. "Yeast" as used herein includes ascosporogenous yeast (Endomycetales), basidiosporogenous yeast, and yeast belonging to the Fungi Imperfecti (Blastomycetes). For purposes of this invention, yeast shall be defined as described in Biology and Activities of Yeast (Skinner, Passmore, and Davenport, editors, Soc. App. Bacteriol. Symposium Series No. 9, 1980).

In a third aspect 3A, the invention relates to a host cell producing a polypeptide of interest and comprising in its genome:

a) a nucleic acid construct according to the first aspect 1A; and/or
b) an expression vector comprising a nucleic acid construct according to the first aspect 1A.

In one embodiment, the host cell is a bacterial host cell, a fungal host cell, or a eukaryotic host cell, preferably a *Bacillus* host cell, a *Trichoderma* host cell, an *Aspergillus* host cell, a *Pichia* host cell, a *Saccharomyces* host cell, or a mammalian host cell.

In one embodiment, the host cell is a yeast recombinant host cell, *e.g.,* a *Candida, Hansenula, Kluyveromyces, Pichia, Saccharomyces, Schizosaccharomyces,* or *Yarrowia* cell, such as a *Kluyveromyces lactis, Saccharomyces carlsbergensis, Saccharomyces cerevisiae, Saccharomyces diastaticus, Saccharomyces douglasii, Saccharomyces kluyveri, Saccharomyces norbensis, Saccharomyces oviformis,* or *Yarrowia lipolytica* cell.

In one embodiment the host cell is a filamentous fungal recombinant host cell, e.g., an *Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes,* or *Trichoderma* cell, in particular, an *Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpuroge-*

*num, Phanerochaete chrysosporium, Phlebia radiata, Pleurotus eryngii, Talaromyces emersonii, Thielavia terrestris, Trametes villosa, Trametes versicolor, Trichoderma harzianum, Trichoderma koningii, Trichoderma longibrachiatum, Trichoderma reesei, or Trichoderma viride* cell.

In one embodiment the host cell is an *Aspergillus oryzae* cell.

In one embodiment, the host cell is a prokaryotic recombinant host cell, e.g., a Gram-positive cell selected from the group consisting of *Bacillus, Clostridium, Enterococcus, Geobacillus, Lactobacillus, Lactococcus, Oceanobacillus, Staphylococcus, Streptococcus,* or *Streptomyces* cells, or a Gram-negative bacteria selected from the group consisting of *Campylobacter, E. coli, Flavobacterium, Fusobacterium, Helicobacter, Ilyobacter, Neisseria, Pseudomonas, Salmonella,* and *Ureaplasma* cells, such as *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis, Bacillus thuringiensis, Streptococcus equisimilis, Streptococcus pyogenes, Streptococcus uberis,* and *Streptococcus equi* subsp. *Zooepidemicus, Streptomyces achromogenes, Streptomyces avermitilis, Streptomyces coelicolor, Streptomyces griseus,* and *Streptomyces lividans* cells.

In a preferred embodiment, the host cell is a *Bacillus* cell, preferably a *Bacillus subtilis* cell, more preferably a *Bacillus licheniformis* cell.

In another preferred embodiment, the host cell is a yeast cell, *e.g.* a *Candida, Hansenula, Kluyveromyces, Pichia, Saccharomyces, Schizosaccharomyces,* or *Yarrowia* cell, such as a *Kluyveromyces lactis, Saccharomyces carlsbergensis, Saccharomyces cerevisiae, Saccharomyces diastaticus, Saccharomyces douglasii, Saccharomyces kluyveri, Saccharomyces norbensis, Saccharomyces oviformis,* or *Yarrowia lipolytica* cell,

In another preferred embodiment, the host cell is a filamentous fungal recombinant host cell, *e.g.,* an *Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes,* or *Trichoderma* cell, in particular, an *Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Talaromyces emersonii, Thielavia terrestris, Trametes villosa, Trametes versicolor, Trichoderma harzianum, Trichoderma koningii, Trichoderma longibrachiatum, Trichoderma reesei,* or *Trichoderma viride* cell.

In another preferred embodiment, the host cell is a mammalian cell selected from the list of a Chinese Hamster Ovary cell (CHO cell), human embryo kidney cell (HEK cell), mouse cell, mouse myeloma cell (NS0), baby hamster kidney cell (BHK cell), or human retinal cells.

In one embodiment, the host cell is isolated.

In one embodiment, the host cell is purified.

In one embodiment, the first donor polypeptide is endogenous to the host cell.

In one embodiment the signal peptide is endogenous to the host cell.

In one embodiment, the second donor polypeptide is endogenous to the host cell.

In one embodiment, the SP-linker is endogenous to the host cell.

In one embodiment, the polypeptide of interest is heterologous to the host cell.

In another embodiment, the host cell comprises at least two copies, e.g., three, four, or five, or more copies of the nucleic acid construct according to the first aspect 1A.

In one embodiment, a yield or secretion of the polypeptide of interest by the host cell is increased by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 100% relative to a yield or secretion of the polypeptide of interest expressed by a parent host cell lacking the second polynucleotide encoding the SP-linker when cultivated under identical conditions, the parent host cell otherwise being isogenic to the host cell according to the previous host cell embodiments.

In a third aspect 3B, the invention relates to a bacterial host cell producing a polypeptide of interest and comprising in its genome:

a) a nucleic acid construct according to the first aspect 1B; and/or

b) an expression vector comprising ca nucleic acid construct according to the first aspect 1B.

In one embodiment, the bacterial host cell is a Gram-positive host cell.

In one embodiment, the bacterial host cell is a *Bacillus* cell selected from the group consisting of *Bacillus alkalophilus*, *Bacillus amyloliquefaciens*, *Bacillus brevis*, *Bacillus circulans*, *Bacillus clausii*, *Bacillus coagulans*, *Bacillus firmus*, *Bacillus lautus*, *Bacillus lentus*, *Bacillus licheniformis*, *Bacillus megaterium*, *Bacillus pumilus*, *Bacillus stearothermophilus*, *Bacillus subtilis*, and *Bacillus thuringiensis* cell.

In a particular embodiment, the bacterial host cell is a *Bacillus* cell.

In another embodiment, the bacterial host cell is a *Bacillus licheniformis* cell.

In one embodiment, the host cell comprises at least two copies of the nucleic acid construct and/or the expression vector, such as two copies, three copies, four copies or more than four copies.

In one embodiment, a yield of the polypeptide of interest expressed by the host cell is increased by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 120%, at least 150%, at least 170%, at least 200%, at least 220%, at least 250%, or at least 270% relative to a yield of the polypeptide of interest expressed by a parent host cell with an alternative signal peptide when cultivated under identical conditions, the parent host cell otherwise being isogenic to the host cell according to any of the embodiments of the third aspect 3B. In one embodiment, the alternative signal peptide comprises or consists of an amino acid sequence having a sequence identity of at least 80%, e.g. at least 85%, at least 90%, at least 91%, at least 92%, at least 93%, at least 94%, at least 95%, at least 96%, at least 97%, at least 98%, at least 99%, or 100%, to the amino acid sequence of SEQ ID NO: 81.

In a third aspect 3C, the invention relates to a fungal host cell producing a polypeptide of interest and comprising in its genome:

a) a nucleic acid construct according to the first aspect 1C; and/or
b) an expression vector comprising ca nucleic acid construct according to the first aspect 1C.

In one embodiment, the fungal host cell is a filamentous fungal host cell.

In another preferred embodiment, the host cell is a filamentous fungal recombinant host cell, *e.g.*, an *Acremonium*, *Aspergillus*, *Aureobasidium*, *Bjerkandera*, *Ceriporiopsis*, *Chrysosporium*, *Coprinus*, *Coriolus*, *Cryptococcus*, *Filibasidium*, *Fusarium*, *Humicola*, *Magnaporthe*, *Mucor*, *Myceliophthora*, *Neocallimastix*, *Neurospora*, *Paecilomyces*, *Penicillium*, *Phanerochaete*, *Phlebia*, *Piromyces*, *Pleurotus*, *Schizophyllum*, *Talaromyces*, *Thermoascus*, *Thielavia*, *Tolypocladium*, *Trametes*, or *Trichoderma* cell, in particular, an *Aspergillus awamori*, *Aspergillus foetidus*, *Aspergillus fumigatus*, *Aspergillus japonicus*, *Aspergillus nidulans*, *Aspergillus niger*, *Aspergillus oryzae*, *Bjerkandera adusta*, *Ceriporiopsis aneirina*, *Ceriporiopsis caregiea*, *Ceriporiopsis gilvescens*, *Ceriporiopsis pannocinta*, *Ceriporiopsis rivulosa*, *Ceriporiopsis subrufa*, *Ceriporiopsis subvermispora*, *Chrysosporium inops*, *Chrysosporium keratinophilum*, *Chrysosporium lucknowense*, *Chrysosporium merdarium*, *Chrysosporium pannicola*, *Chrysosporium queenslandicum*, *Chrysosporium tropicum*, *Chrysosporium zonatum*, *Coprinus cinereus*, *Coriolus hirsutus*, *Fusarium bactridioides*, *Fusarium cerealis*, *Fusarium crookwellense*, *Fusarium culmorum*, *Fusarium graminearum*, *Fusarium graminum*, *Fusarium heterosporum*, *Fusarium negundi*, *Fusarium oxysporum*, *Fusarium reticulatum*, *Fusarium roseum*, *Fusarium sambucinum*, *Fusarium sarcochroum*, *Fusarium sporotrichioides*, *Fusarium sulphureum*, *Fusarium torulosum*, *Fusarium trichothecioides*, *Fusarium venenatum*, *Humicola insolens*, *Humicola lanuginosa*, *Mucor miehei*, *Myceliophthora thermophila*, *Neurospora crassa*, *Penicillium purpurogenum*, *Phanerochaete chrysosporium*, *Phlebia radiata*, *Pleurotus eryngii*, *Talaromyces emersonii*, *Thielavia terrestris*, *Trametes villosa*, *Trametes versicolor*, *Trichoderma harzianum*, *Trichoderma koningii*, *Trichoderma longibrachiatum*, *Trichoderma reesei*, or *Trichoderma viride* cell.

In one embodiment the filamentous fungal host cell is an *Aspergillus* host cell.

In one embodiment the filamentous fungal host cell is an *Aspergillus orzyae* host cell.

In one embodiment the filamentous fungal host cell is an *Aspergillus niger* host cell.

In one embodiment, the host cell comprises at least two copies of the nucleic acid construct and/or the expression vector, such as two copies, three copies, four copies or more than four copies.

In one embodiment, a yield of the polypeptide of interest expressed by the host cell is increased by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 120%, at least 150%, at least 170%, at least 200%, at least 220%, at least 250%, or at least 270% relative to a yield of the polypeptide of interest expressed by a parent host cell with an alternative signal peptide when cultivated under identical conditions, the parent host cell otherwise being isogenic to the host cell according to any of the embodiments of the third aspect 3C.

**Methods of Production**

In a fourth aspect, the invention relates to a method of producing a polypeptide of interest, the method comprising:

a) cultivating a host cell according to the third aspect 3A and/or 3B and/or 3C under conditions conducive for production of the polypeptide of interest; and optionally
b) recovering the polypeptide of interest.

In one embodiment, the method further comprises step c) maturing the polypeptide of interest by contacting the polypeptide of interest with a protease or peptidase configured to cleave off the pro-peptide and/or SP-linker from the mature polypeptide of interest, wherein step c) is carried out prior, after, or in parallel to step b).

In one embodiment, the cultivation is a batch cultivation, a fed-batch cultivation, perfusion cultivation, or a continuous cultivation.

In one embodiment the polypeptide of interest has phytase activity.

In one embodiment the polypeptide of interest has protease activity.

In one embodiment, the polypeptide of interest hast endoglucanase activity.

In one embodiment, the polypeptide of interest has amylase activity.

In one embodiment, the cultivation has a duration of at least 24 hours, at least 48 hours, at least 72 hours, at least 96 hours, at least 120 hours, at least 144 hours, at least 168 hours, at least 192 hours, at least 216 hours, at least 240 hours, at least 264 hours, at least 288 hours, at least 312 hours, at least 336 hours, or at least 360 hours.

In one embodiment, the yield of the polypeptide of interest is increased by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 100% relative to a yield of the polypeptide of interest expressed by a parent host cell lacking the second polynucleotide encoding the SP-linker when cultivated under identical conditions, the parent host cell otherwise being isogenic to the host cell according to the third aspect 3A.

Another aspect of the invention relates to a method of producing a polypeptide of interest, the method comprising:

a) cultivating a bacterial host cell according to the third aspect 3B under conditions conducive for production of the polypeptide of interest; and optionally
b) recovering the polypeptide of interest.

In one embodiment the polypeptide of interest has protease activity.

In one embodiment, a yield of the polypeptide of interest is increased by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 120%, at least 150%, at least 170%, at least 200%, at least 220%, at least 250%, or at least 270% relative to a yield of the polypeptide of interest expressed by a parent host cell with an alternative signal peptide when cultivated under identical conditions, the parent host cell otherwise being isogenic to the host cell according to the third aspect 3B.

In one embodiment, the alternative signal peptide comprises or consists of an amino acid sequence having a sequence identity of at least 80%, e.g. at least 85%, at least 90%, at least 91%, at least 92%, at least 93%, at least 94%, at least 95%, at least 96%, at least 97%, at least 98%, at least 99%, or 100%, to the amino acid sequence of SEQ ID NO: 81.

Another aspect of the invention relates to a method of producing a polypeptide of interest, the method comprising:

a) cultivating a fungal host cell according to the third aspect 3C under conditions conducive for production of the polypeptide of interest; and optionally
b) recovering the polypeptide of interest.

In one embodiment the polypeptide of interest has phytase activity.

In one embodiment the polypeptide of interest has endoglucanase activity.

In one embodiment, a yield of the polypeptide of interest is increased by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 120%, at least 150%, at least 170%, at least 200%, at least 220%, at least 250%, or at least 270% relative to a yield of the polypeptide of interest expressed by a parent host cell with an alternative signal peptide when cultivated under identical conditions, the parent host cell otherwise being isogenic to the host cell according to the third aspect 3C.

The host cell is cultivated in a nutrient medium suitable for production of the polypeptide using methods known in the art. For example, the cell may be cultivated by shake flask cultivation, or small-scale or large-scale fermentation (including continuous, batch, fed-batch, or solid-state, and/or microcarrier-based fermentations) in laboratory or industrial fermentors in a suitable medium and under conditions allowing the polypeptide to be expressed and/or isolated. Suitable media are available from commercial suppliers or may be prepared according to published compositions (e.g., in catalogues of the American Type Culture Collection). If the polypeptide is secreted into the nutrient medium, the polypeptide can be recovered directly from the medium. If the polypeptide is not secreted, it can be recovered from cell lysates.

The polypeptide may be detected using methods known in the art that are specific for the polypeptide, including, but not limited to, the use of specific antibodies, formation of an enzyme product, disappearance of an enzyme substrate, or an assay determining the relative or specific activity of the polypeptide.

The polypeptide may be recovered from the medium using methods known in the art, including, but not limited to, collection, centrifugation, filtration, extraction, spray-drying, evaporation, or precipitation. In one aspect, a whole fermentation broth comprising the polypeptide is recovered. In another aspect, a cell-free fermentation broth comprising the polypeptide is recovered.

The polypeptide may be purified by a variety of procedures known in the art to obtain substantially pure polypeptides and/or polypeptide fragments (see, *e.g.*, Wingfield, 2015, Current Protocols in Protein Science; 80(1): 6.1.1-6.1.35; Labrou, 2014, Protein Downstream Processing, 1129: 3-10).

In an alternative aspect, the polypeptide is not recovered.

**Plants**

The present invention also relates to plants, e.g., a transgenic plant, plant part, or plant cell, comprising a polynucleotide of the present invention so as to express and produce a polypeptide in recoverable quantities.

In a fifth aspect, the invention also relates to a transgenic plant, plant part or plant cell transformed with the nucleic acid construct according to the first aspects.

In a sixth aspect, the invention also relates to a method of producing a polypeptide of interest, comprising cultivating the transgenic plant or plant cell of the fifth aspect under conditions conducive for production of the polypeptide.

In one embodiment, the method further comprises recovering the polypeptide.

The polypeptide may be recovered from the plant or plant part. Alternatively, the plant or plant part containing the polypeptide may be used as such for improving the quality of a food or feed, e.g., improving nutritional value, palatability, and rheological properties, or to destroy an antinutritive factor.

The transgenic plant can be dicotyledonous (a dicot) or monocotyledonous (a monocot). Examples of monocot plants are grasses, such as meadow grass (blue grass, Poa), forage grass such as Festuca, Lolium, temperate grass, such as Agrostis, and cereals, e.g., wheat, oats, rye, barley, rice, sorghum, and maize (corn).

Examples of dicot plants are tobacco, legumes, such as lupins, potato, sugar beet, pea, bean and soybean, and cruciferous plants (family Brassicaceae), such as cauliflower, rape seed, and the closely related model organism *Arabidopsis thaliana.*

Examples of plant parts are stem, callus, leaves, root, fruits, seeds, and tubers as well as the individual tissues comprising these parts, e.g., epidermis, mesophyll, parenchyme, vascular tissues, meristems. Specific plant cell compartments, such as chloroplasts, apoplasts, mitochondria, vacuoles, peroxisomes and cytoplasm are also considered to be a plant part. Furthermore, any plant cell, whatever the tissue origin, is considered to be a plant part. Likewise, plant parts such as specific tissues and cells isolated to facilitate the utilization of the invention are also considered plant parts, e.g., embryos, endosperms, aleurone and seed coats.

Also included within the scope of the present invention are the progeny of such plants, plant parts, and plant cells.

The transgenic plant or plant cell expressing the polypeptide may be constructed in accordance with methods known in the art. In short, the plant or plant cell is constructed by incorporating one or more expression constructs encoding the polypeptide into the plant host genome or chloroplast genome and propagating the resulting modified plant or plant cell into a transgenic plant or plant cell. In an embodiment, a plant cell does not belong to plant varieties.

The expression construct is conveniently a nucleic acid construct that comprises a polynucleotide encoding a polypeptide, wherein the polynucleotide is operably linked with appropriate regulatory sequences required for expression of the polynucleotide in the plant or plant part of choice. Furthermore, the expression construct may comprise a selectable marker useful for identifying plant cells into which the expression construct has been integrated and DNA sequences necessary for introduction of the construct into the plant in question (the latter depends on the DNA introduction method to be used).

The choice of regulatory sequences, such as promoter and terminator sequences and optionally signal or transit sequences, is determined, for example, on the basis of when, where, and how the polypeptide is desired to be expressed (Sticklen, 2008, Nature Reviews 9: 433-443). For instance, the expression of the gene encoding a polypeptide may be constitutive or inducible, or may be developmental, stage or tissue specific, and the gene product may be targeted to a specific tissue or plant part such as seeds or leaves. Regulatory sequences are, for example, described by Tague et al., 1988, Plant Physiology 86: 506.

For constitutive expression, the 35S-CaMV, the maize ubiquitin 1, or the rice actin 1 promoter may be used (Franck et al., 1980, Cell 21: 285-294; Christensen et al., 1992, Plant Mol. Biol. 18: 675-689; Zhang et al., 1991, Plant Cell 3: 1155-1165). Organ-specific promoters may be, for example, a promoter from storage sink tissues such as seeds, potato tubers, and fruits (Edwards and Coruzzi, 1990, Ann. Rev. Genet. 24: 275-303), or from metabolic sink tissues such as meristems (Ito et al., 1994, Plant Mol. Biol. 24: 863-878), a seed specific promoter such as the glutelin, prolamin, globulin, or albumin promoter from rice (Wu et al., 1998, Plant Cell Physiol. 39: 885-889), a *Vicia faba* promoter from the legumin B4 and the unknown seed protein gene from *Vicia faba* (Conrad et al., 1998, J. Plant Physiol. 152: 708-711), a promoter from a seed oil body protein (Chen et al., 1998, Plant Cell Physiol. 39: 935-941), the storage protein *napA* promoter from *Brassica napus,* or any other seed specific promoter known in the art, *e.g.*, as described in WO 91/14772. Furthermore, the promoter may be a leaf specific promoter such as the *rbcs* promoter from rice or tomato (Kyozuka et al., 1993, Plant Physiol. 102: 991-1000), the chlorella virus adenine methyltransferase gene promoter (Mitra and Higgins, 1994, Plant Mol. Biol. 26: 85-93), the *aldP* gene promoter from rice (Kagaya et al., 1995, Mol. Gen. Genet. 248: 668-674), or a wound inducible promoter such as the potato *pin2* promoter (Xu et al., 1993, Plant Mol. Biol. 22: 573-588). Likewise, the promoter may be induced by abiotic treatments such as temperature, drought, or alterations in salinity or induced by exogenously applied substances that activate the promoter, e.g., ethanol, oestrogens, plant hormones such as ethylene, abscisic acid, and gibberellic acid, and heavy metals.

A promoter enhancer element may also be used to achieve higher expression of a polypeptide in the plant. For instance, the promoter enhancer element may be an intron that is placed between the promoter and the polynucleotide encoding a polypeptide. For instance, Xu *et al.*, 1993, *supra*, disclose the use of the first intron of the rice actin 1 gene to enhance expression.

The selectable marker gene and any other parts of the expression construct may be chosen from those available in the art.

The nucleic acid construct is incorporated into the plant genome according to conventional techniques known in the art, including Agrobacterium-mediated transformation, virus-mediated transformation, microinjection, particle bombardment, biolistic transformation, and electroporation (Gasser et al., 1990, Science 244: 1293; Potrykus, 1990, BiolTechnology 8: 535; Shimamoto et al., 1989, Nature 338: 274).

*Agrobacterium tumefaciens*-mediated gene transfer is a method for generating transgenic dicots (for a review, see Hooykas and Schilperoort, 1992, Plant Mol. Biol. 19: 15-38) and for transforming monocots, although other transformation methods may be used for these plants. A method for generating transgenic monocots is particle bombardment (microscopic gold or tungsten particles coated with the transforming DNA) of embryonic calli or developing embryos (Christou, 1992, Plant J. 2: 275-281; Shimamoto, 1994, Curr. Opin. Biotechnol. 5: 158-162; Vasil et al., 1992, Bio/Technology 10: 667-674). An alternative method for transformation of monocots is based on protoplast transformation as described by Omirulleh et al., 1993, Plant Mol. Biol. 21: 415-428. Additional transformation methods include those described in U.S. Patent Nos. 6,395,966 and 7,151,204 (both of which are herein incorporated by reference in their entirety).

Following transformation, the transformants having incorporated the expression construct are selected and regenerated into whole plants according to methods well known in the art. Often the transformation procedure is designed for the selective elimination of selection genes either during regeneration or in the following generations by using, for example, co-transformation with two separate T-DNA constructs or site-specific excision of the selection gene by a specific recombinase.

In addition to direct transformation of a particular plant genotype with a construct of the present invention, transgenic plants may be made by crossing a plant having the construct to a second plant lacking the construct. For example, a construct encoding a polypeptide can be introduced into a particular plant variety by crossing, without the need for ever directly transforming a plant of that given variety. Therefore, the present invention encompasses not only a plant directly regenerated from cells which have been transformed in accordance with the present invention, but also the progeny of such plants. As used herein, progeny may refer to the offspring of any generation of a parent plant prepared in accordance with the present invention. Such progeny may include a DNA construct prepared in accordance with the present invention. Crossing results in the introduction of a transgene into a plant line by cross pollinating a starting line with a donor plant line. Non-limiting examples of such steps are described in U.S. Patent No. 7,151,204.

Plants may be generated through a process of backcross conversion. For example, plants include plants referred to as a backcross converted genotype, line, inbred, or hybrid.

Genetic markers may be used to assist in the introgression of one or more transgenes of the invention from one genetic background into another. Marker assisted selection offers advantages relative to conventional breeding in that it can be used to avoid errors caused by phenotypic variations. Further, genetic markers may provide data regarding the relative degree of elite germplasm in the individual progeny of a particular cross. For example, when a plant with a desired trait which otherwise has a non-agronomically desirable genetic background is crossed to an elite parent, genetic markers may be used to select progeny which not only possess the trait of interest, but also have a relatively large proportion of the desired germplasm. In this way, the number of generations required to introgress one or more traits into a particular genetic background is minimized.

**FUSION POLYPEPTIDES**

In a seventh aspect 7A, the invention also relates to a fusion polypeptide, comprising a polypeptide of interest and a SP-linker consisting of 2 to 15 amino acids, wherein the SP-linker is located at the N-terminal end of the polypeptide of interest and is heterologous to the polypeptide of interest.

The SP-linker and the polypeptide of interest are not expressed on two different polypeptide chains. Instead the second polynucleotide encoding the SP-linker, and the third polynucleotide encoding the polypeptide of interest are translationally fused so that the fusion polypeptide is expressed on a single polypeptide chain.

In one embodiment, the fusion polypeptide is secreted.

In one embodiment, the SP-linker consists of 2 to 10 amino acids preferably consists of 2 to 9 amino acids, 2 to 8 amino acids, 3 to 8 amino acids, 2 to 7 amino acids, 3 to 7 amino acids, 2to 6 amino acids, or 3 to 6 amino acids, 4 to 10 amino acids, 5 to 10 amino acids, 4 to 9 amino acids, 5 to 9 amino acids, 4 to 8 amino acids, or 5 to 8 amino acids.

In one embodiment, the SP-linker consists of 2 to 15 N-terminal amino acids from a second donor polypeptide.

In one embodiment, the second donor polypeptide is heterologous to the polypeptide of interest.

In one embodiment, the SP-linker is derived from a second donor polypeptide selected from the list consisting of a structural polypeptide, a receptor, a secreted polypeptide, a hormone, or a secreted enzyme, such as a hydrolase, isomerase, ligase, lyase, lysozyme, oxidoreductase, or transferase, e.g., an aminopeptidase, amylase, carbohydrase, carboxypeptidase, catalase, cellobiohydrolase, cellulase, chitinase, cutinase, cyclodextrin glycosyltransferase, deoxyribonuclease, endoglucanase, esterase, alpha-galactosidase, beta-galactosidase, glucoamylase, alpha-glucosidase, beta-glucosidase, invertase, laccase, lipase, mannosidase, mutanase, nuclease, oxidase, pectinolytic enzyme, peroxidase, phosphodiesterase, phytase, polyphenoloxidase, proteolytic enzyme, ribonuclease, transglutaminase, xylanase, aspartate phosphatase, or beta-xylosidase.

In a particular embodiment, the SP-linker is derived from a second donor polypeptide comprising or consisting of a protease.

In a particular embodiment, the SP-linker is derived from a second donor polypeptide comprising or consisting of a xylanase.

In a particular embodiment, the SP-linker is derived from a second donor polypeptide comprising or consisting of a endoglucanase.

In one embodiment, the SP-linker of the second donor polypeptide is derived from a bacterial cell or a eukaryotic cell.

In one embodiment, the SP-linker of the second donor polypeptide is derived from a bacterial cell, *e.g.*, a Grampositive cell selected from the group consisting of *Bacillus, Clostridium, Enterococcus, Geobacillus, Lactobacillus, Lactococcus, Oceanobacillus, Staphylococcus, Streptococcus*, or *Streptomyces* cells, or a Gram-negative bacteria selected from the group consisting of *Campylobacter, E. coli, Flavobacterium, Fusobacterium, Helicobacter, Ilyobacter, Neisseria, Pseudomonas, Salmonella*, and *Ureaplasma* cells, such as *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis, Bacillus thuringiensis, Streptococcus equisimilis, Streptococcus pyogenes, Streptococcus uberis*, and *Streptococcus equi* subsp. *Zooepidemicus, Streptomyces achromogenes, Streptomyces avermitilis, Streptomyces coelicolor, Streptomyces griseus*, and *Streptomyces lividans* cells.

In one embodiment, the SP-linker of the second donor polypeptide is derived from a *Bacillus* cell, such as from a *Bacillus subtilis* cell, a *Bacillus licheniformis* cell, or a *Bacillus pumilus* cell.

In one embodiment, the SP-linker of the second donor polypeptide is derived from a *Bacillus* cell or a *Nocardiopsis* cell, preferably from a *Bacillus clausii* cell, or a *Nocardiopsis prasina* cell.

In a particular embodiment, the SP-linker is derived from a second donor polypeptide comprising or consisting of an amino acid sequence having at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% sequence identity to the amino acid sequence of SEQ ID NO: 16 or 17.

In one embodiment, the SP-linker of the second donor polypeptide is derived from a eukaryotic cell selected from the list of a fungal cell, a filamentous fungal cell, a yeast cell, and a mammalian cell.

In one embodiment, the SP-linker of the second donor polypeptide is derived from a yeast cell, *e.g.* a *Candida, Hansenula, Kluyveromyces, Pichia, Saccharomyces, Schizosaccharomyces*, or *Yarrowia* cell, such as a *Kluyveromyces lactis, Saccharomyces carlsbergensis, Saccharomyces cerevisiae, Saccharomyces diastaticus, Saccharomyces douglasii, Saccharomyces kluyveri, Saccharomyces norbensis, Saccharomyces oviformis*, or *Yarrowia lipolytica* cell, or derived from a filamentous fungal recombinant host cell, *e.g.,* an *Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe,*

*Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes,* or *Trichoderma* cell, in particular, an *Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Talaromyces emersonii, Thielavia terrestris, Trametes villosa, Trametes versicolor, Trichoderma harzianum, Trichoderma koningii, Trichoderma longibrachiatum, Trichoderma reesei,* or *Trichoderma viride* cell.

In one embodiment, the SP-linker of the second donor polypeptide is derived from *Thermothielavioides terrestris*, such as a *Thermothielavioides terrestris* endoglucanase.

In one embodiment, the SP-linker of the second donor polypeptide is derived from *Thermomyces lanuginosus,* such as a *Thermomyces lanuginosus* xylanase.

In one embodiment, the SP-linker of the second donor polypeptide is derived from a *Pichia pastoris*, a *Trichoderma reesei*, a *Aspergillus niger*, or a *Aspergillus oryzae* cell.

In one embodiment, the SP-linker is a synthetic polypeptide. A synthetic SP-linker may be combined with a native, non-synthetic or with a synthetic signal peptide to achieve increased expression and/or secretion of the polypeptide of interest. Additionally or alternatively, a synthetic signal peptide may be combined with a native, non-synthetic or with a synthetic SP-linker to achieve increased expression and/or secretion of the polypeptide of interest.

In one embodiment, the SP-linker consists of two amino acids, three amino acids, four amino acids, 5 amino acids, 6 amino acids, 7 amino acids, 8 amino acids, 9 amino acids, 10 amino acids, 11 amino acids, 12 amino acids, 13 amino acids, 14 amino acids, or 15 amino acids.

In one embodiment, the SP-linker consists of 2 - 10 amino acids.

In one embodiment, the polypeptide of interest is selected from the list of a hydrolase, isomerase, ligase, lyase, lysozyme, oxidoreductase, or transferase, e.g., an aminopeptidase, amylase, carbohydrase, carboxypeptidase, catalase, cellobiohydrolase, cellulase, chitinase, cutinase, cyclodextrin glycosyltransferase, deoxyribonuclease, endoglucanase, esterase, alpha-galactosidase, beta-galactosidase, glucoamylase, alpha-glucosidase, beta-glucosidase, invertase, laccase, lipase, mannosidase, mutanase, nuclease, oxidase, pectinolytic enzyme, peroxidase, phosphodiesterase, phytase, polyphenoloxidase, proteolytic enzyme, ribonuclease, transglutaminase, xylanase, or beta-xylosidase.

In one embodiment, the SP-linker is present after maturation of the polypeptide of interest and/or the fusion polypeptide. In one embodiment, the polypeptide of interest comprises a pro-peptide.

In one embodiment, the pro-peptide is located at the N-terminal end of the amino acid sequence of the polypeptide of interest, and the SP-linker is located at the N-terminal end of the pro-peptide.

In one embodiment, the fusion polypeptide comprises a cleavage site located between the SP-linker and the polypeptide of interest, or between the SP-linker and the pro-peptide of the polypeptide of interest.

In one embodiment, the additional cleavage site is a proteolytic cleavage site.

In one embodiment, the additional cleavage site is recognized by a suitable protease, preferably a KexB protease.

In one embodiment, the additional cleavage site is a dibasic amino acid motif such as a Lys-Arg ("KR") or an Arg-Arg motif ("RR"), preferably the additional cleavage site is a KexB or Kex2 cleavage site. In one embodiment, the SP-linker is removed from the polypeptide of interest, preferably removed from the polypeptide of interest together with the pro-peptide.

In one embodiment, the SP-linker is selected from the group consisting of:

(a) a polypeptide having at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% sequence identity to the sequence of SEQ ID NO: 7 or 8, or "T", "TG", "TGA", or SEQ ID NO: 24 to 48, or "A", "AE", AEE", or SEQ ID NO: 52 to 76,or "S", "SH", "SHD", or SEQ ID NO: 83 to 97, or "T", "TP", "TPQ" or SEQ ID NO 101 to 126, or SEQ ID NO: 179 to 186, or "FTP", "FPA", "AS", or "ASG", or SEQ ID NO: 79;

(b) a polypeptide encoded by a polynucleotide having at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% sequence identity to the polypeptide coding sequence of SEQ ID NO: 5, SEQ ID NO: 6, SEQ ID NO: 175, or SEQ ID NO: 176;

(c) a polypeptide derived from a polypeptide sequence of SEQ ID NO: 7 or 8, or "T", "TG", "TGA", or SEQ ID NO: 24 to 48, or "A", "AE", AEE", or SEQ ID NO: 52 to 76,or "S", "SH", "SHD", or SEQ ID NO: 83 to 97, or "T", "TP", "TPQ" or SEQ ID NO 101 to 126, or SEQ ID NO: 179 to 186, or "FTP", "FPA", "AS", or "ASG", or SEQ ID NO: 79,

or

SEQ ID NO: 7 or 8, or "T", "TG", "TGA", or SEQ ID NO: 24 to 48, or "A", "AE", AEE", or SEQ ID NO: 52 to 76,or "S", "SH", "SHD", or SEQ ID NO: 83 to 97, or "T", "TP", "TPQ" or SEQ ID NO 101 to 126, or SEQ ID NO: 179 to 186, or "FTP", "FPA", "AS", or "ASG", or SEQ ID NO: 79 having 1-30 alterations, *e.g.,* substitutions, deletions and/or insertions at one or more positions, *e.g.,* 1 or 2 or 3 or 4 or 5 or 6 or 7 or 8 or 9 or 10 or 11 or 12 or 13 or 14 or 15 or 16 or 17 or 18 or 19 or 20 or 21 or 22 or 23 or 24 or 25 or 26 or 27 or 28 or 29 or 30 alterations, in particular substitutions;

(d) a polypeptide derived from the polypeptide of (a), (b), or (c), wherein the N- and/or C-terminal end of the polypeptide fragment has been extended by addition of one or more amino acids; and

(e) a fragment of the polypeptide of (a), (b), (c), or (d).

In one embodiment, the fusion polypeptide is selected from the group consisting of:

(a) a polypeptide having at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% sequence identity to SEQ ID NO: 14;

(b) a polypeptide having at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% sequence identity to SEQ ID NO: 15;

(c) a polypeptide encoded by a polynucleotide having at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% sequence identity to the mature polypeptide coding sequence of SEQ ID NO: 77-78; and

(d) a polypeptide derived from the polypeptide of (a), (b), or (c), wherein the N- and/or C-terminal end has been extended by addition of one or more amino acids, said polypeptide having amylase activity; and

(e) a fragment of the polypeptide of (a), (b), or (c);

wherein the polypeptide has amylase activity.

In one embodiment, the fusion polypeptide is a variant of SEQ ID NO: 14 or 15, a variant of a mature polypeptide of SEQ ID NO: 14 or 15, or a variant of SEQ ID NO: 14 or 15 comprising a substitution, deletion, and/or insertion at one or more positions.

In one embodiment, the SP-linker comprises an N-terminal extension and/or C-terminal extension of 1-10 amino acids, e.g., 1, 2, 3, 4, 5, 6, 7, 8, 9, or 10 amino acids, preferably and extension of 1-5 amino acid residues in the N-terminus and/or 1-30 amino acids in the C-terminus, such as 1-15. In one embodiment, the extended polypeptide has amylase activity.

In one embodiment, the fusion polypeptide is comprising, consisting essentially of, or consisting of SEQ ID NO: 14.

In one embodiment, the fusion polypeptide is comprising, consisting essentially of, or consisting of SEQ ID NO: 15.

In an eighth aspect the invention relates to an extended fusion polypeptide comprising the fusion polypeptide of the 7th aspect and a second polypeptide of interest.

In a 9th aspect the invention relates to a hybrid polypeptide comprising the fusion polypeptide of the 7th aspect.

In one embodiment, the fusion polypeptide according to the 7th aspect 7A is encoded by a nucleic acid construct according to the first aspect 1A, and/or the expression vector according to the second aspect 2A, and/or the expression vector according to the second aspect 2B, the second aspect 2C, and/or wherein the polypeptide is expressed by a host cell according to the third aspect 3A.

In one embodiment, the fusion polypeptide is isolated.

In one embodiment, the fusion polypeptide is purified.

In one embodiment, the sequence identity of the fusion polypeptide is determined by Sequence Identity Determination Method 1.

In a 10th aspect 10A, the invention also relates to a polypeptide, comprising a polypeptide of interest, and a signal peptide derived from a bacterial yckD polypeptide.

In one embodiment, the polypeptide is secreted.

In one embodiment, the signal peptide is heterologous to the polypeptide of interest.

In one embodiment, the signal peptide is derived from a bacterial cell, *e.g.*, a Gram-positive cell selected from the group consisting of *Bacillus, Clostridium, Enterococcus, Geobacillus, Lactobacillus, Lactococcus, Oceanobacillus, Staphylococcus, Streptococcus,* or *Streptomyces* cells, or a Gram-negative bacteria selected from the group consisting of *Campylobacter, E. coli, Flavobacterium, Fusobacterium, Helicobacter, Ilyobacter, Neisseria, Pseudomonas, Salmonella,* and *Ureaplasma* cells, such as *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis, Bacillus thuringiensis, Streptococcus equisimilis, Streptococcus pyogenes, Streptococcus uberis,* and *Streptococcus equi* subsp. *Zooepidemicus, Streptomyces achromogenes, Streptomyces avermitilis, Streptomyces coelicolor, Streptomyces griseus,* and *Streptomyces lividans* cells.

In one embodiment, the signal peptide is derived from a *Bacillus* cell, such as from a *Bacillus subtilis* cell, a *Bacillus licheniformis* cell, or a *Bacillus pumilus* cell.

In one embodiment, the signal peptide is derived from a first donor polypeptide comprising or consisting of an amino acid sequence having at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% sequence identity to the amino acid sequence of SEQ ID NO: 19.

In one embodiment, the polypeptide of interest comprises a pro-peptide. Preferably the pro-peptide is located at the N-terminal end of the amino acid sequence of the polypeptide of interest, and the signal peptide is located at the N-terminal end of the pro-peptide.

In one embodiment, the signal peptide is removed from the polypeptide of interest, preferably removed from the polypeptide of interest together with the pro-peptide.

In one embodiment, the polypeptide of interest is selected from the list of a hydrolase, isomerase, ligase, lyase, lysozyme, oxidoreductase, or transferase, e.g., an aminopeptidase, amylase, carbohydrase, carboxypeptidase, catalase, cellobiohydrolase, cellulase, chitinase, cutinase, cyclodextrin glycosyltransferase, deoxyribonuclease, endoglucanase, esterase, alpha-galactosidase, beta-galactosidase, glucoamylase, alpha-glucosidase, beta-glucosidase, invertase, laccase, lipase, mannosidase, mutanase, nuclease, oxidase, pectinolytic enzyme, peroxidase, phosphodiesterase, phytase, polyphenoloxidase, proteolytic enzyme, ribonuclease, transglutaminase, xylanase, beta-xylosidase, or a protease.

In one embodiment the polypeptide of interest comprises a protease, preferably the protease having protease activity.

In one embodiment the polypeptide of interest comprises a protease comprising or consisting of an amino acid sequence having at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% sequence identity to the amino acid sequence of SEQ ID NO: 16.

In one embodiment, the signal peptide is selected from the group consisting of:

(a) a polypeptide having at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% sequence identity to the sequence of SEQ ID NO: 4;

(b) a polypeptide encoded by a polynucleotide having at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% sequence identity to the polypeptide coding sequence of SEQ ID NO: 2;

(c) a polypeptide derived from SEQ ID NO: 4 having 1-30 alterations, e.g., substitutions, deletions and/or insertions at one or more positions, e.g., 1 or 2 or 3 or 4 or 5 or 6 or 7 or 8 or 9 or 10 or 11 or 12 or 13 or 14 or 15 or 16 or 17 or 18 or 19 or 20 or 21 or 22 or 23 or 24 or 25 or 26 or 27 alterations, in particular substitutions;

(d) a polypeptide derived from the polypeptide of (a), (b), or (c), wherein the N- and/or C-terminal end of the polypeptide fragment has been extended by addition of one or more amino acids; and

(e) a fragment of the polypeptide of (a), (b), (c), or (d).

In one embodiment, the the polypeptide is selected from the group consisting of:

(a) a polypeptide having at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% sequence identity to SEQ ID NO: 80;

(b) a polypeptide derived from the polypeptide of (a), the N- and/or C-terminal end has been extended by addition of one or more amino acids, said polypeptide having protease activity; and

(c) a fragment of the polypeptide of (a), or (b);

wherein the polypeptide has protease activity.

In one embodiment, the polypeptide is a variant of SEQ ID NO: 80 or 16, a variant of a mature polypeptide of SEQ ID NO: 80 or 16, or a variant of SEQ ID NO: 80 or 16 comprising a substitution, deletion, and/or insertion at one or more positions.

In one embodiment, the signal peptide comprises an N-terminal extension and/or C-terminal extension of 1-10 amino acids, e.g., 1, 2, 3, 4, 5, 6, 7, 8, 9, or 10 amino acids, preferably and extension of 1-5 amino acid residues in the N- terminus and/or 1-30 amino acids in the C-terminus, such as 1-15. Preferably the extended polypeptide has protease activity.

In one embodiment, the polypeptide is consisting essentially of, or consisting of SEQ ID NO: 80 or SEQ ID NO: 16.

In a 10th aspect 10B, the invention also relates to a polypeptide, comprising a polypeptide of interest, and a signal peptide derived from a fungal prolyl dipeptidyl peptidase.

In one embodiment, the polypeptide of interest is secreted.

In one embodiment, the signal peptide is heterologous to the polypeptide of interest.

In one embodiment, the signal peptide is derived from a funga cell, e.g., a filamentous fungal cell.

In another embodiment, the signal peptide is derived from a first donor polypeptide comprising or consisting of a peptidase, such as an polyl dipeptidyl peptidase, preferably a peptidase from *Aspergillus*, such as *Aspergilly oryzae*.

In one embodiment, the signal peptide comprises or consists of an amino acid sequence having at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% sequence identity to the amino acid sequence of SEQ ID NO: 172.

In one embodiment, the signal peptide is removed from the polypeptide of interest.

In one embodiment, the polypeptide of interest is selected from the list of a hydrolase, isomerase, ligase, lyase, lysozyme, oxidoreductase, or transferase, e.g., an aminopeptidase, amylase, carbohydrase, carboxypeptidase, catalase, cellobiohydrolase, cellulase, chitinase, cutinase, cyclodextrin glycosyltransferase, deoxyribonuclease, endoglucanase, esterase, alpha-galactosidase, beta-galactosidase, glucoamylase, alpha-glucosidase, beta-glucosidase, invertase, laccase, lipase, mannosidase, mutanase, nuclease, oxidase, pectinolytic enzyme, peroxidase, phosphodiesterase, phytase, polyphenoloxidase, proteolytic enzyme, ribonuclease, transglutaminase, xylanase, beta-xylosidase, or a protease.

In one embodiment the polypeptide of interest comprises a phytase, preferably the phytase having phytase activity.

In one embodiment the polypeptide of interest comprises a endoglucanase, preferably the endoglucanase having endoglucanase activity.

In one embodiment, the signal peptide is selected from the group consisting of:

(a) a polypeptide having at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% sequence identity to the sequence of SEQ ID NO: 172;
(b) a polypeptide encoded by a polynucleotide having at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% sequence identity to the polypeptide coding sequence of SEQ ID NO: 178;
(b') a polypeptide encoded by a polynucleotide having at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% sequence identity to the polypeptide coding sequence of polynucleotides 1-6 and polynucleotides 90-131 of SEQ ID NO: 178;
(c) a polypeptide derived from SEQ ID NO: 172 having 1-30 alterations, e.g., substitutions, deletions and/or insertions at one or more positions, e.g., 1 or 2 or 3 or 4 or 5 or 6 or 7 or 8 or 9 or 10 or 11 or 12 or 13 or 14 or 15 or 16 or 17 or 18 or 19 or 20 or 21 or 22 or 23 or 24 or 25 or 26 or 27 alterations, in particular substitutions;
(d) a polypeptide derived from the polypeptide of (a), (b), (b'), or (c), wherein the N- and/or C-terminal end of the polypeptide fragment has been extended by addition of one or more amino acids; and
(e) a fragment of the polypeptide of (a), (b), (b'), (c), or (d).

In one embodiment, the signal peptide comprises an N-terminal extension and/or C-terminal extension of 1-10 amino acids, e.g., 1, 2, 3, 4, 5, 6, 7, 8, 9, or 10 amino acids, preferably and extension of 1-5 amino acid residues in the N- terminus and/or 1-30 amino acids in the C-terminus, such as 1-15. Preferably the extended polypeptide has phytase activity, or endoglucanase activity.

In a 11th aspect 11A, the invention relates to a fusion polypeptide comprising the polypeptide of the 10th aspect 10A and a second polypeptide of interest.

In a 11th aspect 11B, the invention relates to a fusion polypeptide comprising the polypeptide of the 10th aspect 10B and a second polypeptide of interest.

In a 12th aspect 12A, the invention relates to a hybrid polypeptide comprising the polypeptide of the 10th aspect 10A.

In a 12th aspect 12B, the invention relates to a hybrid polypeptide comprising the polypeptide of the 10th aspect 10B.

In one embodiment the polypeptide is isolated.

In one embodiment the polypeptide is purified.

In one embodiment the sequence identity of the polypeptide is determined by Sequence Identity Determination Method 1.

In one embodiment, the polypeptide according to the 10th aspect is encoded by a nucleic acid construct according to the first aspect 1B, and/or the expression vector according to the second aspect 2B, and/or wherein the polypeptide is expressed by a host cell according to the third aspect 3B.

In another aspect, the fusion polypeptide is derived from SEQ ID NO: 14, 15, 16 or 80 by substitution, deletion or addition of one or several amino acids. In another aspect, the fusion polypeptide is derived from a mature polypeptide of SEQ ID NO: 14, 15, 16 or 80 by substitution, deletion or addition of one or several amino acids. In some embodiments, the polypeptide is a variant of SEQ ID NO: 14, 15, 16 or 80 comprising a substitution, deletion, and/or insertion at one or more positions. In one aspect, the number of amino acid substitutions, deletions and/or insertions introduced into the polypeptide of SEQ ID NO: 14, 15, 16 or 80 is up to 15, *e.g.,* 1, 2, 3, 4, 5, 6, 7, 8, 9, 10, 11, 12, 13, 14, or 15. The amino acid changes may be of a minor nature, that is conservative amino acid substitutions or insertions that do not significantly affect the folding and/or activity of the protein; small deletions, typically of 1-30 amino acids; small amino- or carboxyl-terminal extensions, such as an amino-terminal methionine residue; a small linker peptide of up to 20-25 residues; or a small extension that facilitates purification by changing net charge or another function, such as a poly-histidine tract, an antigenic epitope or a binding module.

Essential amino acids in a polypeptide can be identified according to procedures known in the art, such as site-directed mutagenesis or alanine-scanning mutagenesis (Cunningham and Wells, 1989, Science 244: 1081-1085). In the latter technique, single alanine mutations are introduced at every residue in the molecule, and the resultant molecules are tested for enzyme activity to identify amino acid residues that are critical to the activity of the molecule. See also, Hilton et al., 1996, J. Biol. Chem. 271: 4699-4708. The active site of the enzyme or other biological interaction can also be determined by physical analysis of structure, as determined by such techniques as nuclear magnetic resonance, crystallography, electron diffraction, or photoaffinity labeling, in conjunction with mutation of putative contact site amino acids. See, for example, de Vos et al., 1992, Science 255: 306-312; Smith et al., 1992, J. Mol. Biol. 224: 899-904; Wlodaver et al., 1992, FEBS Lett. 309: 59-64. The identity of essential amino acids can also be inferred from an alignment with a related polypeptide, and/or be inferred from sequence homology and conserved catalytic machinery with a related polypeptide or within a polypeptide or protein family with polypeptides/proteins descending from a common ancestor, typically having similar three-dimensional structures, functions, and significant sequence similarity. Additionally or alternatively, protein structure prediction tools can be used for protein structure modelling to identify essential amino acids and/or active sites of polypeptides. See, for example, Jumper et al., 2021, "Highly accurate protein structure prediction with AlphaFold", Nature 596: 583-589.

Single or multiple amino acid substitutions, deletions, and/or insertions can be made and tested using known methods of mutagenesis, recombination, and/or shuffling, followed by a relevant screening procedure, such as those disclosed by Reidhaar-Olson and Sauer, 1988, Science 241: 53-57; Bowie and Sauer, 1989, Proc. Natl. Acad. Sci. USA 86: 2152-2156; WO 95/17413; or WO 95/22625. Other methods that can be used include error-prone PCR, phage display (e.g., Lowman et al., 1991, Biochemistry 30: 10832-10837; US 5,223,409; WO 92/06204), and region-directed mutagenesis (Derbyshire et al., 1986, Gene 46: 145; Ner et al., 1988, DNA 7: 127).

Mutagenesis/shuffling methods can be combined with high-throughput, automated screening methods to detect activity of cloned, mutagenized polypeptides expressed by host cells (Ness et al., 1999, Nature Biotechnology 17: 893-896). Mutagenized DNA molecules that encode active polypeptides can be recovered from the host cells and rapidly sequenced using standard methods in the art. These methods allow the rapid determination of the importance of individual amino acid residues in a polypeptide.

**Sources of Polypeptides for a SP-linker**

A SP-linker polypeptide of the present invention may be obtained from organisms of any genus. For purposes of the present invention, the term "obtained from" as used herein in connection with a given source shall mean that the polypeptide encoded by a polynucleotide is produced by the source or by a strain in which the polynucleotide of the invention has been inserted. In one aspect, the polypeptide obtained from a given source is secreted extracellularly.

In one aspect, the second donor polypeptide is a polypeptide obtained from a *Bacillus clausii or Nocardiopsis prasina.*

In one aspect, the second donor polypeptide is a polypeptide obtained from *Thermomyces lanuginosus,* such as a *Thermomyces lanuginosus* xylanase.

In one aspect, the second donor polypeptide is a polypeptide obtained from *Thermothielavioides terrestris*, such as a *Thermothielavioides terrestris* endoglucanase.

It will be understood that for the aforementioned species, the invention encompasses both the perfect and imperfect states, and other taxonomic equivalents, *e.g.*, anamorphs, regardless of the species name by which they are known. Those skilled in the art will readily recognize the identity of appropriate equivalents.

The polypeptides may be identified and obtained from other sources including mammalian cells and microorganisms isolated from nature (*e.g.*, soil, composts, water, etc.) or DNA samples obtained directly from natural materials (*e.g.*, soil, composts, water, etc.) using the above-mentioned probes. Techniques for isolating microorganisms and DNA directly from natural habitats are well known in the art. A polynucleotide encoding the SP-linker polypeptide may then be obtained by similarly screening a genomic DNA or cDNA library of another microorganism or mixed DNA sample. Once a polynucleotide encoding a SP-linker polypeptide has been detected with the probe(s), the polynucleotide encoding the SP-linker can be isolated or cloned by utilizing techniques that are known to those of ordinary skill in the art (see, *e.g.*, Davis et al., 2012, Basic Methods in Molecular Biology, Elsevier).

**Methods for identifying a suitable SP - SP-linker combination**

In a 13th aspect, the invention relates to a method for increasing secretion of a polypeptide of interest by a host cell, the method comprising the steps of

a) **providing** a plurality of first polynucleotide sequences, each first polynucleotide encoding a signal peptide;

b) **expressing** a first polypeptide with a host cell, wherein the polynucleotide encoding the first polypeptide is linked in translational fusion with a first polynucleotide;

c) **measuring** the amount of first polypeptide for each first polynucleotide sequence;

d) **ranking** each first polynucleotide based on the amount of secreted first polypeptide;

e) **selecting** one or more of the ranked first polynucleotides;

f) **fusing** the selected one or more first polynucleotide with a second polynucleotide encoding a SP-linker comprising or consisting of 2-15 N-terminal amino acids from the secreted first polypeptide expressed in step b), wherein the second polynucleotide is located downstream of the first polynucleotide, and

g) **expressing,** in a host cell, the polypeptide of interest in translational fusion with the fusion construct generated in step f), wherein the SP-linker is located upstream of the polypeptide of interest.

In one embodiment the first polypeptide is secreted.

In one embodiment, the fusion construct comprising the SP-linker generated in step f) results in increased secretion or production of the polypeptide of interest relative to the secretion or production of the polypeptide of interest with the signal peptide alone expressed in step b).

In one embodiment, the second polynucleotide encoding the SP-linker, and the first polynucleotide encoding the signal peptide are heterologous to the polynucleotide encoding the polypeptide of interest.

In one embodiment, during step d) the signal peptides are ranked from top (highest secretion/production) to bottom (lowest secretion/production).

In one embodiment, during step d) the method comprises comparing the amount of secreted/produced first polypeptide with the amount of secreted/produced first polypeptide when expressed in translational fusion with one or more reference signal peptide(s).

In one embodiment, during step e) it is selected one or more first polynucleotide encoding the signal peptides from step d) ranked in the top 10%, preferably in the top 5%, the top 4%, the top 3%, the top 2%, or the top1 %,

In one embodiment, during step e) it is selected one or more first polynucleotide encoding signal peptides from step d) having increased secretion/production of the first polypeptide relative to the secretion/production of the reference signal peptide(s),

**Signal Peptide and Propeptide**

The present invention also relates to a polynucleotide encoding a signal peptide comprising or consisting of amino acids 1 to 27 of SEQ ID NO: 80. The present invention also relates to a polynucleotide encoding a propeptide comprising or consisting of amino acids 28 to 55 of SEQ ID NO: 80. The present invention also relates to a polynucleotide encoding a signal peptide and a propeptide comprising or consisting of amino acids 1 to 55 of SEQ ID NO: 80.

The present invention also relates to a polynucleotide encoding a signal peptide comprising or consisting of amino acids 1 to 16 of SEQ ID NO: 172. The present invention also relates to a polynucleotide encoding a signal peptide comprising or consisting of polynucleotides 1 to 131 of SEQ ID NO: 178, polynucleotides 1-6 and 90-131 of SEQ ID NO: 178. The polynucleotides may further comprise a gene encoding a polypeptide of interest, which is operably linked to the signal peptide and/or propeptide. The polypeptide of interest is preferably heterologous to the signal peptide and/or propeptide.

The present invention also relates to nucleic acid constructs, expression vectors and recombinant host cells comprising such polynucleotides.

The present invention also relates to methods of producing a polypeptide of interest, comprising (a) cultivating a recombinant host cell comprising such polynucleotide; and optionally (b) recovering the polypeptide of interest.

The polypeptide of interest may be native or heterologous to a host cell. The term "polypeptide of interest" or "protein of interest" is not meant herein to refer to a specific length of the encoded product and, therefore, encompasses peptides, oligopeptides, and polypeptides. The term "polypeptide of interest" or "protein of interest" also encompasses two or more polypeptides combined to form the encoded product. The proteins also include hybrid polypeptides and fusion polypeptides.

Preferably, the polypeptide of interest is a hormone, enzyme, receptor or portion thereof, antibody or portion thereof, or reporter. For example, the polypeptide of interest may be a hydrolase, isomerase, ligase, lyase, oxidoreductase, or transferase, *e.g.*, an alpha-galactosidase, alpha-glucosidase, aminopeptidase, amylase, beta-galactosidase, beta-glucosidase, beta-xylosidase, carbohydrase, carboxypeptidase, catalase, cellobiohydrolase, cellulase, chitinase, cutinase, cyclodextrin glycosyltransferase, deoxyribonuclease, endoglucanase, esterase, glucoamylase, invertase, laccase, lipase, mannosidase, mutanase, oxidase, pectinolytic enzyme, peroxidase, phytase, polyphenoloxidase, proteolytic enzyme, ribonuclease, transglutaminase, or xylanase.

The gene may be obtained from any prokaryotic, eukaryotic, or other source.

The present invention is further described by the following examples that should not be construed as limiting the scope of the invention.

**Examples**

**Media**

*Bacillus* strains were grown on LB agar (10g/L Tryptone, 5g/L yeast extract, 5g/L NaCl, 15g/L agar) plates or in TY liquid medium (20g/L Tryptone, 5g/L yeast extract, 7mg/L $FeCl_2$, 1mg/L $MnCl_2$, 15mg/L $MgCl_2$). To select for erythromycin resistance, agar and liquid media were supplemented with $5\mu g/ml$ erythromycin. *Aspergillus* strains were grown in YPD (yeast extract 1g/L, peptone 2g/L, dextrose 2g/L).

LB agar: 10 g/l peptone from casein; 5 g/l yeast extract, 10 g/l sodium chloride; 12 g/l Bacto-agar adjusted to pH 7.0 +/- 0.2. Premix from Merck was used (LB-agar (Miller) 110283)

M-9 buffer: di-sodiumhydrogenphosphate, $2H_2O$ 8.8g/l; potassiumdihydrogenphosphate 3g/l; sodium chloride 4 g/l; magnesium sulphate, 7 $H_2O$ 0.2 g/l (deionized water is used in this buffer)

PRK-50: 110 g/l soy grits; di-sodiumhydrogenphosphate, $2H_2O$ 5g/l; Antifoam (Struktol SB2121; Schill & Seilacher, Hamburg, Germany) 1ml/l, pH adjusted to 8.0 with $NaOH/H_2PO_4$ before sterilization.

Make-up medium: Tryptone (Casein hydrolystae from Difco (BactoTM Tryptone pancreatic Digest of Casein 211699) 30 g/l; magnesium sulphate, 7 $H_2O$ 4g/l; di-potassiumdihydrogenphosphate 7g/l; di-sodiumhydrogenphosphate, 2 $H_2O$ 7 g/l; di-ammoniumsulphate 4 g/l; citric acid 0.78 g/l; vitamins (thiamin-dichlorid 34.2 mg/l; riboflavin 2.9 mg/l; nicotinic acid 23 mg/l; calcium D-pantothenate 28.5 mg/l; pyridoxai-HCl 5.7 mg/l; D-biotin 1.1 mg/l; folic acid 2.9 mg/l); trace metals (MnSO4, H2O 39.2 mg/l, FeSO4, 7H2O 157 mg/l, CuSO 4, 5H2O 15.6 mg/l; ZnCl2 15.6 mg/l); Antifoam (Struktol SB2121; Schill & Seilacher, Hamburg, Germany) 1.25 ml/l; pH adjusted to 6.0 with $NaOH/H_2PO_4$ before sterilization.

Feed-medium: Glucose, 1H 2O 820 g/l

**Molecular biological methods**

DNA manipulations and transformations were performed by standard molecular biology methods as described in:

- Sambrook et al. (1989): Molecular cloning: A laboratory manual. Cold Spring Harbor laboratory, Cold Spring Harbor, NY.
- Ausubel et al. (eds) (1995): Current pro7tocols in Molecular Biology. John Wiley and Sons.
- Harwood and Cutting (eds) (1990): Molecular Biological Methods for Bacillus. John Wiley and Sons.

Enzymes for DNA manipulation were obtained from New England Biolabs, Inc. and used essentially as recommended by the supplier.

Direct transformation into *B. licheniformis* was one as previously described in patent US 2019/0185847 A1. Conjugation into *B. licheniformis* was performed as described in WO 2018/077796 A1.

Genomic DNA was prepared by using the commercially available QIAamp DNA Blood Kit from Qiagen. The respective DNA fragments were amplified by PCR using the Phusion Hot Start DNA Polymerase system (Thermo Scientific). PCR amplification reaction mixtures contained $1\mu L$ (0,1pg) of template DNA, $1\mu L$ of sense primer (20pmol/$\mu L$), $1\mu L$ of anti-sense primer (20pmol/$\mu L$), $10\mu L$ of 5X PCR buffer with 7,5mM $MgCl_2$, $8\mu L$ of dNTP mix (1,25mM each), $39\mu L$ water, and $0.5\mu L$ (2 U/) DNA polymerase. A thermocycler was used to amplify the fragment. The PCR products were purified from a 1.2% agarose gel with 1x TBE buffer using the Qiagen QIAquick Gel Extraction Kit (Qiagen, Inc., Valencia, CA) according to the manufacturer's instructions.

The condition for POE-PCR is as follows: purified PCR products were used in a subsequent PCR reaction to create a single fragment using splice overlapping PCR (SOE) using the Phusion Hot Start DNA Polymerase system (Thermo Scientific) as follows. The very 5' end fragment and the very 3' end fragment have complementary end which will allow the SOE to concatemer into the POE PCR product. The PCR amplification reaction mixture contained 50 ng of each of the three gel purified PCR products. POE PCR was performed as described in (You, C et al (2017) Methods Mol. Biol. 116, 183-92).

Fermentations

For Biolector fermentations (example 8 to 10), strains were fermented in flower plates (MTP-48-B) in 1mL TY media for 24hr at 37C, 1000rpm in the Biolector (m2p-labs). The cultivation plates were inoculated from an over-night culture grown in a M-tube in 10mL TY media at 37C, 250rpm. The flower plates were inoculated to a OD(450nm) of 0,05. For small scale fermentations (example 11), strains were grown in microtiter plates with nutrient controlled media.

Lab-scale fermentations:

Procedure for inoculum steps

    a) Grow the strain on LB agar slants o/n at 37°C.
    b) Wash the agar with M-9 buffer and collect the cell suspension. Measure OD650.
    c) Inoculate PRK-50 shake flask (OD650 x ml cell suspension = 1).
    d) Incubate the shake flask at 300rpm o/n at 37°C.
    e) Start the main fermentor by adding the growing shake flask culture (10% of make-up media, i.e. 80 ml to 800ml).

Fermentor equipment:

Standard lab fermenters equipped with a temperature control system, pH control with ammoniawater and phosphoric acid, dissolved oxygen electrode to measure >20% oxygen saturation through the entire fermentation.

Fermentation parameters:

Temperature: 37°C.

Keep pH between 6,8 and 7,2 using ammonia water and phosphoric acid.

Aeration: 1,5 L/min/kg broth weight

Agitation: 1500 rpm.

*Aspergillus oryzae* Lab-scale fermentations:

Seed cultivation:

Spores from cove-N-gly agar slant were transferred to shake flask (glycerol 20g/L, yeast extract 18 g/L) and incubated for 1 day at 30 °C and 250 rpm.

Fermentor equipment:

Standard lab fermenters equipped with a temperature control system, pH control with ammoniawater and phosphoric acid, dissolved oxygen electrode to measure >20% oxygen saturation through the entire fermentation.

Fed batch fermentation:

Tank medium (sucrose 24 g/L, yeast extract 10 g/L, (NH4)2SO4 5 g/L, MgSO4 7H2O 2 g/L, K2SO4 2 g/L, citric acid 1 g/L, KH2PO4 2 g/L trace metal solution 0,5 ml/L) was adjusted to 34 °C. Aeration was 1 vvm and pH was controlled at 6,0 using 10% NH4OH. Main medium was inoculated from seed cultivation. When pH > 6,4 feeding (400 g/L maltose sirup, 1 g/L citric acid) was started at a rate of 3,33 g/L/h. Stirrer speed was controlled to avoid too low (<20%) oxygen tension.

**Amylase assay**

Amylase activity was measured in culture supernatants using the AMYL (Roche/Hitachi # 11876473 001). Culture supernatants from DWP were diluted to 1/50 in Stabilizer buffer (0,03M CaCl2; 0,0083% Brij 35). Samples from bioreactor samples were diluted in Stabilizer buffer. Reagent 1 and reagent 2 of the AMYL kit were mixed 10:1 to generate the assay substrate. 20$\mu$L diluted sample was mixed with 180$\mu$L assay substrate. Assay was incubated as 37C w/shaking for 30min. Absorbance was measured at 405nm in plate reader.

**SDS-PAGE electrophoresis**

Raw supernatant from the Biolector fermentation were prepared for SDS electrophoresis as follows: 75$\mu$L raw supernatant was mixed with 25$\mu$L NuPAGE™ LDS Sample Buffer (4X) plus 10$\mu$L NuPAGE™ Sample Reducing Agent and boiled for 5min. 15$\mu$L was loaded onto NuPAGE Bis-Tris gel.

**Protease assay**

The serine endopeptidase hydrolyses the substrate N-Succinyl-Ala-Ala-Pro-Phe p-nitroanilide. The reaction was performed at Room Temperature at pH 9.0. The release of pNA results in an increase of absorbance at 405 nm and this increase is proportional to the enzymatic activity measured against a standard.

**Phytase assay**

The assay is based on determination of soluble phosphate by complexation with molybdate/iron and photometric measurement of the blue color in microtiter plates.
The substrate is 0.5mM Na-phytate (Sigma, P-8810) dissolved in 0.1 M acetate-buffer, pH=5.5. In a particular embodiment the substrate concentration is 5mM.
The color reagent is prepared as follows: 1% Ammoniummolybdat (Merck 1181, $(NH_4)_6Mo_7O_{24}$, $4H_2O$) is dissolved in 3.2% sulfuric acid (Merck 731). 1.1 g ferrosulfate (Merck 3965) is dissolved in 15 ml of the above molybdate reagent and 10 ml of 0.5 M sulfuric acid is added. Is freshly prepared every day, and stored in the dark.
Blind: 20ul sample, 100ul substrate and 120ul color reagent is mixed, incubated 5 min at 37°C and $OD_{Blind}$ measured at 750nm.
Sample: 20ul sample, 100ul substrate is mixed, incubated 30 min at 37°C, 120ul color reagent is added, incubated 5 min at 37°C, and $OD_{sample}$ is measured at 750nm.

$$OD = OD_{sample} - OD_{Blind}.$$

**Strains**

| | |
|---|---|
| MOL3320: | Bacillus licheniformis, amyL, aprL, bglC, cypX, forD, gntP, lacA2, mprL, sacB, spoIIAC, xylA, ara:: Mad7d-sgRNA::mecA-ERM (as described in patent US 2019/0185847 A1) |
| THKK0298: | B. Licheniformis MOL3320, ara::P3-SP(Bacillus pumilus aspartate phosphatase)-Protease 1 -ERM |
| BT11032: | B. Licheniformis MOL3320, ara::P3-SP(aprL)-Protease 1 -ERM |
| BT11054: | B. Licheniformis MOL3320, ara::P3-SP(B. pum asp phos)-Amylase-ERM |
| BT11055: | B. Licheniformis MOL3320, ara::P3-SP(B. pum asp phos)-7 N-term codon from Protease 1-Amylase-ERM |
| BT11063: | B. Licheniformis MOL3320, ara::P3-SP(B. pum yckD)-Protease 2-ERM |
| BT11078 | B. Licheniformis MOL3320, ara::P3-SP(B.pum yckD)-Amylase-ERM |
| BT11079: | B. Licheniformis MOL3320, ara::P3-SP(B.pum yckD)-7 N-term codon from Protease 2-Amylase-ERM |
| PP3724: | Bacillus subtilis as in WO 2018/077796 A1 |
| BT11161 | *B. subtilis* PP3724, pBT11161 |
| BT11162 | *B. subtilis* PP3724, pBT11162 |
| BT11163 | *B. subtilis* PP3724, pBT11163 |
| BT11164 | *B. subtilis* PP3724, pBT11164 |
| BT11165 | *B. subtilis* PP3724, pBT11165 |
| BT11166 | *B. subtilis* PP3724, pBT11166 |
| BT11167 | *B. subtilis* PP3724, pBT11167 |
| BT11168 | *B. subtilis* PP3724, pBT11168 |
| BT11169 | *B. subtilis* PP3724, pBT11169 |
| BT11170 | *B. subtilis* PP3724, pBT11170 |
| BT11171 | *B. subtilis* PP3724, pBT11171 |
| BT11172 | *B. subtilis* PP3724, pBT11172 |
| BT11173 | *B. subtilis* PP3724, pBT11173 |
| BT11174 | *B. subtilis* PP3724, pBT11174 |
| BT11178 | *B. subtilis* PP3724, pBT11178 |
| SJ14538: | aprL, mprL, spoIIAC, cypX, L12, sacB, amyL, bglC, xylA+, gntP+, lacA2::P3-2SD-FRT-F-dsRED-FRT-F3 (WO 2018/077796 A1) |
| BT11195 | as *B. licheniformis* SJ14538, lacA2::P3-2SD-FRT-F-SP(B.pum yckD)-1 N-term codon from Protease 2-Amylase-FRT-F3 |
| BT11196 | as B. licheniformis SJ14538, lacA2::P3-2SD-FRT-F-SP(B.pum yckD)-2 N-term codon from Protease 2-Amylase-FRT-F3 |
| BT11197 | as B. licheniformis SJ14538, lacA2::P3-2SD-FRT-F-SP(B.pum yckD)-3 N-term codon from Protease 2-Amylase-FRT-F3 |
| BT11198 | as B. licheniformis SJ14538, lacA2::P3-2SD-FRT-F-SP(B.pum yckD)-4 N-term codon from Protease 2-Amylase-FRT-F3 |
| BT11199 | as B. licheniformis SJ14538, lacA2::P3-2SD-FRT-F-SP(B.pum yckD)-5 N-term codon from Protease 2-Amylase-FRT-F3 |
| BT11200 | as B. licheniformis SJ14538, lacA2::P3-2SD-FRT-F-SP(B.pum yckD)-6 N-term codon from Protease 2-Amylase-FRT-F3 |
| BT11201 | as B. licheniformis SJ14538, lacA2::P3-2SD-FRT-F-SP(B.pum yckD)-8 N-term codon from Protease 2-Amylase-FRT-F3 |

(continued)

| BT11202 | as B. licheniformis SJ14538, lacA2::P3-2SD-FRT-F-SP(B.pum yckD)-9 N-term codon from Protease 2-Amylase-FRT-F3 |
|---|---|
| BT11203 | as B. licheniformis SJ14538, lacA2::P3-2SD-FRT-F-SP(B.pum yckD)-10 N-term codon from Protease 2-Amylase-FRT-F3 |
| BT11204 | as B. licheniformis SJ14538, lacA2::P3-2SD-FRT-F-SP(B.pum yckD)-11 N-term codon from Protease 2-Amylase-FRT-F3 |
| BT11205 | as B. licheniformis SJ14538, lacA2::P3-2SD-FRT-F-SP(B.pum yckD)-12 N-term codon from Protease 2-Amylase-FRT-F3 |
| BT11206 | as B. licheniformis SJ14538, lacA2::P3-2SD-FRT-F-SP(B.pum yckD)-13 N-term codon from Protease 2-Amylase-FRT-F3 |
| BT11207 | as B. licheniformis SJ14538, lacA2::P3-2SD-FRT-F-SP(B.pum yckD)-14 N-term codon from Protease 2-Amylase-FRT-F3 |
| BT11208 | as B. licheniformis SJ14538, lacA2::P3-2SD-FRT-F-SP(B.pum yckD)-15 N-term codon from Protease 2-Amylase-FRT-F3 |
| BT11212 | as B. licheniformis SJ14538, lacA2::P3-2SD-FRT-F-SP(B.pum yckD)-7 N-term codon from Protease 2-Amylase-FRT-F3 |
| BT11146 | as B. licheniformis SJ14538, lacA2::P3-2SD-FRT-F-SP(std)-Amylase-FRT-F3 |
| SJ13837 | *B. subtilis* PP3724, pSJ13835 |
| BT11176 | *B. subtilis* PP3724, pBT11176 |
| BT11210 | as B. licheniformis SJ14538, lacA2::P3-2SD-FRT-F-SP(B.pum yckD)-7 N-term codon from Protease 1-Amylase-FRT-F3 |
| BT11177 | *B. subtilis* PP3724, pBT11177 |
| BT11211 | as B. licheniformis SJ14538, lacA2::P3-2SD-FRT-F-SP(B.pum yckD)-Amylase-FRT-F3 |
| COLS1300 | *A. oryzae* amyA, amyB, amyC, alpA, NprA, kusA, niaD, niiA, amdS+ (as described in WO2013/119302) |
| AT6552 | *A. oryzae* COLS1300 niaD/niiA::SP (*A. oryzae* Tryacylglycerol lipase) - Phytase |
| AT6502 | *A. oryzae* COLS1300 niaD/niiA::SP (*A. oryzae* Tryacylglycerol lipase) - 21 N-term codon from Xylanase (T. lanuginosus) - Phytase |
| AT6553 | *A. oryzae* COLS1300 niaD/niiA::SP (*A. oryzae* Prolyl dipeptidyl peptidase) - Phytase |
| AT6503 | *A. oryzae* COLS1300 niaD/niiA::SP (*A. oryzae* Prolyl dipeptidyl peptidase) - 21 N-term codon from Endoglucanase (T. terrestris) - Phytase |

**Plasmids**

| pSJ13835 | flp-FRT plasmid as described in WO 2018/077796 A1, expressing Amylase w/ standard SP (SEQ ID NO: 168) |
|---|---|
| pBT11161 | as pSJ13835, FRT-F-SP(B.pum yckD)-1 N-term codon from Protease 2-Amylase-FRT-F3 |
| pBT11162 | as pSJ13835, FRT-F-SP(B.pum yckD)-2 N-term codon from Protease 2-Amylase-FRT-F3 |
| pBT11163 | as pSJ13835, FRT-F-SP(B.pum yckD)-3 N-term codon from Protease 2-Amylase-FRT-F3 |
| pBT11164 | as pSJ13835, FRT-F-SP(B.pum yckD)-4 N-term codon from Protease 2-Amylase-FRT-F3 |
| pBT11165 | as pSJ13835, FRT-F-SP(B.pum yckD)-5 N-term codon from Protease 2-Amylase-FRT-F3 |
| pBT11166 | as pSJ13835, FRT-F-SP(B.pum yckD)-6 N-term codon from Protease 2-Amylase-FRT-F3 |
| pBT11167 | as pSJ13835, FRT-F-SP(B.pum yckD)-8 N-term codon from Protease 2-Amylase-FRT-F3 |

(continued)

| pBT11168 | as pSJ13835, FRT-F-SP(B.pum yckD)-9 N-term codon from Protease 2-Amylase-FRT-F3 |
|---|---|
| pBT11169 | as pSJ13835, FRT-F-SP(B.pum yckD)-10 N-term codon from Protease 2-Amylase-FRT-F3 |
| pBT11170 | as pSJ13835, FRT-F-SP(B.pum yckD)-11 N-term codon from Protease 2-Amylase-FRT-F3 |
| pBT11171 | as pSJ13835, FRT-F-SP(B.pum yckD)-12 N-term codon from Protease 2-Amylase-FRT-F3 |
| pBT11172 | as pSJ13835, FRT-F-SP(B.pum yckD)-13 N-term codon from Protease 2-Amylase-FRT-F3 |
| pBT11173 | as pSJ13835, FRT-F-SP(B.pum yckD)-14 N-term codon from Protease 2-Amylase-FRT-F3 |
| pBT11174 | as pSJ13835, FRT-F-SP(B.pum yckD)-15 N-term codon from Protease 2-Amylase-FRT-F3 |
| pBT11178 | as pSJ13835, FRT-F-SP(B.pum yckD)-7 N-term codon from Protease 2-Amylase-FRT-F3 |
| pBT11176 | as pSJ13835, FRT-F-SP(B.pum yckD)-7 N-term codon from Protease 1-Amylase-FRT-F3 |
| pBT11177 | as pSJ13835, FRT-F-SP(B.pum yckD)-Amylase-FRT-F3 |

**Identification of SP-linkers**

With the help of SP-linkers, as shown in the following examples, productivity and/or yield of recombinant proteins can be significantly increased. Below description is a non-limiting example of how to identify a combination of signal peptide and SP-linker which increases the yield for a polypeptide of interest. Although below description focuses on the heterologous combinations of SP and SP-linker, SP-linkers which are endogenous to the SP may be identified too.

First, a plurality or library of first polynucleotide sequences is provided, wherein each first polynucleotide is encoding a signal peptide. Each signal peptide is derived from a first donor polypeptide. The first donor is a polypeptide which is natively expressed together with the signal peptide. Then, a first polypeptide (second donor) is produced within a host cell, wherein the polynucleotide encoding the first polypeptide is linked in translational fusion with the first polynucleotide encoding the signal peptide. For each combination of SP and first polypeptide, the yield of secreted first polypeptide is measured. Each signal peptide is ranked based on the yield of secreted first polypeptide (second donor). The ranking may be from top (highest secretion or yield) to bottom (lowest secretion or yield). Optionally, the yield of secreted first polypeptide is compared with the yield of secreted first polypeptide when expressed in translational fusion with one or more reference signal peptide(s). Notably, the secreted first polypeptide identified as second donor, is not necessarily the polypeptide of interest for which the identified SP will be used in the final host cell.

Next, one or more of the first polynucleotides encoding the signal peptides ranked in the top 10% are selected, preferably in the top 5%, the top 4%, the top 3%, the top 2%, or the top 1 %. Additionally or alternatively, one or more first polynucleotide each encoding a signal peptide having increased yield relative to the yield of the reference signal peptide(s) are selected.

One or more of the selected first polynucleotide(s) encoding the selected SP sequence is fused with a second polynucleotide encoding a SP-linker. The SP-linker is comprising or consisting of 2-15 N-terminal amino acids from the secreted first polypeptide (second donor) The generated construct comprises the second polynucleotide encoding the SP-linker downstream of the first polynucleotide encoding the SP sequence. Finally, the polypeptide of interest in translational fusion with the fusion construct is expressed in a recombinant host cell.

The fusion construct comprising the SP and SP-linker results in increased secretion/yield of the polypeptide of interest relative to the secretion/yield of the polypeptide of interest with the signal peptide alone. Typically, the second polynucleotide encoding the SP-linker, and the first polynucleotide encoding the signal peptide are heterologous to the third polynucleotide encoding the polypeptide of interest.

**Example 1: Cloning of Protease 1 strain with aspartate phosphatase signal peptide**

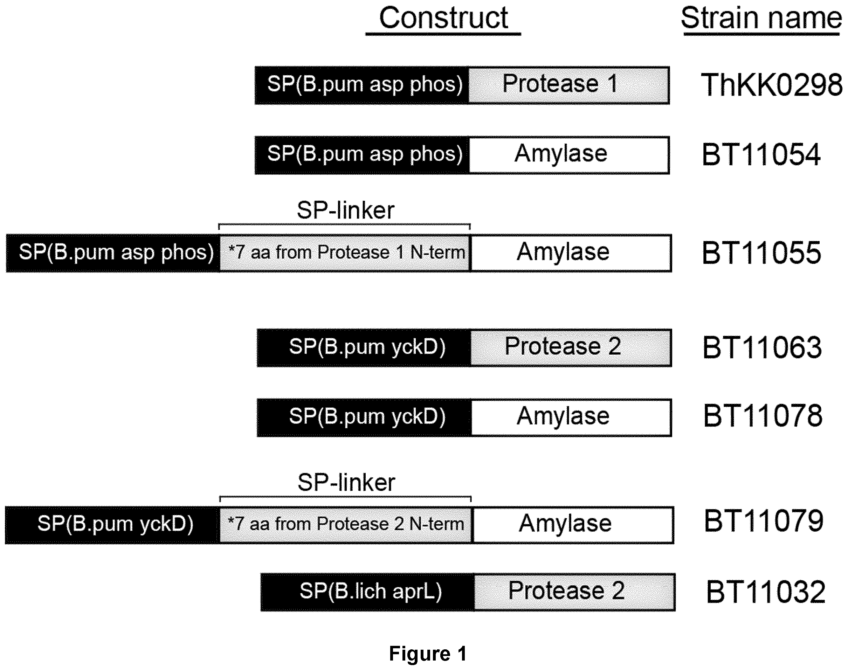

The signal peptide sequence with SEQ ID NO: 3 (encoded by SEQ ID NO: 1) from the aspartate phosphotase from *B. pumilus* (SEQ ID NO: 18, first donor) was identified as an efficient signal peptide for expression of protease 1 (SEQ ID NO: 17, second donor). Synthetic DNA #1 (SEQ ID NO: 127) was ordered to contain the protease 1 expression cassette under control of the triple promoter (SEQ ID NO: 20) (as described in WO 99/43835) and with the signal peptide sequence from the aspartate phosphotase from *B. pumilus*. The protease 1 expression cassette was combined with upstream ara flanking region including the triple promoter (SEQ ID NO: 128) and downstream flanking region (SEQ ID NO: 129) of the ara locus, including the ERM selection marker, in a POE PCR (Table 1). The generated material was used for transformation into MOL3320 as described in patent US 2019/0185847 A1. Selection was done on ERM. The resulting strain was named ThKK0298 as depicted in Figure 1.

**Table 1**

| 1. PCRs | | | | POE-PCR |
|---|---|---|---|---|
| Fragment | Template DNA from | Primers | Size | Template |
| Ara upstream +promoter (A) (SEQ ID NO: 128) | MOL3320 | 77257-POE-106-832 (SEQ ID NO:130) + pep0839 (SEQ ID NO: 131) | App. 3900bp | A+B+C |
| Synthetic DNA #1 (B) (SEQ ID NO: 127) | Synthetic DNA #1 | | App. 1800bp | |
| ERM+ara downstream (C) (SEQ ID NO: 129) | MOL3320 | OTK0657 (SEQ ID NO: 132) + 77262-POE 106 831 (SEQ ID NO: 133) | App. 4300bp | |

**Example 2: Cloning of Protease 2 strain with yckD signal peptide**

The signal peptide sequence with SEQ ID NO: 4 (encoded by SEQ ID NO: 2) from the *yckD* gene from *B. pumilus* encoding the polypeptide with SEQ ID NO: 19 (first donor) was identified as an efficient signal peptide for expression of Protease 2 (SEQ ID NO: 16, second donor). Synthetic DNA #2 (SEQ ID NO: 134) was ordered to contain the Protease 2 expression cassette under control of the triple promoter (as described in WO 99/43835) and with the signal peptide sequence from the *yckD* gene from *B. pumilus*. The Protease 2 expression cassette was combined with upstream ara flanking region including the triple promoter (SEQ ID NO: 128) and downstream flanking region (SEQ ID NO: 129) of the ara locus, including the ERM selection marker, in a POE PCR (Table 2). The generated material was used for transformation into MOL3320 as described in patent US 2019/0185847 A1. Selection was done on ERM. The resulting strain was named BT11063 (see Figure 1).

**Table 2**

| 1. PCRs | | | | POE-PCR |
|---|---|---|---|---|
| Fragment | Template DNA from | Primers | Size | Template |
| Ara upstream +promoter (A) (SEQ ID NO: 128) | MOL3320 | 77257-POE-106-832 (SEQ ID NO: 130) + pep0839 (SEQ ID NO: 131) | App. 3900bp | A+B+C |
| Synthetic DNA #2 (B) (SEQ ID NO: 134) | Synthetic DNA #2 | | App. 1800bp | |
| ERM+ara downstream (C) (SEQ ID NO: 129) | MOL3320 | OTK0657 (SEQ ID NO: 132) + 77262-POE 106 831 (SEQ ID NO: 133) | App. 4300bp | |

**Example 3: Cloning of Amylase cassette with aspartate phosphatase signal peptide**

The first polynucleotide with SEQ ID NO: 1 encoding the signal peptide sequence of SEQ ID NO: 3 from the *aspartate phosphotase* gene from *B. pumilus* from ThKK0298 was placed in front of the Amylase gene. Synthetic DNA #3 (SEQ ID NO: 127) was ordered to contain the Amylase expression cassette under control of the triple promoter (as described in WO 99/43835) and with the signal peptide sequence from the *aspartate phosphotase* gene from *B. pumilus*. The Amylase expression cassette was combined with upstream ara flanking region including the triple promoter (SEQ ID NO: 128) and downstream flanking region (SEQ ID NO: 129) of the ara locus, including the ERM selection marker, in a POE PCR (Table 2). The generated material was used for transformation into MOL3320 as described in patent US 2019/0185847 A1. Selection was done on ERM. The resulting strain was named BT11054 (Figure 1).

**Table 3**

| 1. PCRs | | | | POE-PCR |
|---|---|---|---|---|
| Fragment | Template DNA from | Primers | Size | Template |
| Ara upstream +promoter (A) (SEQ ID NO: 128) | MOL3320 | 77257-POE-106-832 (SEQ ID NO: 130) + pep0839 (SEQ ID NO: 131) | App. 3900bp | A+B+C |
| Synthetic DNA # 3(B) (SEQ ID NO: 135) | Synthetic DNA #3 | | App. 2200bp | |
| ERM+ara down stream (C) (SEQ ID NO: 129) | MOL3320 | OTK0657 (SEQ ID NO: 132) + 77262-POE 106 831 (SEQ ID NO: 133) | App. 4300bp | |

**Example 4: Cloning of Amylase cassette with SP-linker derived from Protease 1**

The first polynucleotide with SEQ ID NO: 1 (encoding the signal peptide sequence of SEQ ID NO: 3) from the *aspartate phosphotase* gene from *B. pumilus,* plus the second polynucleotide with SEQ ID NO: 5 encoding 7 amino acids/codons (SP-linker with SEQ ID NO: 7) from the N-terminal of the protease 1 pro-peptide from ThKK0298, forming the SP - SP-linker polynucleotide construct with SEQ ID NO: 9 and encoding the SP - SP-linker polypeptide with SEQ ID NO: 11, was placed in front of the Amylase gene (Figure 1). Synthetic DNA #4 (SEQ ID NO: 136) was ordered to contain the Amylase expression cassette under control of the triple promoter (as described in WO 99/43835) and with the signal peptide sequence from the *aspartate phosphotase* gene from *B. pumilus* plus the 7 codons/amino acids from the N-terminal protease 1 pro-peptide. The Amylase expression cassette was combined with upstream ara flanking region including the triple promoter (SEQ ID NO: 128) and downstream flanking region (SEQ ID NO: 129) of the ara locus, including the ERM selection marker, in a POE PCR (Table 2). The generated material was used for transformation into MOL3320 as described in patent US 2019/0185847 A1. Selection was done on ERM. The resulting strain was named BT11055, see Figure 1.

**Table 4**

| 1. PCRs | | | | POE-PCR |
|---|---|---|---|---|
| Fragment | Template DNA from | Primers | Size | Template |
| Ara upstream +promoter (A) (SEQ ID NO: 128) | MOL3320 | 77257-POE-106-832 (SEQ ID NO: 130) + pep0839 (SEQ ID NO: 131) | App. 3900bp | A+B+C |
| Synthetic DNA # 4(B) (SEQ ID NO: 136) | Synthetic DNA #4 | | App. 2200bp | |
| ERM+ara downstream (C) (SEQ ID NO: 129) | MOL3320 | OTK0657 (SEQ ID NO: 132) + 77262-POE 106 831 (SEQ ID NO: 133) | App. 4300bp | |

**Example 5: Cloning of Amylase cassette with yckD signal peptide**

The polynucleotide with SEQ ID NO: 2 encoding the signal peptide sequence of SEQ ID NO: 4) from the *yckD* gene from *B. pumilus* was placed in front of the Amylase gene. Synthetic DNA #5 (SEQ ID NO: 137) from BT11063 was ordered to contain the Amylase expression cassette under control of the triple promoter (as described in WO 99/43835) and with the signal peptide sequence from the *yckD* gene from *B. pumilus.* The Amylase expression cassette was combined with upstream ara flanking region including the triple promoter (SEQ ID NO: 128) and downstream flanking region (SEQ ID NO: 129) of the ara locus, including the ERM selection marker, in a POE PCR (Table 2). The generated material was used for transformation into MOL3320 as described in patent US 2019/0185847 A1. Selection was done on ERM. The resulting strain was named BT11078 (see Figure 1).

**Table 5**

| 1. PCRs | | | | POE-PCR |
|---|---|---|---|---|
| Fragment | Template DNA from | Primers | Size | Template |
| Ara upstream +promoter (A) (SEQ ID NO: 128) | MOL3320 | 77257-POE-106-832 (SEQ ID NO: 130) + pep0839 (SEQ ID NO: 131) | App. 3900bp | A+B+C |
| Synthetic DNA # 5 (B) (SEQ ID NO: 137) | Synthetic DNA #5 | | App. 2200bp | |
| ERM+ara downstream (C) (SEQ ID NO: 129) | MOL3320 | OTK0657 (SEQ ID NO: 132) + 77262-POE 106 831 (SEQ ID NO: 133) | App. 4300bp | |

**Example 6: Cloning of Amylase cassette with SP-linker derived from Protease 2**

The first polynucleotide with SEQ ID NO: 2 (encoding the signal peptide sequence of SEQ ID NO: 4) from the *yckD* gene from *B. pumilus,* plus the second polynucleotide with SEQ ID NO: 6 encoding 7 amino acids/codons (SP-linker with SEQ ID NO: 8) from the N-terminal of the protease 2 pro-peptide from BT1 1063, forming the SP - SP-linker polynucleotide construct with SEQ ID NO: 10 and encoding the SP - SP-linker polypeptide with SEQ ID NO: 12, was placed in front of the Amylase gene. Synthetic DNA #6 (SEQ ID NO: 99) was ordered to contain the Amylase expression cassette under control of the triple promoter (as described in WO 99/43835) and with the signal peptide sequence from the *yckD* gene plus 7 amino acids/codons from the N-terminal of the Protease 2 pro-peptide from *B. pumilus* in BT11063. The Amylase expression cassette was combined with upstream ara flanking region including the triple promoter (SEQ ID NO: 128) and downstream flanking region (SEQ ID NO: 129) of the ara locus, including the ERM selection marker, in a POE PCR (Table 2). The generated material was used for transformation into MOL3320 as described in patent US 2019/0185847 A1. Selection was done on ERM. The resulting strain was named BT11079 (see Figure 1).

**Table 6**

| 1. PCRs | | | | POE-PCR |
|---|---|---|---|---|
| Fragment | Template DNA from | Primers | Size | Template |
| Ara upstream +promoter (A) (SEQ ID NO: 128) | MOL3320 | 77257-POE-106-832 (SEQ ID NO: 130) + pep0839 (SEQ ID NO: 131) | App. 3900bp | A+B+C |
| Synthetic DNA # 6 (B) (SEQ ID NO: 99) | Synthetic DNA #6 | | App. 2200bp | |
| ERM+ara downstream (C) (SEQ ID NO: 129) | MOL3320 | OTK0657 (SEQ ID NO: 132) + 77262-POE 106 831 (SEQ ID NO: 133) | App. 4300bp | |

**Example 7: Cloning of protease 2 expression cassette with standard signal peptide**

The signal peptide with SEQ ID NO: 81 (encoded by SEQ ID NO: 82) from *B. Licheniformis aprL* was used as reference signal peptide for protease 2 expression. Synthetic DNA #7 (SEQ ID NO: 100) was ordered to contain the protease 2 expression cassette under control of the triple promoter (as described in WO 99/43835) and with the signal peptide sequence from *aprL* from *B. licheniformis.* The protease 2 expression cassette was combined with upstream ara flanking region including the triple promoter (SEQ ID NO: 128) and downstream flanking region (SEQ ID NO: 129) of the ara locus, including the ERM selection marker, in a POE PCR (Table 7). The generated material was used for transformation into MOL3320 as described in patent US 2019/0185847 A1. Selection was done on ERM. The resulting strain was named BT11032 (see Figure 1).

**Table 7**

| 1. PCRs | | | | POE-PCR |
|---|---|---|---|---|
| Fragment | Template DNA from | Primers | Size | Template |
| Ara upstream +promoter (A) (SEQ ID NO: 128) | MOL3320 | 77257-POE-106-832 (SEQ ID NO: 130) + pep0839 (SEQ ID NO: 131) | App. 3900bp | A+B+C |
| Synthetic DNA #7 (B) (SEQ ID NO: 100) | Synthetic DNA #7 | | App. 1800bp | |
| ERM+ara downstream (C) (SEQ ID NO: 129) | MOL3320 | OTK0657 (SEQ ID NO: 132) + 77262-POE 106 831 (SEQ ID NO: 133) | App. 4300bp | |

**Example 8: Adding a SP-linker from Protease 1 to an Amylase polypeptide improves expression and/or secretion of the Amylase.**

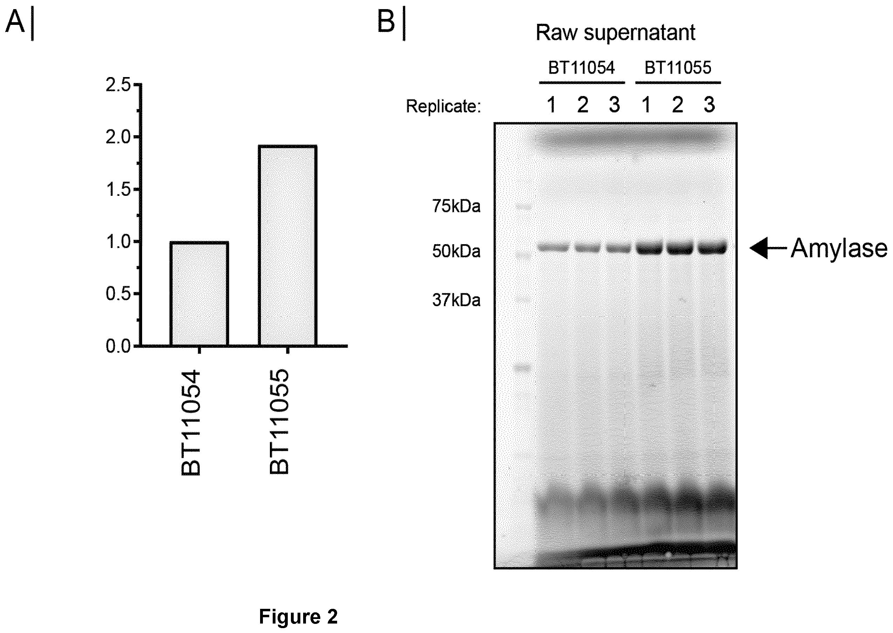

To explore the expression capacity of including the SP-linker together with the signal peptide on another product gene, the signal peptide with the amino acid sequence of SEQ ID NO: 3 and the same signal peptide plus SP-linker at the C-terminal end of the signal peptide, represented by SEQ ID NO: 11, from ThKK0298 was transferred to an amylase, strain BT11054 (SP only) and BT11055 (SP - SP-linker fusion), respectively. The strains were fermented for 24hrs in TY media in the Biolector. Amylase activity was determined, and amylase activity was normalized/benchmarked to BT11054. The construct including the SP-linker, BT11055 with the amylase of SEQ ID NO: 14, resulted in a app. 100% increase in amylase productivity (Figure 2A, n=3).

Adding the SP-linker will result in a 7 amino acid N-terminal extension of the amylase. To examine whether the app. 100% increase in productivity was due to a more active amylase, because of the 7AA extension, raw supernatants from the Biolector fermentation was analyzed on SDS page gel (Figure 2B). The gel shows that more Amylase was made in BT11055 (signal peptide plus SP-linker), thus the increase in activity is due to increased production of Amylase.

**Example 9: Adding a SP-linker from Protease 2 to an Amylase polypeptide improves expression and/or secretion of the Amylase.**

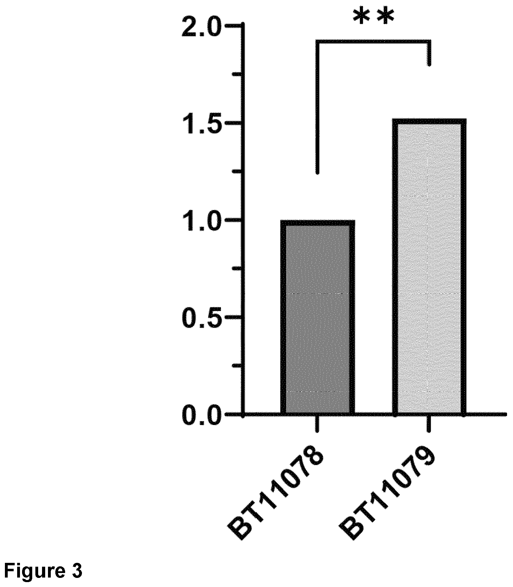

To explore if the improved expression capacity by including the SP-linker from ThKK00298 to an Amylase was specific for the construct in BT11055, the signal peptide with the amino acid sequence of SEQ ID NO: 4, and the same signal peptide plus SP-linker at the C-terminal end of the signal peptide, represented by SEQ ID NO: 12, from BT11063 was transferred to the same Amylase, BT11078 (SP only) and BT11079 (SP - SP-linker fusion), respectively. The strains were fermented in the Biolector. Amylase activity was determined, and amylase activity was normalized/benchmarked to BT11054. The construct including the SP-linker, with the amylase of SEQ ID NO: 15, resulted in a app. 50% increase in amylase productivity (Figure 3, n=3). This indicates that a combination of SP - SP-linker construct to increase polypeptide yield is generally applicable.

**Example 10: The yckD signal peptide in combination with the protease 2 SP-linker achieves high productivities**

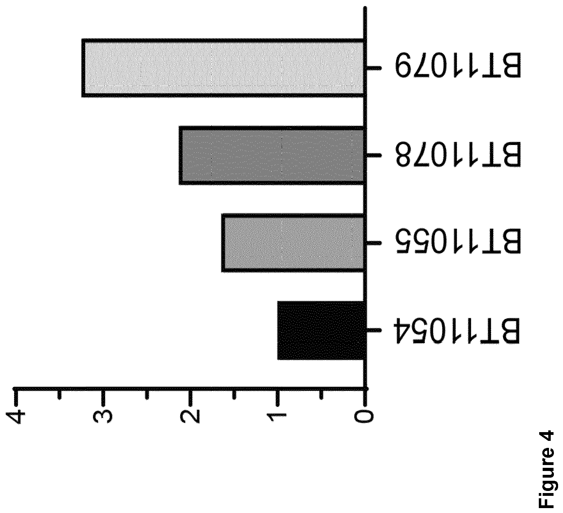

To benchmark the signal peptides and SP-linkers to each other, BT11054 (SP only), BT11055 (SP - SP-linker fusion), BT11078 (SP only) and BT11079 (SP - SP-linker fusion) were grown in the Biolector. Amylase activity was determined and normalized to BT11054. The data shows that the *yckD* signal peptide plus the SP-linker derived from protease 2, represented by SEQ ID NO: 12, in BT11079 performed the best, producing app. 230% more amylase than the aspartate phosphatase signal peptide in BT11054 (SP only, no SP-linker) (Figure 4, n=3).

**Example 11: yckD as an optimal signal peptide for Protease 2**

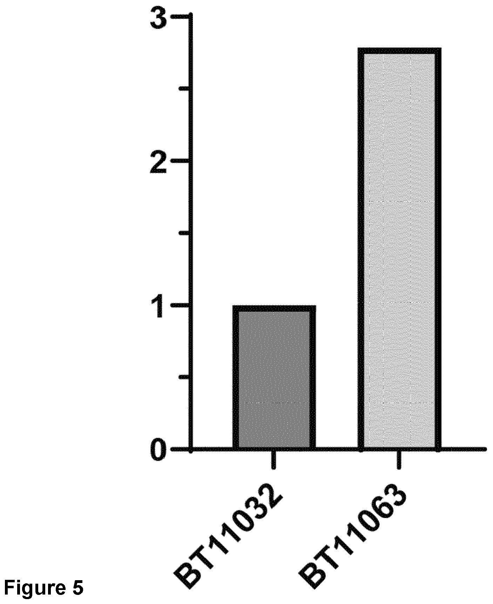

The expression from the yckD signal peptide fused to Protease 2 in BT11063 was benchmarked against the reference signal peptide (aprL SP) in BT11032. BT11032 and BT11063 were cultivated for 3 days in small scale fermentations in microtiter plates and protease activity was analyzed. Protease activity was normalized to BT11032. BT11063 displayed a 2.7-fold increase in productivity compared to the reference signal peptide in BT11032 (Figure 5, n=1). Surprisingly, the *yckD* signal peptide has been found to increase protease expression significantly, compared to a well-established reference signal peptide which is industrially used to express high levels of proteases.

**Example 12: Single copy Amylase reference strains**

As reference strains, single copy Amylase *B. licheniformis* strains were made. A POEs was made consisting of the Amylase with its standard SP (SEQ ID NO: 13, encoding sequence SEQ ID NO: 169) and the Amylase with the yckD SP (SEQ ID NO: 2) and plasmid elements needed to generate flp-FRT donor plasmids (WO 2018/077796 A1), resulting in plasmid pSJ13835 and pBT11177. POEs were transformed into *B. subtilis* donor PP3724, to generate conjugation donors SJ13837 and BT11177. To generate single copy *B. licheniformis* strains, SJ13837 and BT11177 were conjugated with SJ14358 harboring a single flp-FRT integration site (WO 2018/077796 A1), resulting in the following strains: BT11146 (no SP-linker) and BT11211 (with 7 AA SP-linker of SEQ ID NO:8).

**Example 13: Screening for optimal SP-linker length**

Table 8.

| Strain | SEQ info | sequence (yckD SP and SP-linker) |
|---|---|---|
| pBT11161 | SEQ ID NO:153 | MMKKVSGLIILSMMLLSGFYMETAYGAA |
| pBT11162 | SEQ ID NO:154 | MMKKVSGLIILSMMLLSGFYMETAYGAAE |
| pBT11163 | SEQ ID NO:155 | MMKKVSGLIILSMMLLSGFYMETAYGAAEE |
| pBT11164 | SEQ ID NO:156 | MMKKVSGLIILSMMLLSGFYMETAYGAAEEA |
| pBT11165 | SEQ ID NO:157 | MMKKVSGLIILSMMLLSGFYMETAYGAAEEAK |
| pBT11166 | SEQ ID NO:158 | MMKKVSGLIILSMMLLSGFYMETAYGAAEEAKE |
| pBT11167 | SEQ ID NO:159 | MMKKVSGLIILSMMLLSGFYMETAYGAAEEAKEKY |
| pBT11168 | SEQ ID NO:160 | MMKKVSGLIILSMMLLSGFYMETAYGAAEEAKEKYL |
| pBT11169 | SEQ ID NO:161 | MMKKVSGLIILSMMLLSGFYMETAYGAAEEAKEKYLI |
| pBT11170 | SEQ ID NO:162 | MMKKVSGLIILSMMLLSGFYMETAYGAAEEAKEKYLIG |
| pBT11171 | SEQ ID NO:163 | MMKKVSGLIILSMMLLSGFYMETAYGAAEEAKEKYLIGF |
| pBT11172 | SEQ ID NO:164 | MMKKVSGLIILSMMLLSGFYMETAYGAAEEAKEKYLIGFN |
| pBT11173 | SEQ ID NO:165 | MMKKVSGLIILSMMLLSGFYMETAYGAAEEAKEKYLIGFNE |
| pBT11174 | SEQ ID NO:166 | MMKKVSGLIILSMMLLSGFYMETAYGAAEEAKEKYLIGFNEQ |
| pBT11178 | SEQ ID NO:167 | MMKKVSGLIILSMMLLSGFYMETAYGAAEEAKEK |

To identify the optimal linker length, the 1 to 15 amino acids from the mature N-terminal end of Protease 2 (SEQ ID NO: 16, second donor polypeptide) was inserted between the *yckD* signal peptide from *B. pumilus* (SEQ ID NO: 2 encoding the signal peptide sequence of SEQ ID NO: 4) and the Amylase mature 5'-end (amylase is encoded by SEQ ID NO: 168). These constructs were ordered as synthetic DNA fragments (Synthetic DNA #8-22, SEQ ID NO: 138-152, encoding sequences of SEQ ID NO: 153-167, each of SEQ ID NO: 153-167 representing a SP-linker with length of 1 to 15 AA) and assembled into donor plasmids (pBT11161 - pBT11174 + pBT11178) by POE.The POEs were transformed into *B. subtilis* donor PP3724, to generate conjugation donors BT11161-BT11174 + BT11178. To generate single copy *B. licheniformis* strains, donors mentioned above were conjugated with SJ14358 harboring a single flp-FRT integration site (WO2018/077796 A1), resulting in the following strains: BT11195-BT11208 + BT11212, see table 8 for SP-linker length.

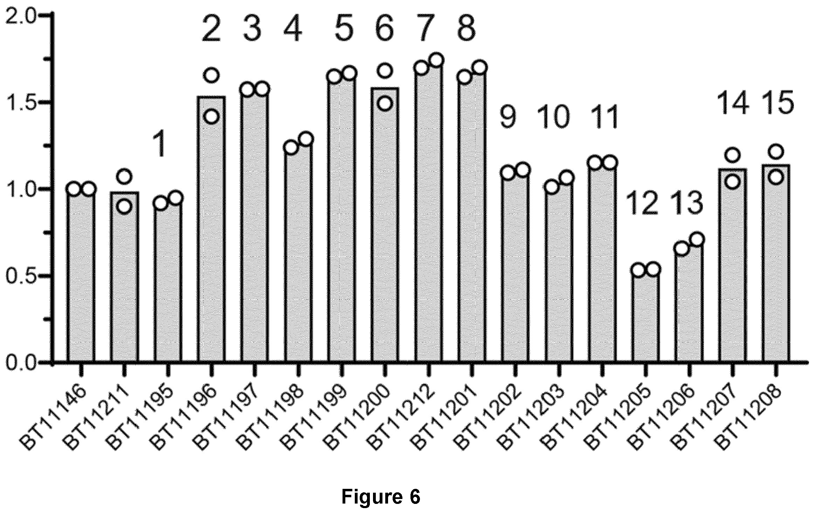

Strains were evaluated in Biolector fermentations, in duplicates, and amylase activity was assayed. As can be seen from Figure 6, productivity was normalized to the reference strain BT11146 (no SP-linker). In Figure 6, for each strain comprising an SP-linker the linker length is indicated by a number 1-15 representing the number of AA of which the SP-linker consists of. Compared to control strains BT11146 and BT11211 (no SP-linker) an increase in productivity of ca. 20-70% was observed for strains BT11196 - BT11200, BT11212, and BT11201, harboring SP-linkers with a SP-linker length ranging from 2-8 amino acids. The SP-linkers with a length of 2-8 AA are the linkers with following AA sequences: AE", "AEE", SEQ ID NO: 52 "AEEA", SEQ ID NO: 53 "AEEAK", SEQ ID NO: 54 "AEEAKE", SEQ ID NO: 55 "AEEAKEK", and SEQ ID NO: 56 "AEEAKEKY". According to these results, a SP-linker consisting of 2-8 AA is advantageous.

**Example 14: Lab scale fermentation**

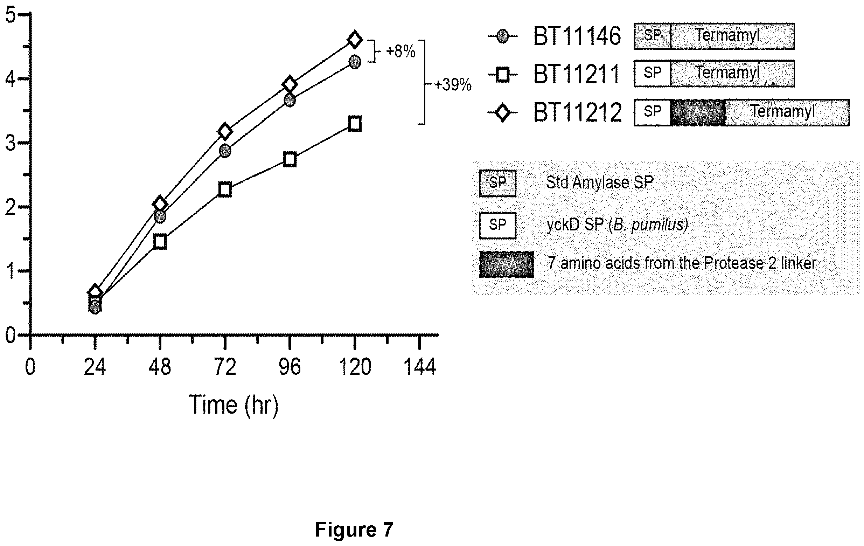

To evaluate the effect of the linker addition under industrial fermentation conditions, BT11212 (SP(B.pum yckD)-7 N-term codon from Protease 2-Amylase) with the SP-linker of SEQ ID NO:8was grown in lab-scale bioreactor. The strain was benchmarked against the standard Amylase expression construct (BT11146, no SP-linker) and the SP(B.pum yckD)-Amylase construct (BT11211, no SP-linker) (Figure 7). Strains were fermented for app. 120 hours and amylase acitivty was meassured every 24 hours (arbitrary expression units plotted on y-axis). The BT11212 strain with a 7 AA long SP-linker (=7 AA N-terminal end of protease 2) outperformed the standard construct BT11146 (no SP-linker) by 8% on the final day of fermentation. Additionally, the 7 AA SP-linker construct (BT11212) produced app. 39% more amylase compared to the control strain only carrying the yckD signal peptide (BT11211, no SP-linker).

**Example 15: Swapping of SP and SP-linker maintains increased product yield**

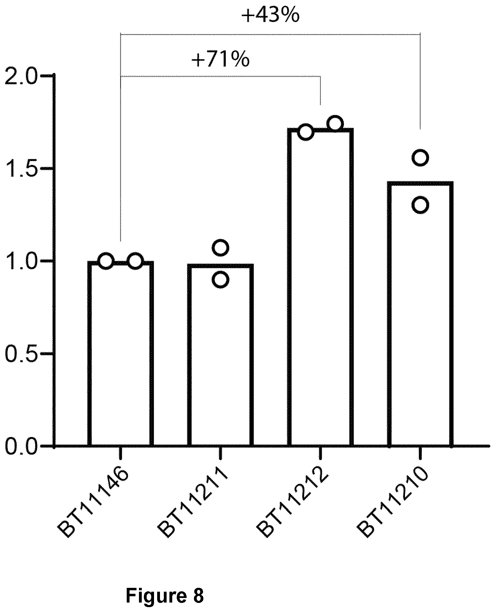

The SP-linker from protease 1 (SEQ IN NO: 5 encoding the SP-linker of SEQ ID NO: 7) is swapped out with the SP-linker from protease 2 (SEQ IN NO: 6 encoding the SP-linker of SEQ ID NO: 8) in pBT11178 with synthetic DNA #23 (SEQ ID NO: 169, encoding SEQ ID NO: 170) by POE PCR to generate donor plasmid pBT11176. PBT11176 was transformed into *B. subtilis* donor PP3724, to generate conjugation donor BT11176, see Table 9 for an overview of constructs. To generate single copy *B. licheniformis* strains, BT11176 was conjugated with SJ14358 harboring a single flp-FRT integration site (as described in WO 2018/077796 A1), resulting in BT11210 (yckD SP + 7AA SP-linker from protease 1). Strains were evaluated in Biolector fermentations, in duplicates, and amylase activity was assayed. Productivity was normalized to the reference strains BT11146 (Figure 8). Relative to the standard amylase control (BT11146) and the control with the yckD signal peptide without any SP-linker (BT11211), the construct with the 7 amino acid SP-linker from protease 2 (BT11212) yields app. 71% more amylase, whereas the construct with the yckD signal peptide and the 7 amino acid SP-linker from protease 1 (BT11210) yields app. 43% more amylase. Thus, swapping a SP-linker from one signal peptide construct to another signal peptide construct improves productivity, compared to strains without SP-linker sequence, and demonstrates the strength of using SP-linkers in expression cassettes.

**Table 9**

| Strain | SP derived from: | SP-linker, derived from N-terminal end of: | polypeptide of interest | Relative amylase yield (Fig. 8) |
|---|---|---|---|---|
| BT11055 | asp phosphatase | protease 1 | amylase (Termamyl) | n.a. |
| BT11079 | yckD | protease 2 | amylase (Termamyl) | n.a. |
| BT11146 | amylase | no SP-linker | amylase (Termamyl) | 100% |
| BT11211 | yckD | no SP-linker | amylase (Termamyl) | 100% |
| BT11212 | yckD | protease 2 | amylase (Termamyl) | 171% |
| BT11210 | yckD | protease 1 | amylase (Termamyl) | 143% |

**Example 16: Adding a SP-linker from Xylanase or Endonuclease to a Phytase polypeptide improves expression and/or secretion of the Phytase in A. *oryzae.***

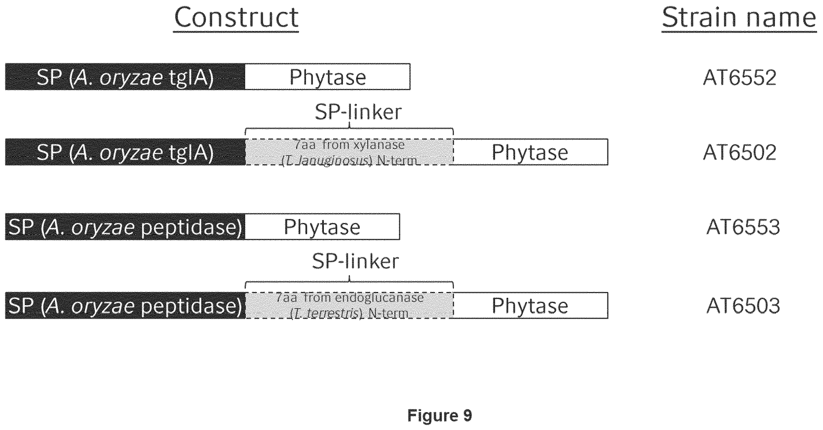

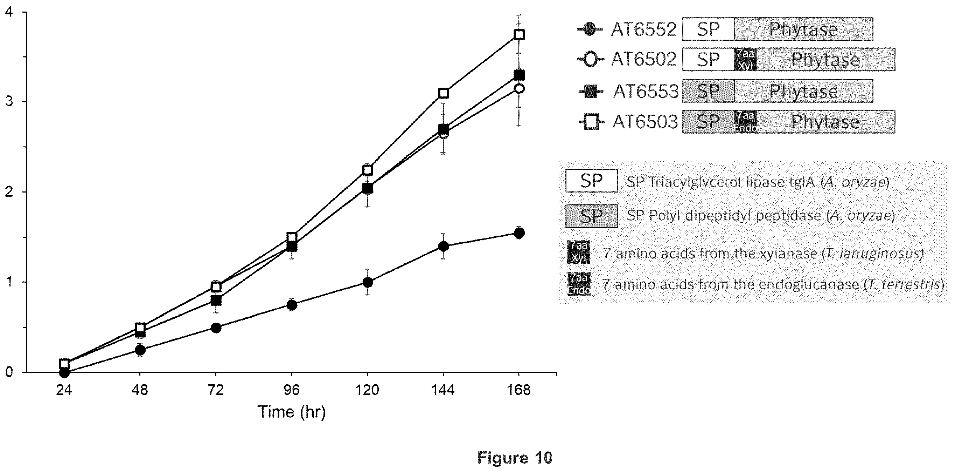

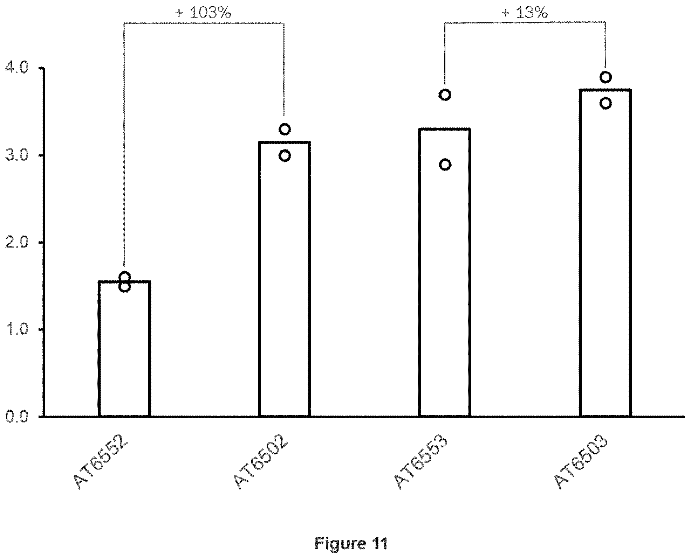

The previous examples show that SP-linkers can be utilized to increase product yield in bacterial cells. This example explores if the expression capacity of fungal cells, e.g. *Aspergillus oryzae,* can be improved by including the SP-linker to a Phytase coding gene. The *A. oryzae* triacylglycerol lipase signal peptide with the amino acid sequence of SEQ ID NO: 171, and the same signal peptide plus SP-linker from Thermomyces lanuginosus xylanase at the C-terminal end of the signal peptide, represented by SEQ ID NO: 173, was transferred to the same Phytase in 1-copy, strain AT6552 (SP only) and strain AT6502 (SP - SP-linker fusion), respectively (Figure 9 & Table 10, 11). The *A. oryzae* polyl dipeptidyl peptidase signal peptide with the amino acid sequence of SEQ ID NO: 172, and the same signal peptide plus SP-linker from Thermothielavioides terrestris endoglucanase at the C-terminal end of the signal peptide, represented by SEQ ID NO: 174, was transferred to the same Phytase in 1- copy, strain AT6553 (SP only) and strain AT6503 (SP - SP-linker fusion), respectively (Figure 9 & Table 10, 11). The strains were fermented in lab-scale fermentation and the phytase activity was determined. The construct including the SP-linker, with the phytase of SEQ ID NO: 173, resulted in an app. 103% increase in phytase productivity at the end of the fermentation when compared to the same strain lacking the SP-linker (Figures 10 & 11). The construct including the SP-linker, with the phytase of SEQ ID NO: 174, resulted in an app. 13% increase in phytase productivity at the end of the fermentation when compared to the same strain lacking the SP-linker (Figures 10 & 11). This indicates that the concept of an combination of SP - SP-linker construct to increase polypeptide yield is generally applicable to filamentous fungi.

**Table 10**

| Strain | SEQ info | sequence (SP and SP-linker) |
|---|---|---|
| AT6552 | SEQ ID NO:171 | MHLAIKSLFVSLLGASVLASPLPSNALVERA |
| AT6502 | SEQ ID NO:173 | MHLAIKSLFVSLLGASVLASPLPSNALVERAFPAGNAT |
| AT6553 | SEQ ID NO:172 | MKYSKLLLLLVSVVQA |
| AT6503 | SEQ ID NO:174 | MKYSKLLLLLVSVVQAASGSGQS |

**Table 11**

| SEQ info | SP and SP-linker | DNA sequence (SP, SP-linker and intron) |
|---|---|---|
| SEQ ID NO: 187 | *A. oryzae* triacylglycerol lipase SP + N-terminal amino acids of *T. lanuginosus* xylanase | atgcatcttgctatcaagtctctctttgtct ctctcctcggagccagcgttctcgcaa gccctcttcccagcaatgctctggttga gagagccttcccggcagggaatgcc acg |
| SEQ ID NO: 188 | *A. oryzae* prolyl dipeptidyl peptidase SP + N-terminal amino acids of *T. terrestris* endoglucanase | Atgaaggtacgtcaattccactgatta aacattatttgttacatacactccatcatt gagtcaattataattaacacctcataat tcagtactccaagcttctgctgctcctg gtcagtgtggtccaggccgccagtgg cagtggccagtcc |

Sequence overview

SEQ ID NO: 1, *B. pumilus* aspartate phosphatase SP coding sequence
SEQ ID NO: 2, *B. pumilus* yckD SP coding sequence
SEQ ID NO: 3, *B. pumilus* aspartate phosphatase SP
SEQ ID NO: 4, *B. pumilus* YckD SP
SEQ ID NO: 5, 7 AA SP-linker from protease 1
SEQ ID NO: 6, 7 AA SP-linker from protease 2
SEQ ID NO: 7, 7 AA SP-linker from protease 1
SEQ ID NO: 8, 7 AA SP-linker from protease 2
SEQ ID NO: 9, asp phosphatase SP+SP linker protease 1 coding sequence

SEQ ID NO: 10, yckD SP+SP linker protease 2 coding sequence

SEQ ID NO: 11, *B. pumilus* asp phosphatase SP+ SP linker protease 1

SEQ ID NO: 12, *B. pumilus* yckD SP+ SP linker protease 2

SEQ ID NO: 13 amylase coding sequence

SEQ ID NO: 14 amylase AA sequence with protease 1 SP linker

SEQ ID NO: 15 amylase AA sequence with protease 2 SP linker

SEQ ID NO: 16 AA sequence of protease 2

SEQ ID NO: 17 AA sequence of protease 1

SEQ ID NO: 18 AA sequence of B. pumilus aspartate phosphatase

SEQ ID NO: 19 AA sequence of yckD

SEQ ID NO: 20 P3 promoter

SEQ ID NO: 21 *B. licheniformis* SPase AA yaaT

SEQ ID NO: 22 *B. licheniformis* SPase AA sipV

SEQ ID NO: 23 *B. licheniformis* SPase AA sipT

SEQ ID NO: 24 -48: N-terminal amino acids of protease 1

SEQ ID NO: 49 *B. licheniformis* SPase AA lspA

SEQ ID NO: 50 *B. licheniformis* SPase AA sipS

SEQ ID NO: 51 *B. licheniformis* SPase AA sipW

SEQ ID NO: 52 - 76: N-terminal amino acids of protease 2

SEQ ID NO: 77 coding sequence amylase with protease 1 SP linker

SEQ ID NO: 78 coding sequence amylase with protease 2 SP linker

SEQ ID NO: 79 7 N-terminal AA of *B. pumilus* asp phosphatase

SEQ ID NO: 80 yckD SP + protease 2

SEQ ID NO: 81 *B. licheniformis aprL* SP AA

SEQ ID NO: 82 *B. licheniformis aprL* SP DNA

SEQ ID NO: 83 - 96: N-terminal AA B. pumilus asp polypeptide

SEQ ID NO: 97 7 N-terminal AA from yckD

SEQ ID NO: 98 B. licheniformis SPase LepB

SEQ ID NO: 99 synthetic DNA #6

SEQ ID NO: 100 synthetic DNA #7

SEQ ID NO: 101 - 126: N-terminal amino acids of yckD

SEQ ID NO: 127 synthetic DNA #1

SEQ ID NO: 128 Upstream ara flanking region + triple promoter

SEQ ID NO: 129 Downstream ara flanking region

SEQ ID NO: 130 Oligo 77257-POE-106-832

SEQ ID NO: 131 Oligo pep0839

SEQ ID NO: 132 Oligo oTK0657

SEQ ID NO: 133 Oligo 77262-POE 106 831

SEQ ID NO: 134 Synthetic DNA #2

SEQ ID NO: 135 Synthetic DNA#3

SEQ ID NO: 136 Synthetic DNA #4

SEQ ID NO: 137 Synthetic DNA #5

SEQ ID NO: 138 Synthetic DNA #8

SEQ ID NO: 139 Synthetic DNA #9

SEQ ID NO: 140 Synthetic DNA #10

SEQ ID NO: 141 Synthetic DNA #11

SEQ ID NO: 142 Synthetic DNA #12

SEQ ID NO: 143 Synthetic DNA #13

SEQ ID NO: 144 Synthetic DNA #14

SEQ ID NO: 145 Synthetic DNA #15

SEQ ID NO: 146 Synthetic DNA #16

SEQ ID NO: 147 Synthetic DNA #17

SEQ ID NO: 148 Synthetic DNA #18

SEQ ID NO: 149 Synthetic DNA #19

SEQ ID NO: 150 Synthetic DNA #20

SEQ ID NO: 151 Synthetic DNA #21

SEQ ID NO: 152 Synthetic DNA #22

SEQ ID NO: 153 pBT11161 (+1 AA)

SEQ ID NO: 154 pBT11162 (+2 AA)
SEQ ID NO: 155 pBT11163 (+3 AA)
SEQ ID NO: 156 pBT11164 (+4 AA)
SEQ ID NO: 157 pBT11165 (+5 AA)
SEQ ID NO: 158 pBT11166 (+6 AA)
SEQ ID NO: 159 pBT11167 (+8 AA)
SEQ ID NO: 160 pBT11168 (+9 AA)
SEQ ID NO: 161 pBT11169 (+10 AA)
SEQ ID NO: 162 pBT11170 (+11 AA)
SEQ ID NO: 163 pBT11171 (+12 AA)
SEQ ID NO: 164 pBT11172 (+13 AA)
SEQ ID NO: 165 pBT11173 (+14 AA)
SEQ ID NO: 166 pBT11174 (+15 AA)
SEQ ID NO: 167 pBT11178 (+7 AA)
SEQ ID NO: 168 amylase coding sequence
SEQ ID NO: 169 Synthetic DNA #23
SEQ ID NO: 170 pBT11176 (+7 AA)
SEQ ID NO: 171 *A. oryzae* triacylglycerol lipase SP
SEQ ID NO: 172 *A. oryzae* prolyl dipeptidyl peptidase SP
SEQ ID NO: 173 *A. oryzae* triacylglycerol lipase SP + N-terminal amino acids of xylanase
SEQ ID NO: 174 *A. oryzae* prolyl dipeptidyl peptidase SP + N-terminal amino acids of endoglucanase
SEQ ID NO: 175 encoding the SP-linker derived from the xylanase (used in SEQ ID NO: 173)
SEQ ID NO: 176 encoding the SP-linker derived from the endoglucanase (used in SEQ ID NO: 174)
SEQ ID NO: 177 coding sequence SP A. oryzae Lipase
SEQ ID NO: 178 coding sequence SP A. oryzae peptidase
SEQ ID NO: 179 AA sequence SP-linker Thermomyces lanuginosus xylanase "FPAGNAT"
SEQ ID NO: 180 AA sequence SP-linker Thermothielavioides terrestris endoglucanase "ASGSGQS"
SEQ ID NO: 181 "FPAG"
SEQ ID NO: 182 "FPAGN"
SEQ ID NO: 183 "FPAGNA"
SEQ ID NO: 184 "ASGS"
SEQ ID NO: 185 "ASGSG"
SEQ ID NO: 186 "ASGSGQ"
SEQ ID NO: 187 coding sequence *A. oryzae* triacylglycerol lipase SP + 7 N-terminal amino acids of *T. lanuginosus* xylanase
SEQ ID NO: 188 coding sequence *A. oryzae* prolyl dipeptidyl peptidase SP + 7 N-terminal amino acids of *T. terrestris* endoglucanase

The invention described and claimed herein is not to be limited in scope by the specific aspects herein disclosed, since these aspects are intended as illustrations of several aspects of the invention. Any equivalent aspects are intended to be within the scope of this invention. Indeed, various modifications of the invention in addition to those shown and described herein will become apparent to those skilled in the art from the foregoing description. Such modifications are also intended to fall within the scope of the appended claims. In the case of conflict, the present disclosure including definitions will control.

The invention is further defined by the following numbered paragraphs:

1. A nucleic acid construct comprising or consisting of

a) a first polynucleotide encoding a signal peptide (SP),
b) a second polynucleotide located downstream of the first polynucleotide and encoding a SP-linker consisting of 2 to 15 amino acids, wherein the SP-linker amino acid sequence consists of the N-terminal amino acid sequence of a second donor polypeptide; and
c) at least one third polynucleotide located downstream of the second polynucleotide and encoding a polypeptide of interest;

wherein the first polynucleotide and the second polynucleotide are heterologous to the third polynucleotide, and wherein the first polynucleotide, the second polynucleotide, and the third polynucleotide are operably linked in translational fusion.

2. The nucleic acid construct according to paragraph 1, wherein the first polynucleotide encoding the signal peptide is derived from a polynucleotide encoding a first donor polypeptide.

3. The nucleic acid construct according to any of paragraphs 1 to 2, wherein the first donor polypeptide is a matured first donor polypeptide.

4. The nucleic acid construct according to any of paragraphs 1 to 3, wherein the SP-linker is comprised in the N-terminal sequence of the second donor polypeptide.

5. The nucleic acid construct according to paragraph 4, wherein the second donor polypeptide is a matured second donor polypeptide.

6. The nucleic acid construct according to any of the preceding paragraphs, wherein the polypeptide of interest is a matured polypeptide of interest.

7. The nucleic acid construct according to any preceding paragraph, wherein the second donor polypeptide is heterologous to the polypeptide of interest.

8. The nucleic acid construct according to any preceding paragraph, wherein the SP-linker consists of 1 to 10 amino acids, preferably consists of 2 to 9 amino acids, 2 to 8 amino acids, 3 to 8 amino acids, 3 to 9 amino acids, 2 to 7 amino acids, 3 to 7 amino acids, 2 to 6 amino acids, or 3 to 6 amino acids, 4 to 10 amino acids, 5 to 10 amino acids, 4 to 9 amino acids, 5 to 9 amino acids, 4 to 8 amino acids, or 5 to 8 amino acids. 8a. The nucleic acid construct according to any preceding paragraph, wherein the SP-linker consists of 5, 6, or 7 amino acids.

9. The nucleic acid construct according to any preceding paragraph, wherein the first polynucleotide encoding the SP is endogenous to the second polynucleotide encoding the SP-linker, and/or endogenous to the polynucleotide encoding the second donor polypeptide.

10. The nucleic acid construct according to any of paragraphs 1 to 9, wherein the amino acid sequence of the matured first donor polypeptide has at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% sequence identity to the amino acid sequence of the matured second donor polypeptide.

11. The nucleic acid construct according to any of paragraphs 1 to 10, wherein the matured first donor polypeptide and the matured second donor polypeptide have a different biological function.

12. The nucleic acid construct according to any of paragraphs 1 to 10, wherein the matured second donor polypeptide and the matured polypeptide of interest have a different biological function.

13. The nucleic acid construct according to any of paragraphs 1 to 12, wherein a signal peptidase cleavage site is located downstream of the signal peptide encoded by the first polynucleotide.

14. The nucleic acid construct according to any of paragraphs 1 to 13, wherein a signal peptidase cleavage site is located upstream of the SP-linker encoded by the second polynucleotide.

15. The nucleic acid construct according to any of paragraphs 1 to 14, wherein the signal peptide is derived from a first donor polypeptide selected from the list consisting of a structural polypeptide, a receptor, a secreted polypeptide, a hormone, or a secreted enzyme, such as a hydrolase, isomerase, ligase, lyase, lysozyme, oxidoreductase, or transferase, e.g., an aminopeptidase, amylase, carbohydrase, carboxypeptidase, catalase, cellobiohydrolase, cellulase, chitinase, cutinase, cyclodextrin glycosyltransferase, deoxyribonuclease, endoglucanase, esterase, alpha-galactosidase, beta-galactosidase, glucoamylase, alpha-glucosidase, beta-glucosidase, invertase, laccase, lipase, mannosidase, mutanase, nuclease, oxidase, pectinolytic enzyme, peroxidase, phosphodiesterase, phytase, polyphenoloxidase, proteolytic enzyme, ribonuclease, transglutaminase, xylanase, aspartate phosphatase, or beta-xylosidase.

16. The nucleic acid construct according to any of paragraphs 1 to 15, wherein the signal peptide is derived from a first donor polypeptide comprising or consisting of an aspartate phosphatase.

17. The nucleic acid construct according to any of paragraphs 1 to 16, wherein the signal peptide is derived from a bacterial cell or a eukaryotic cell.

18. The nucleic acid construct according to any of paragraphs 1 to 17, wherein the signal peptide is derived from a bacterial cell, *e.g.*, a Gram-positive cell selected from the group consisting of *Bacillus*, *Clostridium*, *Enterococcus*, *Geobacillus*, *Lactobacillus*, *Lactococcus*, *Oceanobacillus*, *Staphylococcus*, *Streptococcus*, or *Streptomyces* cells, or a Gram-negative bacteria selected from the group consisting of *Campylobacter*, *E. coli*, *Flavobacterium*, *Fusobacterium*, *Helicobacter*, *Ilyobacter*, *Neisseria*, *Pseudomonas*, *Salmonella*, and *Ureaplasma* cells, such as *Bacillus alkalophilus*, *Bacillus amyloliquefaciens*, *Bacillus brevis*, *Bacillus circulans*, *Bacillus clausii*, *Bacillus coagulans*, *Bacillus firmus*, *Bacillus lautus*, *Bacillus lentus*, *Bacillus licheniformis*, *Bacillus megaterium*, *Bacillus pumilus*, *Bacillus stearothermophilus*, *Bacillus subtilis*, *Bacillus thuringiensis*, *Streptococcus equisimilis*, *Streptococcus pyogenes*, *Streptococcus uberis*, and *Streptococcus equi* subsp. *Zooepidemicus*, *Streptomyces achromogenes*, *Streptomyces avermitilis*, *Streptomyces coelicolor*, *Streptomyces griseus*, and *Streptomyces lividans* cells.

19. The nucleic acid construct according to any of paragraphs 1 to 18, wherein the signal peptide is derived from a *Bacillus* cell, such as from a *Bacillus subtilis* cell, a *Bacillus licheniformis* cell, or a *Bacillus pumilus* cell.

20. The nucleic acid construct according to any of paragraphs 1 to 19, wherein the signal peptide is derived from a *Bacillus pumilus* cell.

21. The nucleic acid construct according to paragraph 20, wherein the signal peptide is derived from a first donor polypeptide comprising or consisting of an amino acid sequence having at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% sequence identity to the amino acid sequence of SEQ ID NO: 18 or 19.

22. The nucleic acid construct according to any of paragraphs 1 to 17, wherein the signal peptide is derived from a eukaryotic cell selected from the list of a fungal cell, a filamentous fungal cell, a yeast cell, and a mammalian cell.

23. The nucleic acid construct according to paragraph 22, wherein the signal peptide is derived from a yeast cell, *e.g.* a *Candida*, *Hansenula*, *Kluyveromyces*, *Pichia*, *Saccharomyces*, *Schizosaccharomyces*, or *Yarrowia* cell, such as a *Kluyveromyces lactis*, *Saccharomyces carlsbergensis*, *Saccharomyces cerevisiae*, *Saccharomyces diastaticus*, *Saccharomyces douglasii*, *Saccharomyces kluyveri*, *Saccharomyces norbensis*, *Saccharomyces oviformis*, or *Yarrowia lipolytica* cell,
or derived from a filamentous fungal recombinant host cell, *e.g.*, an *Acremonium*, *Aspergillus*, *Aureobasidium*, *Bjerkandera*, *Ceriporiopsis*, *Chrysosporium*, *Coprinus*, *Coriolus*, *Cryptococcus*, *Filibasidium*, *Fusarium*, *Humicola*, *Magnaporthe*, *Mucor*, *Myceliophthora*, *Neocallimastix*, *Neurospora*, *Paecilomyces*, *Penicillium*, *Phanerochaete*, *Phlebia*, *Piromyces*, *Pleurotus*, *Schizophyllum*, *Talaromyces*, *Thermoascus*, *Thielavia*, *Tolypocladium*, *Trametes*, or *Trichoderma* cell, in particular, an *Aspergillus awamori*, *Aspergillus foetidus*, *Aspergillus fumigatus*, *Aspergillus japonicus*, *Aspergillus nidulans*, *Aspergillus niger*, *Aspergillus oryzae*, *Bjerkandera adusta*, *Ceriporiopsis aneirina*, *Ceriporiopsis caregiea*, *Ceriporiopsis gilvescens*, *Ceriporiopsis pannocinta*, *Ceriporiopsis rivulosa*, *Ceriporiopsis subrufa*, *Ceriporiopsis subvermispora*, *Chrysosporium inops*, *Chrysosporium keratinophilum*, *Chrysosporium lucknowense*, *Chrysosporium merdarium*, *Chrysosporium pannicola*, *Chrysosporium queenslandicum*, *Chrysosporium tropicum*, *Chrysosporium zonatum*, *Coprinus cinereus*, *Coriolus hirsutus*, *Fusarium bactridioides*, *Fusarium cerealis*, *Fusarium crookwellense*, *Fusarium culmorum*, *Fusarium graminearum*, *Fusarium graminum*, *Fusarium heterosporum*, *Fusarium negundi*, *Fusarium oxysporum*, *Fusarium reticulatum*, *Fusarium roseum*, *Fusarium sambucinum*, *Fusarium sarcochroum*, *Fusarium sporotrichioides*, *Fusarium sulphureum*, *Fusarium torulosum*, *Fusarium trichothecioides*, *Fusarium venenatum*, *Humicola insolens*, *Humicola lanuginosa*, *Mucor miehei*, *Myceliophthora thermophila*, *Neurospora crassa*, *Penicillium purpurogenum*, *Phanerochaete chrysosporium*, *Phlebia radiata*, *Pleurotus eryngii*, *Talaromyces emersonii*, *Thielavia terrestris*, *Trametes villosa*, *Trametes versicolor*, *Trichoderma harzianum*, *Trichoderma koningii*, *Trichoderma longibrachiatum*, *Trichoderma reesei*, or *Trichoderma viride* cell.

24. The nucleic acid construct according to paragraph 23, wherein the signal peptide is derived from a *Pichia pastoris*, a *Trichoderma reesei*, a *Aspergillus niger*, or a *Aspergillus oryzae* cell.

24a. The nucleic acid construct according to any one of paragrapsh 1 to 24, wherein the signal peptide is derived from a lipase, preferably an *Aspergillus oryzae* lipase.

24b. The nucleic acid construct according to any one of paragraphs 1 to 24, wherein the signal peptide is derived from a peptidase, preferably an *Aspergillus oryzae* peptidase.

25. The nucleic acid construct according to any of paragraphs 1 to 24, wherein the second donor polypeptide is selected from the list consisting of a structural polypeptide, a receptor, a secreted polypeptide, a hormone, or a secreted enzyme, such as a hydrolase, isomerase, ligase, lyase, lysozyme, oxidoreductase, or transferase, e.g., an aminopeptidase, amylase, carbohydrase, carboxypeptidase, catalase, cellobiohydrolase, cellulase, chitinase, cutinase, cyclodextrin glycosyltransferase, deoxyribonuclease, endoglucanase, esterase, alpha-galactosidase, beta-galactosidase, glucoamylase, alpha-glucosidase, beta-glucosidase, invertase, laccase, lipase, mannosidase, mutanase, nuclease, oxidase, pectinolytic enzyme, peroxidase, phosphodiesterase, phytase, polyphenoloxidase, proteolytic enzyme, ribonuclease, transglutaminase, xylanase, aspartate phosphatase, or beta-xylosidase.

26. The nucleic acid construct according to any of paragraphs 1 to 25, wherein the second donor polypeptide comprises or consists of a protease.

26a. The nucleic acid construct according to any one of paragraphs 1 to 25, wherein the second donor polypeptide comprises or consists of a xylanase.

26b. The nucleic acid construct according to any one of paragraphs 1 to 25, wherein the second donor polypeptide comprises or consists of an endoglucanase.

27. The nucleic acid construct according to any of paragraphs 1 to 26, wherein the second donor polypeptide is endogenous to a bacterial cell or a eukaryotic cell.

28. The nucleic acid construct according to any of paragraphs 1 to 27, wherein the second donor polypeptide is endogenous to a bacterial cell, *e.g.*, a Gram-positive cell selected from the group consisting of *Bacillus*, *Clostridium*, *Enterococcus*, *Geobacillus*, *Lactobacillus*, *Lactococcus*, *Oceanobacillus*, *Staphylococcus*, *Streptococcus*, or *Streptomyces* cells, or a Gram-negative bacteria selected from the group consisting of *Campylobacter*, *E. coli*, *Flavobacterium*, *Fusobacterium*, *Helicobacter*, *Ilyobacter*, *Neisseria*, *Pseudomonas*, *Salmonella*, and *Ureaplasma* cells, such as *Bacillus alkalophilus*, *Bacillus amyloliquefaciens*, *Bacillus brevis*, *Bacillus circulans*, *Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis, Bacillus thuringiensis, Streptococcus equisimilis, Streptococcus pyogenes, Streptococcus uberis*, and *Streptococcus equi* subsp. *Zooepidemicus*, *Streptomyces achromogenes*, *Streptomyces avermitilis*, *Streptomyces coelicolor*, *Streptomyces griseus*, and *Streptomyces lividans* cells.

29. The nucleic acid construct according to any of paragraphs 1 to 28, wherein the second donor polypeptide is endogenous to a *Bacillus* cell, such as a *Bacillus subtilis* cell, a *Bacillus licheniformis* cell, or a *Bacillus pumilus* cell.

30. The nucleic acid construct according to any of paragraphs 1 to 28, wherein the second donor polypeptide is endogenous to a *Bacillus* cell or a *Nocardiopsis* cell, preferably a *Bacillus clausii* cell, or a *Nocardiopsis prasina* cell.

30a. The nucleic acid construct according to any of paragrapsh 1 to 28, wherein the second donor polypeptide is endogenous to *Thermomyces lanuginosus.*

30b. The nucleic acid construct according to any of paragrapsh 1 to 28, wherein the second donor polypeptide is endogenous to *Thermothielavioides terrestris.*

31. The nucleic acid construct according to any of paragraphs 1 to 30, wherein the second donor polypeptide is comprising or consisting of an amino acid sequence having at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% sequence identity to the amino acid sequence of SEQ ID NO: 16 or 17.

32. The nucleic acid construct according to any of paragraphs 1 to 27, wherein the second donor polypeptide is endogenous to a eukaryotic cell selected from the list of a fungal cell, a filamentous fungal cell, a yeast cell, and a mammalian cell.

33. The nucleic acid construct according to paragraph 32, wherein the second donor polypeptide is endogenous to a yeast cell, *e.g.* a *Candida, Hansenula, Kluyveromyces, Pichia, Saccharomyces, Schizosaccharomyces,* or *Yarrowia* cell, such as a *Kluyveromyces lactis, Saccharomyces carlsbergensis, Saccharomyces cerevisiae, Saccharomyces diastaticus, Saccharomyces douglasii, Saccharomyces kluyveri, Saccharomyces norbensis, Saccharomyces oviformis,* or *Yarrowia lipolytica* cell,

or endogenous to a filamentous fungal recombinant host cell, *e.g.,* an *Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes,* or *Trichoderma* cell, in particular, an *Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Talaromyces emersonii, Thielavia terrestris, Trametes villosa, Trametes versicolor, Trichoderma harzianum, Trichoderma koningii, Trichoderma longibrachiatum, Trichoderma reesei,* or *Trichoderma viride* cell.

34. The nucleic acid construct according to paragraph 33, wherein the second donor polypeptide is endogenous to a *Pichia pastoris,* a *Trichoderma reesei,* a *Aspergillus niger,* or a *Aspergillus oryzae* cell.

35. The nucleic acid construct according to any of paragraphs 1 to 34, wherein the first polynucleotide and/or the second polynucleotide is a synthetic polynucleotide.

36. The nucleic acid construct according to any of the preceding paragraphs, wherein the nucleic acid construct further comprises a heterologous promoter operably linked to the first polynucleotide.

37. The nucleic acid construct according to paragraph 36, wherein the heterologous promoter comprises or consists of a nucleotide sequence having at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% sequence identity to the polynucleotide sequence of SEQ ID NO: 20.

38. The nucleic acid construct according to any of the preceding paragraphs, wherein the second donor polypeptide has protease activity.

38a. The nucleic acid construct according to any of the preceding paragraphs, wherein the second donor polypeptide has xylanase activity.

38b. The nucleic acid construct according to any of the preceding paragraphs, wherein the second donor polypeptide has endoglucanase activity.

39. The nucleic acid construct according to any of the preceding paragraphs, wherein the second donor polypeptide comprises a pro-peptide at the N-terminal end of the amino acid sequence.

40. The nucleic acid construct according to paragraph 39, wherein the SP-linker is derived from the pro-peptide of the second donor polypeptide.

41. The nucleic acid construct according to any of the preceding paragraphs, wherein the first polynucleotide sequence comprises or consists of a nucleic acid sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the nucleic acid sequence of SEQ ID NO: 1.

42. The nucleic acid construct according to any of the preceding paragraphs, wherein the first polynucleotide sequence comprises or consists of a nucleic acid sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the nucleic acid sequence of SEQ ID NO: 2.

42a. The nucleic acid construct according to any one of paragraphs 1 to 41, wherein the first polynucleotide sequence comprises or consists of a nucleic acid sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the nucleic acid sequence of SEQ ID NO: 177.

42b. The nucleic acid construct according to any one of paragrapsh 1 to 41, wherein the first polynucleotide sequence comprises or consists of a nucleic acid sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the nucleic acid sequence of SEQ ID NO: 178.

43. The nucleic acid construct according to any of paragraphs 1 to 41, wherein the first polynucleotide sequence encodes a signal peptide comprising or consisting of an amino acid sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the amino acid sequence of SEQ ID NO: 3 "MKFKTLSVVFVMISVISVVSYFTSEVTA".

44. The nucleic acid construct according to any of paragraphs 1 to 40 or paragraph 42, wherein the first polynucleotide sequence encodes a signal peptide comprising or consisting of an amino acid sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the amino acid sequence of SEQ ID NO: 4 "MMKKVSGLIILSMMLLSGFYMETAYGA".

44a. The nucleic acid construct according to any of paragraphs 1 to 44, wherein the first polynucleotide sequence encodes a signal peptide comprising or consisting of an amino acid sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the amino acid sequence of SEQ ID NO: 171 "MHLAIKSLFVSLLGASVLASPLPSNALVERA".

44b. The nucleic acid construct according to any of paragraphs 1 to 44, wherein the first polynucleotide sequence encodes a signal peptide comprising or consisting of an amino acid sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the amino acid sequence of SEQ ID NO: 172 "MKYSKLLLLLVSVVQA".

45. The nucleic acid construct according to any of paragraphs 1 to 44, wherein the second polynucleotide comprises or consists of a polynucleotide sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the polynucleotide sequence of SEQ ID NO: 5.

46. The nucleic acid construct according to any of paragraphs 1 to 44, wherein the second polynucleotide comprises or consists of a polynucleotide sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the polynucleotide sequence of SEQ ID NO: 6.

46a. The nucleic acid construct according to any of paragraphs 1 to 45, wherein the second polynucleotide comprises or consists of a polynucleotide sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the polynucleotide sequence of SEQ ID NO: 175.

46b. The nucleic acid construct according to any of paragraphs 1 to 45, wherein the second polynucleotide comprises or consists of a polynucleotide sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the polynucleotide sequence of SEQ ID NO: 176.

47. The nucleic acid construct according to any of paragraphs 1 to 45, wherein the second polynucleotide sequence encodes a SP-linker comprising or consisting of an amino acid sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the amino acid sequence of SEQ ID NO: 7 "TGALPQS".

48. The nucleic acid construct according to any of paragraphs 1 to 44 or paragraph 46, wherein the second polynucleotide sequence encodes a SP-linker comprising or consisting of an amino acid sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the amino acid sequence of SEQ ID NO: 8 "AEEAKEK".

48a. The nucleic acid construct according to any preceding paragraphs, wherein the second polynucleotide sequence encodes a SP-linker comprising or consisting of an amino acid sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the amino acid sequence of SEQ ID NO: 179 "FPAGNAT".

48b. The nucleic acid construct according to any preceding paragraphs, wherein the second polynucleotide sequence encodes a SP-linker comprising or consisting of an amino acid sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the amino acid sequence of SEQ ID NO: 180 "ASGSGQS".

49. The nucleic acid construct according to any preceding paragraphs, wherein the first and the second polynucleotides encode a SP with a SP-linker comprising or consisting of an amino acid sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the amino acid sequence of SEQ ID NO: 12 "MMKKVSGLIILSMMLLSGFYMETAYGAAEEAKEK".

49a. The nucleic acid construct according to any preceding paragraphs, wherein the first and the second polynucleotides encode a SP with a SP-linker comprising or consisting of an amino acid sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the amino acid sequence of SEQ ID NO: 173 "MHLAIKSLFVSLLGASVLASPLPSNALVERA**FPAGNAT**", SP-linker sequence in bold letters.

49b. The nucleic acid construct according to any preceding paragraphs, wherein the first and the second polynucleotides encode a SP with a SP-linker comprising or consisting of an amino acid sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the amino acid sequence of SEQ ID NO: 174 "MKYSKLLLLLVSVVQA**ASGSGQS**", SP-linker sequence in bold letters.

50. The nucleic acid construct according to any preceding paragraphs, wherein the first and the second polynucleotides encode a SP with SP-linker comprising or consisting of an amino acid sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the amino acid sequence of SEQ ID NO: 11 "MKFKTLSVVFVMISVISVVSYFTSEVTATGALPQS".

50 a. The nucleic acid construct according to any preceding paragraphs, wherein the first and the second polynucleotides encode a SP - SP-linker construct comprising or consisting of an amino acid sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the amino acid sequence of SEQ ID NO: 11, SEQ ID NO: 12, SEQ ID NO: 153- 167, SEQ ID NO: 170, SEQ ID NO: 173, or SEQ ID NO: 174.

51. The nucleic acid construct according to any preceding paragraphs, wherein the second polynucleotide encodes a SP-linker comprising or consisting of an amino acid sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the amino acid sequence of "T", "TG", "TGA",

SEQ ID NO: 24 "TGAL",
SEQ ID NO: 25 "TGALP",
SEQ ID NO: 26 "TGALPQ",
SEQ ID NO: 27 "TGALPQS",
SEQ ID NO: 28 "TGALPQSP",
SEQ ID NO: 29 "TGALPQSPT",
SEQ ID NO: 30 "TGALPQSPTP",
SEQ ID NO: 31 "TGALPQSPTPE",
SEQ ID NO: 32 "TGALPQSPTPEA",
SEQ ID NO: 33 "TGALPQSPTPEAD",
SEQ ID NO: 34 "TGALPQSPTPEADA",
SEQ ID NO: 35 "TGALPQSPTPEADAV",
SEQ ID NO: 36 "TGALPQSPTPEADAVS",
SEQ ID NO: 37 "TGALPQSPTPEADAVSM",
SEQ ID NO: 38 "TGALPQSPTPEADAVSMQ",
SEQ ID NO: 39 "TGALPQSPTPEADAVSMQE",
SEQ ID NO: 40 "TGALPQSPTPEADAVSMQEA",
SEQ ID NO: 41 "TGALPQSPTPEADAVSMQEAL",
SEQ ID NO: 42 "TGALPQSPTPEADAVSMQEALQ",
SEQ ID NO: 43 "TGALPQSPTPEADAVSMQEALQR",
SEQ ID NO: 44 "TGALPQSPTPEADAVSMQEALQRD",
SEQ ID NO: 45 "TGALPQSPTPEADAVSMQEALQRDL",
SEQ ID NO: 46 "TGALPQSPTPEADAVSMQEALQRDLD",
SEQ ID NO: 47 "TGALPQSPTPEADAVSMQEALQRDLDL",
SEQ ID NO: 48 "TGALPQSPTPEADAVSMQEALQRDLDLT",
,"A", "AE", "AEE",
SEQ ID NO: 52 "AEEA",
SEQ ID NO: 53 "AEEAK",
SEQ ID NO: 54 "AEEAKE",
SEQ ID NO: 55 "AEEAKEK",
SEQ ID NO: 56 "AEEAKEKY",
SEQ ID NO: 57 "AEEAKEKYL",
SEQ ID NO: 58 "AEEAKEKYLI",
SEQ ID NO: 59 "AEEAKEKYLIG",
SEQ ID NO: 60 "AEEAKEKYLIGF",
SEQ ID NO: 61 "AEEAKEKYLIGFN",
SEQ ID NO: 62 "AEEAKEKYLIGFNE",
SEQ ID NO: 63 "AEEAKEKYLIGFNEQ",
SEQ ID NO: 64 "AEEAKEKYLIGFNEQE",
SEQ ID NO: 65 "AEEAKEKYLIGFNEQEA",
SEQ ID NO: 66 "AEEAKEKYLIGFNEQEAV",
SEQ ID NO: 67 "AEEAKEKYLIGFNEQEAVS",
SEQ ID NO: 68 "AEEAKEKYLIGFNEQEAVSE",
SEQ ID NO: 69 "AEEAKEKYLIGFNEQEAVSEF",
SEQ ID NO: 70 "AEEAKEKYLIGFNEQEAVSEFV",
SEQ ID NO: 71 "AEEAKEKYLIGFNEQEAVSEFVE",
SEQ ID NO: 72 "AEEAKEKYLIGFNEQEAVSEFVEQ",
SEQ ID NO: 73 "AEEAKEKYLIGFNEQEAVSEFVEQV",

SEQ ID NO: 74 "AEEAKEKYLIGFNEQEAVSEFVEQVE",
SEQ ID NO: 75 "AEEAKEKYLIGFNEQEAVSEFVEQVEA",
SEQ ID NO: 76 "AEEAKEKYLIGFNEQEAVSEFVEQVEAN",
"S", "SH", "SHD",
SEQ ID NO: 83 "SHDE",
SEQ ID NO: 84 "SHDEA",
SEQ ID NO: 85 "SHDEAR",
SEQ ID NO: 86 "SHDEARR",
SEQ ID NO: 87 "SHDEARRG",
SEQ ID NO: 88 "SHDEARRGH",
SEQ ID NO: 89 "SHDEARRGHT",
SEQ ID NO: 90 "SHDEARRGHTA",
SEQ ID NO: 91 "SHDEARRGHTAS",
SEQ ID NO: 92 "SHDEARRGHTASI",
SEQ ID NO: 93 "SHDEARRGHTASIG",
SEQ ID NO: 94 "SHDEARRGHTASIGY",
SEQ ID NO: 95 "SHDEARRGHTASIGYT",
SEQ ID NO: 96 "SHDEARRGHTASIGYTA",
"T", "TP", "TPQ",
SEQ ID NO: 97 "TPQMEKR"
SEQ ID NO: 101 "TPQM",
SEQ ID NO: 102 "TPQME",
SEQ ID NO: 103 "TPQMEK",
SEQ ID NO: 104 "TPQMEKR",
SEQ ID NO: 105 "TPQMEKRP",
SEQ ID NO: 106 "TPQMEKRPV",
SEQ ID NO: 107 "TPQMEKRPVM",
SEQ ID NO: 108 "TPQMEKRPVMH",
SEQ ID NO: 109 "TPQMEKRPVMHQ",
SEQ ID NO: 110 "TPQMEKRPVMHQV",
SEQ ID NO: 111 "TPQMEKRPVMHQVK",
SEQ ID NO: 112 "TPQMEKRPVMHQVKL",
SEQ ID NO: 113 "TPQMEKRPVMHQVKLT",
SEQ ID NO: 114 "TPQMEKRPVMHQVKLTT",
SEQ ID NO: 115 "TPQMEKRPVMHQVKLTTE",
SEQ ID NO: 116 "TPQMEKRPVMHQVKLTTEQ",
SEQ ID NO: 117 "TPQMEKRPVMHQVKLTTEQQ",
SEQ ID NO: 118 "TPQMEKRPVMHQVKLTTEQQK",
SEQ ID NO: 119 "TPQMEKRPVMHQVKLTTEQQKQ",
SEQ ID NO: 120 "TPQMEKRPVMHQVKLTTEQQKQI",
SEQ ID NO: 121 "TPQMEKRPVMHQVKLTTEQQKQIE",
SEQ ID NO: 122 "TPQMEKRPVMHQVKLTTEQQKQIEM",
SEQ ID NO: 123 "TPQMEKRPVMHQVKLTTEQQKQIEML",
SEQ ID NO: 124 "TPQMEKRPVMHQVKLTTEQQKQIEMLE",
SEQ ID NO: 125 "TPQMEKRPVMHQVKLTTEQQKQIEMLEQ",
SEQ ID NO: 126 "TPQMEKRPVMHQVKLTTEQQKQIEMLEQQ",
"FP", "FPA",
SEQ ID NO: 181 "FPAG",
SEQ ID NO: 182 "FPAGN",
SEQ ID NO: 183 "FPAGNA",
SEQ ID NO: 179 "FPAGNAT",
"AS", "ASG",
SEQ ID NO: 184 "ASGS",
SEQ ID NO: 185 "ASGSG",
SEQ ID NO: 186 "ASGSGQ", or
SEQ ID NO: 180 "ASGSGQS".

52. The nucleic acid construct according to any of paragraphs 1 to 51, wherein the signal peptidase cleavage site is recognized by a signal peptidase selected from the list of a SPase I, SPase II, SPase III, and SPase IV.

53. The nucleic acid construct according to any of paragraphs 1 to 52, wherein the signal peptidase cleavage site is recognized by a signal peptidase derived from *Bacillus licheniformis,* preferably by a signal peptidase comprising or consisting of an amino acid sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the amino acid sequence of SEQ ID NO: 21, 22, 23, 49, 50, 51, or 98.

54. The nucleic acid construct according to any of paragraphs 1 to 53, wherein the second polynucleotide encodes a SP-linker consisting of two amino acids, three amino acids, four amino acids, 5 amino acids, 6 amino acids, 7 amino acids, 8 amino acids, 9 amino acids, 10 amino acids, 11 amino acids, 12 amino acids, 13 amino acids, 14 amino acids, or 15 amino acids.

55. The nucleic acid construct according to any of paragraphs 1 to 54, wherein the second polynucleotide encodes a SP-linker consisting of 2 - 10 amino acids, or consisting of 2 to 9 amino acids, 2 to 8 amino acids, 3 to 8 amino acids, 3 to 9 amino acids, 2 to 7 amino acids, 3 to 7 amino acids, 2 to 6 amino acids, or 3 to 6 amino acids, 4 to 10 amino acids, 5 to 10 amino acids, 4 to 9 amino acids, 5 to 9 amino acids, 4 to 8 amino acids, or 5 to 8 amino acids.

56. The nucleic acid construct according to any of the preceding paragraphs, wherein the polypeptide of interest is selected from the list of an enzyme, a therapeutic polypeptide, and a food or feed polypeptide, such as an antibody, an antigen, an antimicrobial peptide, a growth factor, a hormone, an immunodilator, a neurotransmitter, a receptor, a reporter protein, a structural protein, a transcription factor, a heme-containing polypeptide, a brazzein, a casein, a patatin, an ovalbumin, an osteopontin, an ovotransferrin, an ovomucin, an ovomucoid, an ovostatin, a lactoferrin, an alpha-lactalbumin, a beta-lactalbumin, a glycomacropeptide, or a collagen.

57. The nucleic acid construct according to any of paragraphs 1 to 55, wherein the polypeptide of interest is selected from a hydrolase, isomerase, ligase, lyase, lysozyme, oxidoreductase, or transferase, e.g., an aminopeptidase, amylase, carbohydrase, carboxypeptidase, catalase, cellobiohydrolase, cellulase, chitinase, cutinase, cyclodextrin glycosyltransferase, deoxyribonuclease, endoglucanase, esterase, alpha-galactosidase, beta-galactosidase, glucoamylase, alpha-glucosidase, beta-glucosidase, invertase, laccase, lipase, mannosidase, mutanase, nuclease, oxidase, pectinolytic enzyme, peroxidase, phosphodiesterase, phytase, polyphenoloxidase, proteolytic enzyme, ribonuclease, transglutaminase, xylanase, aspartate phosphatase, or beta-xylosidase.

58. The nucleic acid construct according to any of the preceding paragraphs, wherein the third polynucleotide sequence comprises or consists of a nucleotide sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the nucleotide sequence of SEQ ID NO: 13.

59. The nucleic acid construct according to any of paragraphs 1 to 58, wherein the polypeptide of interest comprises a pro-peptide.

60. The nucleic acid construct according to any of paragraphs 1 to 59, wherein the pro-peptide is located at the N-terminal end of the amino acid sequence of the protein of interest.

61. The nucleic acid construct according to any of paragraphs 1 to 60, wherein the pro-peptide is located downstream of the SP-linker.

62. The nucleic acid construct according to any of paragraphs 1 to 61, wherein the SP-linker and the pro-peptide are cleaved off the amino acid sequence of the matured protein of interest.

63. The nucleic acid construct according to any of paragraphs 1 to 62, wherein the protein of interest is not secreted when expressed in its native form.

64. The nucleic acid construct according to paragraph 63, wherein the protein of interest is selected from the list of an epimerase, a heme-containing polypeptide, a mannanase, an endoglucanase, an asparaginase, a xylanase, a glucoisomerase, a xyloseisomerase, and a protease.

65. The nucleic acid construct according to any of paragraphs 1 to 64, wherein the nucleic acid construct encodes an additional cleavage site located between the SP-linker and the polypeptide of interest, or between the SP-linker and the pro-peptide of the polypeptide of interest.

66. The nucleic acid construct according to paragraph 65, wherein the additional cleavage site is a proteolytic cleavage site.

67. The nucleic acid construct according to any of paragraphs 65 to 66, wherein the additional cleavage site is recognized by a suitable protease, preferably a KexB protease.

68. The nucleic acid construct according to paragraph 67, wherein the additional cleavage site is a dibasic amino acid motif such as a Lys-Arg ("KR") or an Arg-Arg motif ("RR"), preferably the additional cleavage site is a KexB or Kex2 cleavage site.

69. The nucleic acid construct according to any preceding paragraph, which is isolated.

70. The nucleic acid construct according to any preceding paragraph, which is purified.

70 a. The nucleic acid construct according to any preceding paragraph, wherein the polypeptide of interest does not comprise a chymosin.

71. An expression vector comprising a nucleic acid construct according to any of paragraphs 1 to 70.

72. A host cell producing a polypeptide of interest and comprising in its genome:

   a) a nucleic acid construct according to any of paragraphs 1 to 70; and/or
   b) an expression vector according to paragraph 71.

73. The host cell according to paragraph 72, wherein the host cell is a bacterial host cell, preferably a *Bacillus* host cell, a fungal host cell, or a eukaryotic host cell, a *Trichoderma* host cell, an *Aspergillus* host cell, a *Pichia* host cell, a *Saccharomyces* host cell, or a mammalian host cell.

74. The host cell of any one of paragraphs 72 to 73, which is a yeast recombinant host cell, *e.g.,* a *Candida*, *Hansenula*, *Kluyveromyces*, *Pichia*, *Saccharomyces*, *Schizosaccharomyces*, or *Yarrowia* cell, such as a *Kluyveromyces lactis*, *Saccharomyces carlsbergensis*, *Saccharomyces cerevisiae*, *Saccharomyces diastaticus*, *Saccharomyces douglasii*, *Saccharomyces kluyveri*, *Saccharomyces norbensis*, *Saccharomyces oviformis*, or *Yarrowia lipolytica* cell.

75. The host cell of any one of paragraphs 72 to 73, which is a filamentous fungal recombinant host cell, *e.g.,* an *Acremonium*, *Aspergillus*, *Aureobasidium*, *Bjerkandera*, *Ceriporiopsis*, *Chrysosporium*, *Coprinus*, *Coriolus*, *Cryptococcus*, *Filibasidium*, *Fusarium*, *Humicola*, *Magnaporthe*, *Mucor*, *Myceliophthora*, *Neocallimastix*, *Neurospora*, *Paecilomyces*, *Penicillium*, *Phanerochaete*, *Phlebia*, *Piromyces*, *Pleurotus*, *Schizophyllum*, *Talaromyces*, *Thermoascus*, *Thielavia*, *Tolypocladium*, *Trametes,* or *Trichoderma* cell, in particular, an *Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Talaromyces emersonii, Thielavia terrestris, Trametes villosa, Trametes versicolor, Trichoderma harzianum, Trichoderma koningii, Trichoderma longibrachiatum, Trichoderma reesei,* or *Trichoderma viride* cell.

75a. The host cell of any preceding paragraphs, wherein the host cell is an *Aspergillus oryzae* cell.

76. The host cell of any one of paragraphs 72 to 73, which is a prokaryotic recombinant host cell, *e.g.,* a Gram-positive cell selected from the group consisting of *Bacillus, Clostridium, Enterococcus, Geobacillus, Lactobacillus, Lactococcus, Oceanobacillus, Staphylococcus, Streptococcus,* or *Streptomyces* cells, or a Gram-negative bacteria selected from the group consisting of *Campylobacter, E. coli, Flavobacterium, Fusobacterium, Helicobacter, Ilyobacter, Neisseria, Pseudomonas, Salmonella,* and *Ureaplasma* cells, such as *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis, Bacillus thuringiensis, Streptococcus equisimilis, Streptococcus pyogenes, Streptococcus uberis,* and *Streptococcus equi* subsp. *Zooepidemicus, Streptomyces achromogenes, Streptomyces avermitilis, Streptomyces coelicolor, Streptomyces griseus,* and *Streptomyces lividans* cells.

77. The host cell of any one of paragraphs 72 to 73, which is a *Bacillus* cell, preferably a *Bacillus subtilis* cell, more preferably a *Bacillus licheniformis* cell.

78. The host cell of any one of paragraphs 72 to 73, wherein the host cell is a yeast cell, *e.g.* a *Candida, Hansenula, Kluyveromyces, Pichia, Saccharomyces, Schizosaccharomyces,* or *Yarrowia* cell, such as a *Kluyveromyces lactis, Saccharomyces carlsbergensis, Saccharomyces cerevisiae, Saccharomyces diastaticus, Saccharomyces douglasii, Saccharomyces kluyveri, Saccharomyces norbensis, Saccharomyces oviformis,* or *Yarrowia lipolytica* cell, or a filamentous fungal recombinant host cell, *e.g.,* an *Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes,* or *Trichoderma* cell, in particular, an *Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Talaromyces emersonii, Thielavia terrestris, Trametes villosa, Trametes versicolor, Trichoderma harzianum, Trichoderma koningii, Trichoderma longibrachiatum, Trichoderma reesei,* or *Trichoderma viride* cell.

79. The host cell of any one of paragraphs 72 to 73, wherein the host cell is a mammalian cell selected from the list of a Chinese Hamster Ovary cell (CHO cell), human embryo kidney cell (HEK cell), mouse cell, mouse myeloma cell (NS0), baby hamster kidney cell (BHK cell), or human retinal cells.

80. The host cell of any one of paragraphs 72 to 79, which is isolated.

81. The host cell of any one of paragraphs 72 to 80, which is purified.

82. The host cell according to paragraphs 72 to 81, wherein the first donor polypeptide is endogenous to the host cell.

83. The host cell according to paragraphs 72 to 82, wherein the second donor polypeptide is endogenous to the host cell.

84. The host cell of any one of paragraphs 72 to 83, wherein the signal peptide is endogenous to the host cell.

85. The host cell of any one of paragraphs 72 to 84, wherein the SP-linker is endogenous to the host cell.

86. The host cell according to any of paragraphs 72 to 85, wherein the polypeptide of interest is heterologous to the host cell.

87. The host cell of any one of paragraphs 72 to 86, which comprises at least two copies, e.g., three, four, five, or more copies of the nucleic acid construct of any one of paragraphs 1-70.

88. The host cell of any of paragraphs 72 to 87, wherein a yield or secretion of the polypeptide of interest is increased by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 100% relative to a yield or secretion of the polypeptide of interest expressed by a parent host cell lacking the second polynucleotide encoding the SP-linker when cultivated under identical conditions, the parent host cell otherwise being isogenic to the host cell according to any of paragraphs 72 to 87.

89. A method of producing a polypeptide of interest, the method comprising:

a) cultivating a host cell according to any of paragraphs 72 to 88 under conditions conducive for production of the polypeptide of interest; and optionally
b) recovering the polypeptide of interest.

90. The method according to paragraph 89, the method further comprising step c) maturing the polypeptide of interest by contacting the polypeptide of interest with a protease or peptidase configured to cleave off the pro-peptide and/or SP-linker from the mature polypeptide of interest, wherein step c) is carried out prior, after, or in parallel to step b).

91. The method according to any of paragraphs 89 to 90, wherein the cultivation is a batch cultivation, a fed-batch cultivation, perfusion cultivation, or a continuous cultivation.

92. The method according to any of paragraphs 89 to 91, wherein the cultivation has a duration of at least 24 hours, at least 48 hours, at least 72 hours, at least 96 hours, at least 120 hours, at least 144 hours, at least 168 hours, at least 192 hours, at least 216 hours, at least 240 hours, at least 264 hours, at least 288 hours, at least 312 hours, at least 336 hours, or at least 360 hours.

93. The method according to any of paragraphs 89 to 92, wherein a yield of the polypeptide of interest is increased by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 100% relative to a yield of the polypeptide of interest expressed by a parent host cell lacking the second polynucleotide encoding the SP-linker when cultivated under identical conditions, the parent host cell otherwise being isogenic to the host cell according to any of paragraphs 72 to 88.

94. A transgenic plant, plant part or plant cell transformed with the nucleic acid construct according to any of paragraphs 1 to 70, or the expression construct according to paragraph 71.

95. A method of producing a polypeptide of interest, comprising cultivating the transgenic plant or plant cell of paragraph 94 under conditions conducive for production of the polypeptide.

96. The method of paragraph 95, further comprising recovering the polypeptide.

97. A fusion polypeptide, comprising a polypeptide of interest and a SP-linker consisting of 2 to 15 amino acids, wherein the SP-linker is located at the N-terminal end of the polypeptide of interest and is heterologous to the polypeptide of interest.

98. The fusion polypeptide according to paragraph 97, wherein the fusion polypeptide is secreted.

99. The fusion polypeptide according to any of paragraphs 97 to 98, wherein the SP-linker consists of 2 to 9 amino acids, 2 to 8 amino acids, 3 to 8 amino acids, 3 to 9 amino acids, 2 to 7 amino acids, 3 to 7 amino acids, 2 to 6 amino acids, or 3 to 6 amino acids, 4 to 10 amino acids, 5 to 10 amino acids, 4 to 9 amino acids, 5 to 9 amino acids, 4 to 8 amino acids, or 5 to 8 amino acids.

100. The fusion polypeptide according to any of paragraphs 97 to 99, wherein the SP-linker consists of 2 to 15 N-terminal amino acids from a second donor polypeptide.

101. The fusion polypeptide according to any of paragraphs 97 to 100, wherein the second donor polypeptide is heterologous to the polypeptide of interest.

102. The fusion polypeptide according to any of paragraphs 97 to 101, wherein the second donor polypeptide is selected from the list consisting of a structural polypeptide, a receptor, a secreted polypeptide, a hormone, or a secreted enzyme, such as a hydrolase, isomerase, ligase, lyase, lysozyme, oxidoreductase, or transferase, e.g., an aminopeptidase, amylase, carbohydrase, carboxypeptidase, catalase, cellobiohydrolase, cellulase, chitinase, cutinase, cyclodextrin glycosyltransferase, deoxyribonuclease, endoglucanase, esterase, alpha-galactosidase, beta-galactosidase, glucoamylase, alpha-glucosidase, beta-glucosidase, invertase, laccase, lipase, mannosidase, mutanase, nuclease, oxidase, pectinolytic enzyme, peroxidase, phosphodiesterase, phytase, polyphenoloxidase, proteolytic enzyme, ribonuclease, transglutaminase, xylanase, aspartate phosphatase, or beta-xylosidase.

103. The fusion polypeptide according to any of paragraphs 97 to 102, wherein the second donor polypeptide has protease activity.

103a. The fusion polypeptide according to any of paragraphs 97 to 102, wherein the second donor polypeptide has xylanase activity.

103b. The fusion polypeptide according to any of paragraphs 97 to 102, wherein the second donor polypeptide has endoglucanase activity.

104. The fusion polypeptide according to any of paragraphs 97 to 103, wherein the second donor polypeptide is derived from or endogenous to a bacterial cell or a eukaryotic cell.

105. The fusion polypeptide according to any of paragraphs 97 to 104, wherein the second donor polypeptide is derived from or endogenous to a bacterial cell, *e.g.,* a Gram-positive cell selected from the group consisting of *Bacillus, Clostridium, Enterococcus, Geobacillus, Lactobacillus, Lactococcus, Oceanobacillus, Staphylococcus, Streptococcus,* or *Streptomyces* cells, or a Gram-negative bacteria selected from the group consisting of *Campylobacter, E. coli, Flavobacterium, Fusobacterium, Helicobacter, Ilyobacter, Neisseria, Pseudomonas, Salmonella,* and *Ureaplasma* cells, such as *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis, Bacillus thuringiensis, Streptococcus equisimilis, Streptococcus pyogenes, Streptococcus uberis,* and *Streptococcus equi* subsp. *Zooepidemicus, Streptomyces achromogenes, Streptomyces avermitilis, Streptomyces coelicolor, Streptomyces griseus,* and *Streptomyces lividans* cells.

106. The fusion polypeptide according to any of paragraphs 97 to 105, wherein the second donor polypeptide is derived from or endogenous to a *Bacillus* cell, such as from a *Bacillus subtilis* cell, a *Bacillus licheniformis* cell, or a *Bacillus pumilus* cell.

107. The fusion polypeptide according to any of paragraphs 97 to 106, wherein the second donor polypeptide is derived from or endogenous to a *Bacillus* cell or a *Nocardiopsis* cell, preferably from a *Bacillus clausii* cell, or a *Nocardiopsis prasina* cell.

108. The fusion polypeptide according to any of paragraphs 97 to 107, wherein the second donor polypeptide is comprising or consisting of an amino acid sequence having at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% sequence identity to the amino acid sequence of SEQ ID NO: 16 or 17.

109. The fusion polypeptide according to any of paragraphs 97 to 104, wherein the second donor polypeptide is derived from or endogenous to a eukaryotic cell selected from the list of a fungal cell, a filamentous fungal cell, a yeast cell, and a mammalian cell.

110. The fusion polypeptide according to any of paragraphs 97 to 104, wherein the second donor polypeptide is derived from or endogenous to a yeast cell, *e.g.* a *Candida, Hansenula, Kluyveromyces, Pichia, Saccharomyces, Schizosaccharomyces,* or *Yarrowia* cell, such as a *Kluyveromyces lactis, Saccharomyces carlsbergensis, Saccharomyces cerevisiae, Saccharomyces diastaticus, Saccharomyces douglasii, Saccharomyces kluyveri, Saccharomyces norbensis, Saccharomyces oviformis,* or *Yarrowia lipolytica* cell,
or derived from or endogenous to a filamentous fungal recombinant host cell, *e.g.,* an *Acremonium, Aspergillus, Aureobasidium, Bjerkandera, Ceriporiopsis, Chrysosporium, Coprinus, Coriolus, Cryptococcus, Filibasidium, Fusar-*

*ium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes,* or *Trichoderma* cell, in particular, an *Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Talaromyces emersonii, Thielavia terrestris, Trametes villosa, Trametes versicolor, Trichoderma harzianum, Trichoderma koningii, Trichoderma longibrachiatum, Trichoderma reesei,* or *Trichoderma viride* cell.

111. The fusion polypeptide according to any of paragraphs 97 to 104, wherein the second donor polypeptide is derived from or endogenous to a *Pichia pastoris,* a *Trichoderma reesei,* a *Aspergillus niger,* or a *Aspergillus oryzae* cell.

111a. The fusion polypeptide according to any of paragraphs 97 to 104, wherein the second donor polypeptide is derived from or endogenous to *Thermothielavioides terrestris.*

111b. The fusion polypeptide according to any of paragraphs 97 to 104, wherein the second donor polypeptide is derived from or endogenous to *Thermomyces lanuginosus.*

112. The fusion polypeptide according to any of paragraphs 97 to 111, wherein the SP-linker is a synthetic polypeptide.

113. The fusion polypeptide according to any of paragraphs 97 to 112, wherein the SP-linker consists of two amino acids, three amino acids, four amino acids, 5 amino acids, 6 amino acids, 7 amino acids, 8 amino acids, 9 amino acids, 10 amino acids, 11 amino acids, 12 amino acids, 13 amino acids, 14 amino acids, or 15 amino acids.

114. The fusion polypeptide according to any of paragraphs 97 to 113, wherein the SP-linker consists of 2 - 10 amino acids.

115. The fusion polypeptide according to any of paragraphs 97 to 114, wherein the polypeptide of interest is selected from the list of a hydrolase, isomerase, ligase, lyase, lysozyme, oxidoreductase, or transferase, e.g., an aminopeptidase, amylase, carbohydrase, carboxypeptidase, catalase, cellobiohydrolase, cellulase, chitinase, cutinase, cyclodextrin glycosyltransferase, deoxyribonuclease, endoglucanase, esterase, alpha-galactosidase, beta-galactosidase, glucoamylase, alpha-glucosidase, beta-glucosidase, invertase, laccase, lipase, mannosidase, mutanase, nuclease, oxidase, pectinolytic enzyme, peroxidase, phosphodiesterase, phytase, polyphenoloxidase, proteolytic enzyme, ribonuclease, transglutaminase, xylanase, or beta-xylosidase.

116. The fusion polypeptide according to any of paragraphs 97 to 115, wherein the SP-linker is present after maturation of the polypeptide of interest and/or the fusion polypeptide.

117. The fusion polypeptide according to any of paragraphs 97 to 116, wherein the polypeptide of interest comprises a pro-peptide.

118. The fusion polypeptide according to paragraph 117, wherein the pro-peptide is located at the N-terminal end of the amino acid sequence of the polypeptide of interest, and the SP-linker is located at the N-terminal end of the pro-peptide.

119. The fusion polypeptide according to any of paragraphs 97 to 118, wherein the polypeptide comprises an additional cleavage site located between the SP-linker and the polypeptide of interest, or between the SP-linker and the pro-peptide of the polypeptide of interest.

120. The fusion polypeptide according to paragraph 119, wherein the additional cleavage site is a proteolytic cleavage site.

121. The fusion polypeptide according to any of paragraphs 119 to 120, wherein the additional cleavage site is recognized by a suitable protease, preferably a KexB protease.

122. The fusion polypeptide according to paragraph 121, wherein the additional cleavage site is a dibasic amino acid motif such as a Lys-Arg ("KR") or an Arg-Arg motif ("RR"), preferably the additional cleavage site is a KexB or Kex2 cleavage site.

123. The fusion polypeptide according to any of paragraphs 97 to 122, wherein the SP-linker is removed from the polypeptide of interest, preferably removed from the polypeptide of interest together with the pro-peptide.

124. The fusion polypeptide according to any of paragraphs 97 to 123, wherein the SP-linker is selected from the group consisting of:

(a) a polypeptide having at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% sequence identity to the sequence of SEQ ID NO: 7 or 8, or "T", "TG", "TGA", or SEQ ID NO: 24 to 48, or "A", "AE", AEE", or SEQ ID NO: 52 to 76,or "S", "SH", "SHD", or SEQ ID NO: 83 to 97, "T", "TP", "TPQ", or SEQ ID NO 101 to 126, or SEQ ID NO: 179 to 186, or "FTP, "FPA", "AS", or "ASG", or SEQ ID NO: 79;
(b) a polypeptide encoded by a polynucleotide having at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% sequence identity to the polypeptide coding sequence of SEQ ID NO: 5, SEQ ID NO: 6, SEQ ID NO: 175, or SEQ ID NO: 176;
(c) a polypeptide derived from a polypeptide sequence of SEQ ID NO: 7 or 8, or "T", "TG", "TGA", or SEQ ID NO: 24 to 48, or "A", "AE", AEE", or SEQ ID NO: 52 to 76,or "S", "SH", "SHD", or SEQ ID NO: 83 to 97, or "T", "TP", "TPQ", or SEQ ID NO 101 to 126, or SEQ ID NO: 179 to 186, or "FTP, "FPA", "AS", or "ASG", or SEQ ID NO: 79,
or
SEQ ID NO: 7 or 8, or "T", "TG", "TGA", or SEQ ID NO: 24 to 48, or "A", "AE", AEE", or SEQ ID NO: 52 to 76,or "S", "SH", "SHD", or SEQ ID NO: 83 to 97, or "T", "TP", "TPQ", or SEQ ID NO 101 to 126, or SEQ ID NO: 179 to 186, or "FTP, "FPA", "AS", or "ASG", or SEQ ID NO: 79 having 1-30 alterations, *e.g.,* substitutions, deletions and/or insertions at one or more positions, *e.g.,* 1 or 2 or 3 or 4 or 5 or 6 or 7 or 8 or 9 or 10 or 11 or 12 or 13 or 14 or 15 or 16 or 17 or 18 or 19 or 20 or 21 or 22 or 23 or 24 or 25 or 26 or 27 or 28 or 29 or 30 alterations, in particular substitutions;
(d) a polypeptide derived from the polypeptide of (a), (b), or (c), wherein the N- and/or C-terminal end of the polypeptide fragment has been extended by addition of one or more amino acids; and
(e) a fragment of the polypeptide of (a), (b), (c), or (d).

125. The fusion polypeptide according to any of paragraphs 97 to 124, wherein the fusion polypeptide is selected from the group consisting of:

(a) a polypeptide having at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% sequence identity to SEQ ID NO: 14;
(b) a polypeptide having at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% sequence identity to SEQ ID NO: 15;
(c) a polypeptide encoded by a polynucleotide having at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% sequence identity to the mature polypeptide coding sequence of SEQ ID NO: 77-78; and
(d) a polypeptide derived from the polypeptide of (a), (b), or (c), wherein the N- and/or C-terminal end has been extended by addition of one or more amino acids, said polypeptide having amylase activity; and
(e) a fragment of the polypeptide of (a), (b), or (c);

wherein the polypeptide has amylase activity.

126. The fusion polypeptide of any one of paragraphs 97 to 125, which is a variant of SEQ ID NO: 14 or 15, a variant of a mature polypeptide of SEQ ID NO: 14 or 15, or a variant of SEQ ID NO: 14 or 15 comprising a substitution, deletion, and/or insertion at one or more positions.

127. The fusion polypeptide of any one of paragraphs 97 to 126, wherein the SP-linker comprises an N-terminal extension and/or C-terminal extension of 1-10 amino acids, e.g., 1, 2, 3, 4, 5, 6, 7, 8, 9, or 10 amino acids, preferably and extension of 1-5 amino acid residues in the N- terminus and/or 1-30 amino acids in the C-terminus, such as 1-15.

128. The fusion polypeptide according to paragraph 127, wherein the extended polypeptide has amylase activity.

129. The fusion polypeptide of any of paragraphs 97 to 128, comprising, consisting essentially of, or consisting of SEQ ID NO: 14.

130. The fusion polypeptide or any of paragraphs 97 to 129, comprising, consisting essentially of, or consisting of SEQ ID NO: 15.

130a. The fusion polypeptide according to any of the preceding paragraphs, wherein the fusion polypeptide does not comprise a chymosin.

131. An extended fusion polypeptide comprising the fusion polypeptide of any one of paragraphs 97 to 130 and a second polypeptide of interest.

132. A hybrid polypeptide comprising the fusion polypeptide of any one of paragraphs 97 to 130 or the extended fusion polypeptide of paragraph 131.

133. The fusion polypeptide of any one of paragraphs 97 to 132, which is isolated.

134. The fusion polypeptide of any one of paragraphs 97 to 133, which is purified.

135. The fusion polypeptide of any of paragraphs 97 to 134, wherein sequence identity is determined by Sequence Identity Determination Method 1.

136. The fusion polypeptide according to any of paragraphs 97 to 134, wherein the polypeptide is encoded by a nucleic acid construct according to any of paragraphs 1 to 70, and/or the expression vector according to paragraph 71, and/or wherein the polypeptide is expressed by a host cell according to any of paragraphs 72 to 88.

137. A nucleic acid construct comprising:

    a) a first polynucleotide encoding a signal peptide from a bacterial yckD polypeptide; and
    b) a second polynucleotide encoding a polypeptide of interest;

wherein the first polynucleotide and the second polynucleotide are operably linked in translational fusion.

138. The nucleic acid construct according to paragraph 137, wherein the nucleic acid construct further comprises a heterologous promoter, and wherein said promoter, the first polynucleotide, and the second polynucleotide are operably linked.

139. The nucleic acid construct according to paragraph 138, wherein the promoter is a P3 promoter or a P3-based promoter.

140. The nucleic acid construct according to paragraph 139, wherein the promoter is a P3 promoter or a P3-based promoter, preferably the heterologous promoter is a tandem promoter comprising the P3 promoter or is a tandem promoter derived from the P3 promoter.

141. The nucleic acid construct according to any of paragraphs 138 to 140, wherein the promoter comprises or consists of a nucleic acid sequence having a sequence identity of least 80%, e.g. at least 85%, at least 90%, at least 91%, at least 92%, at least 93%, at least 94%, at least 95%, at least 96%, at least 97%, at least 98%, at least 99%, or 100%, to SEQ ID NO: 20, preferably the promoter comprises, consists essentially of, or consists of SEQ ID NO: 20.

142. The nucleic acid construct according to any of paragraphs 137 to 141, wherein the signal peptide is a naturally occurring signal peptide, or a functional fragment or functional variant of a naturally occurring signal peptide.

143. The nucleic acid construct according to any of paragraphs 137 to 142, wherein the signal peptide is obtained from a yckD polypeptide from a *Bacillus* species selected from the group consisting of *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* and *Bacillus thuringiensis* cells.

144. The nucleic acid construct according to any of paragraphs 137 to 143, wherein the signal peptide is obtained from a yckD polypeptide from *Bacillus licheniformis, Bacillus subtilis* or *Bacillus pumilus.*

145. The nucleic acid construct according to any of paragraphs 137 to 144, wherein the signal peptide is obtained from a yckD polypeptide from *Bacillus pumilus.*

146. The nucleic acid construct according to any of paragraphs 137 to 145, wherein the signal peptide is obtained from a bacterial yckD polypeptide having a sequence identity of at least 80%, e.g. at least 85%, at least 90%, at least 91%, at least 92%, at least 93%, at least 94%, at least 95%, at least 96%, at least 97%, at least 98%, at least 99%, or 100%, to SEQ ID NO: 19, preferably the bacterial yckD polypeptide comprises, consists essentially of, or consists of SEQ ID NO: 19.

147. The nucleic acid construct according to any of paragraphs 137 to 146, wherein the first polynucleotide encoding the signal peptide has a sequence identity of at least 80%, e.g. at least 85%, at least 90%, at least 91%, at least 92%, at least 93%, at least 94%, at least 95%, at least 96%, at least 97%, at least 98%, at least 99%, or 100%, to the mature polypeptide coding sequence of SEQ ID NO:2; most preferably the polynucleotide comprises, consists essentially of, or consists of the mature polypeptide coding sequence of SEQ ID NO: 2.

148. The nucleic acid construct according to any of paragraphs 137 to 147, wherein the signal peptide has a sequence identity of at least 80%, e.g. at least 85%, at least 90%, at least 91%, at least 92%, at least 93%, at least 94%, at least 95%, at least 96%, at least 97%, at least 98%, at least 99%, or 100%, to SEQ ID NO: 4; preferably the signal peptide comprises, consists essentially of, or consists of SEQ ID NO: 4.

148a. A nucleic acid construct comprising:

a) a first polynucleotide encoding a signal peptide from a fungal polyl dipeptidyl peptidase; and
b) a second polynucleotide encoding a polypeptide of interest;

wherein the first polynucleotide and the second polynucleotide are operably linked in translational fusion.

149. The nucleic acid construct according to paragraph 148a, wherein the nucleic acid construct further comprises a heterologous promoter, and wherein said promoter, the first polynucleotide, and the second polynucleotide are operably linked.

150. The nucleic acid construct according to any of paragraphs 148a to 149, wherein the signal peptide is a naturally occurring signal peptide, or a functional fragment or functional variant of a naturally occurring signal peptide.

151. The nucleic acid construct according to any of paragraphs 148a to 150, wherein the signal peptide is obtained from a polyl dipeptidyl peptidase from a *Aspergillus* species selected from the group consisting of *Aspergillus niger* cells and *Aspergillus oryzae* cells.

152. The nucleic acid construct according to any of paragraphs 148a to 151, wherein the signal peptide is obtained from a polyl dipeptidyl peptidase from *Aspergillus oryzae.*

153. The nucleic acid construct according to any of paragraphs 148a to 152, wherein the signal peptide is obtained from a polyl dipeptidyl peptidase having a sequence identity of at least 80%, e.g. at least 85%, at least 90%, at least 91%, at least 92%, at least 93%, at least 94%, at least 95%, at least 96%, at least 97%, at least 98%, at least 99%, or 100%, to SEQ ID NO: 172, preferably the polyl dipeptidyl peptidase comprises, consists essentially of, or consists of SEQ ID NO: 172.

154. The nucleic acid construct according to any of paragraphs 148a to 153, wherein the first polynucleotide encoding the signal peptide has a sequence identity of at least 80%, e.g. at least 85%, at least 90%, at least 91%, at least 92%, at least 93%, at least 94%, at least 95%, at least 96%, at least 97%, at least 98%, at least 99%, or 100%, to the mature polypeptide coding sequence of SEQ ID NO:178; most preferably the polynucleotide comprises, consists essentially of, or consists of the mature polypeptide coding sequence of SEQ ID NO: 178.

155. The nucleic acid construct according to any of paragraphs 137 to 147, wherein the signal peptide has a sequence identity of at least 80%, e.g. at least 85%, at least 90%, at least 91%, at least 92%, at least 93%, at least 94%, at least 95%, at least 96%, at least 97%, at least 98%, at least 99%, or 100%, to SEQ ID NO: 4; preferably the signal peptide comprises, consists essentially of, or consists of SEQ ID NO: 4.

156. The nucleic acid construct according to any of paragraphs 137 to 155, wherein the N- and/or C-terminal end of the signal peptide has been extended by addition of one or more amino acids.

157. The nucleic acid construct according to any of paragraphs 137 to 155, wherein the signal peptide is a fragment of the signal peptides of any of paragraphs 137 to 155.

158. The nucleic acid construct according to any of paragraphs 137 to 157, wherein the polypeptide of interest is selected from the list of a hydrolase, isomerase, ligase, lyase, lysozyme, oxidoreductase, or transferase, e.g. an aminopeptidase, amylase, carbohydrase, carboxypeptidase, catalase, cellobiohydrolase, cellulase, chitinase, cutinase, cyclodextrin glycosyltransferase, deoxyribonuclease, endoglucanase, esterase, alpha-galactosidase, beta-galactosidase, glucoamylase, alpha-glucosidase, beta-glucosidase, invertase, laccase, lipase, mannosidase, mutanase, nuclease, oxidase, pectinolytic enzyme, peroxidase, phosphodiesterase, phytase, polyphenoloxidase, proteolytic enzyme, ribonuclease, transglutaminase, xylanase, beta-xylosidase, or a protease having protease activity, preferably the polypeptide of interest is a microbial polypeptide; preferably a bacterial polypeptide.

159. The nucleic acid construct according to paragraph 158, wherein the polypeptide of interest is a protease obtained from *Bacillus licheniformis, Bacillus subtilis, Nocardiopsis prasina* or *Bacillus clausii*.

160. The nucleic acid construct according to any of paragraphs 137 to 159, wherein the protease is obtained from *Bacillus clausii*.

161. The nucleic acid construct according to any of paragraphs 137 to 158, wherein the polypeptide of interest is a phytase, or an endoglucanase.

162. The nucleic acid construct according to any of paragraphs 137 to 160, wherein the protease has a sequence identity of at least 80%, e.g. at least 85%, at least 90%, at least 91%, at least 92%, at least 93%, at least 94%, at least 95%, at least 96%, at least 97%, at least 98%, at least 99%, or 100%, to the mature polypeptide of SEQ ID NO: 16, preferably the protease comprises, consists essentially of, or consists of the mature polypeptide of SEQ ID NO: 16.

163. The nucleic acid construct according to any of paragraphs 137 to 162, wherein the N- and/or C-terminal end of the polypeptide of interest and/or signal peptide has been extended by addition of one or more amino acids.

164. The nucleic acid construct according to any of paragraphs 137 to 163, wherein the protease or polypeptide of interest is a fragment of the protease or polypeptide of interest according to any of embodiments 137 to 163, respectively.

165. An expression vector comprising a nucleic acid construct according to any of paragraphs 137 to 164.

166. A bacterial host cell producing a polypeptide of interest and comprising in its genome:

a) a nucleic acid construct according to any of paragraphs 137 to 164; and/or

b) an expression vector according to paragraph 165.

167. The bacterial host cell of paragraph 166, wherein the bacterial host cell is a Gram-positive host cell.

168. The bacterial host cell according to any of paragraphs 166 to 167, wherein the bacterial host cell is a *Bacillus* cell.

169. The bacterial host cell according to any of paragraphs 166 to 168, wherein the bacterial host cell is a *Bacillus* cell selected from the group consisting of *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* and *Bacillus thuringiensis* cell.

170. The bacterial host cell according to any of paragraphs 166 to 169, wherein the bacterial host cell is a *Bacillus licheniformis* cell.

171. The bacterial host cell according to any of paragraphs 166 to 170, wherein the host cell comprises at least two copies of the nucleic acid construct and/or the expression vector, such as two copies, three copies, four copies or more than four copies.

172. The host cell according to any of paragraphs 166 to 171, wherein a yield of the polypeptide of interest is increased by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 120%, at least 150%, at least 170%, at least 200%, at least 220%, at least 250%, or at least 270% relative to a yield of the polypeptide of interest expressed by a parent host cell with an alternative signal peptide when cultivated under identical conditions, the parent host cell otherwise being isogenic to the host cell according to any of paragraphs 166 to 171.

173. The host cell of paragraph 172 wherein the alternative signal peptide comprises or consists of an amino acid sequence having a sequence identity of at least 80%, e.g. at least 85%, at least 90%, at least 91%, at least 92%, at least 93%, at least 94%, at least 95%, at least 96%, at least 97%, at least 98%, at least 99%, or 100%, to the amino acid sequence of SEQ ID NO: 81.

174. A method of producing a polypeptide of interest, the method comprising:

a) cultivating a bacterial host cell according to any of paragraphs 166 to 173 under conditions conducive for production of the polypeptide of interest; and optionally
b) recovering the polypeptide of interest.

175. The method according to paragraph 174, wherein a yield of the polypeptide of interest is increased by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 120%, at least 150%, at least 170%, at least 200%, at least 220%, at least 250%, or at least 270% relative to a yield of the polypeptide of interest expressed by a parent host cell with an alternative signal peptide when cultivated under identical conditions, the parent host cell otherwise being isogenic to the host cell according to any of paragraphs 166 to 173.

176. The method of paragraph 175 wherein the alternative signal peptide comprises or consists of an amino acid sequence having a sequence identity of at least 80%, e.g. at least 85%, at least 90%, at least 91%, at least 92%, at least 93%, at least 94%, at least 95%, at least 96%, at least 97%, at least 98%, at least 99%, or 100%, to the amino acid sequence of SEQ ID NO: 81.

176a. A fungal host cell producing a polypeptide of interest and comprising in its genome:

a) a nucleic acid construct according to any of paragraphs 137 to 164; and/or
b) an expression vector according to paragraph 165.

177. The fungal host cell of paragraph 176a, wherein the bacterial host cell is a filamentous fungal host cell.

178. The fungal host cell according to any of paragraphs 176a to 177, wherein the fungal host cell is a *Aspergillus* cell.

179. The fungal host cell according to any of paragraphs 176a to 178, wherein the fungal host cell is a *Aspergillus* cell selected from the group consisting of *Aspergillus niger* and *Aspergillus oryzae.*

180. The fungal host cell according to any of paragraphs 176a to 179, wherein the fungal host cell is a *Aspergillus oryzae* cell.

181. The fungal host cell according to any of paragraphs 176a to 180, wherein the host cell comprises at least two copies of the nucleic acid construct and/or the expression vector, such as two copies, three copies, four copies or more than four copies.

182. The host cell according to any of paragraphs 176a to 181, wherein a yield of the polypeptide of interest is increased by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 120%, at least 150%, at least 170%, at least 200%, at least 220%, at least 250%, or at least 270% relative to a yield of the polypeptide of interest expressed by a parent host cell with an alternative signal peptide when cultivated under identical conditions, the parent host cell otherwise being isogenic to the host cell according to any of paragraphs 176a to 181.

183. A method of producing a polypeptide of interest, the method comprising:

a) cultivating a host cell according to any of paragraphs 166 to 182 under conditions conducive for production of the polypeptide of interest; and optionally
b) recovering the polypeptide of interest.

184. The method according to paragraph 183, wherein a yield of the polypeptide of interest is increased by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 120%, at least 150%, at least 170%, at least 200%, at least 220%, at least 250%, or at least 270% relative to a yield of the polypeptide of interest expressed by a parent host cell with an alternative signal peptide when cultivated under identical conditions, the parent host cell otherwise being isogenic to the host cell according to any of paragraphs 166 to 182.

185. The method of paragraph 184 wherein the alternative signal peptide comprises or consists of an amino acid sequence having a sequence identity of at least 80%, e.g. at least 85%, at least 90%, at least 91%, at least 92%, at least 93%, at least 94%, at least 95%, at least 96%, at least 97%, at least 98%, at least 99%, or 100%, to the amino acid sequence of SEQ ID NO: 81 or SEQ ID NO: 172.

186. A polypeptide, comprising a polypeptide of interest, and a signal peptide derived from a bacterial yckD polypeptide, or a signal peptide derived from a fungal polyl dipeptidyl peptidase.

187. The polypeptide according to paragraph 186, wherein the polypeptide is secreted.

188. The polypeptide according to any of paragraphs 186 to 187, wherein the signal peptide is heterologous to the polypeptide of interest.

189. The polypeptide according to any of paragraphs 186 to 188, wherein the signal peptide is derived from a bacterial cell, *e.g.,* a Gram-positive cell selected from the group consisting of *Bacillus, Clostridium, Enterococcus, Geobacillus, Lactobacillus, Lactococcus, Oceanobacillus, Staphylococcus, Streptococcus,* or *Streptomyces* cells, or a Gram-negative bacteria selected from the group consisting of *Campylobacter, E. coli, Flavobacterium, Fusobacterium, Helicobacter, Ilyobacter, Neisseria, Pseudomonas, Salmonella,* and *Ureaplasma* cells, such as *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis, Bacillus thuringiensis, Streptococcus equisimilis, Streptococcus pyogenes, Streptococcus uberis,* and *Streptococcus equi* subsp. *Zooepidemicus, Streptomyces achromogenes, Streptomyces avermitilis, Streptomyces coelicolor, Streptomyces griseus,* and *Streptomyces lividans* cells.

190. The polypeptide according to any of paragraphs 186 to 189, wherein the signal peptide is derived from a *Bacillus* cell, such as from a *Bacillus subtilis* cell, a *Bacillus licheniformis* cell, or a *Bacillus pumilus* cell.

190a. The polypeptide according to any of paragraphs 186 to 189, wherein the signal peptide is derived from an *Aspergillus* cell, such as an *Aspergillus niger* cell, or an *Aspergillus oryzae* cell.

191. The polypeptide according to any of paragraphs 186 to 190, wherein signal peptide is derived from a first donor polypeptide comprising or consisting of an amino acid sequence having at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% sequence identity to the amino acid sequence of SEQ ID NO: 19.

192. The polypeptide according to any of paragraphs 186 to 191, wherein the polypeptide of interest comprises a pro-peptide.

193. The polypeptide according to any of paragraphs 186 to 191, wherein the pro-peptide is located at the N-terminal end of the amino acid sequence of the polypeptide of interest, and the signal peptide is located at the N-terminal end of the pro-peptide.

194. The polypeptide according to any of paragraphs 186 to 193, wherein the signal peptide is removed from the polypeptide of interest, preferably removed from the polypeptide of interest together with the pro-peptide.

195. The polypeptide according to any of paragraphs 186 to 194, wherein the polypeptide of interest is selected from the list of a hydrolase, isomerase, ligase, lyase, lysozyme, oxidoreductase, or transferase, e.g., an aminopeptidase, amylase, carbohydrase, carboxypeptidase, catalase, cellobiohydrolase, cellulase, chitinase, cutinase, cyclodextrin glycosyltransferase, deoxyribonuclease, endoglucanase, esterase, alpha-galactosidase, beta-galactosidase, glucoamylase, alpha-glucosidase, beta-glucosidase, invertase, laccase, lipase, mannosidase, mutanase, nuclease, oxidase, pectinolytic enzyme, peroxidase, phosphodiesterase, phytase, polyphenoloxidase, proteolytic enzyme, ribonuclease, transglutaminase, xylanase, beta-xylosidase, or a protease having protease activity, preferably the polypeptide of interest comprises a protease comprising or consisting of an amino acid sequence having at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% sequence identity to the amino acid sequence of SEQ ID NO: 16.

196. The polypeptide according to any of paragraphs 186 to 195, wherein the signal peptide is selected from the group consisting of:

(a) a polypeptide having at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% sequence identity to the sequence of SEQ ID NO: 4 or SEQ ID NO: 172;
(b) a polypeptide encoded by a polynucleotide having at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% sequence identity to the polypeptide coding sequence of SEQ ID NO: 2 or SEQ ID NO: 178;
(c) a polypeptide derived from SEQ ID NO: 4 or SEQ ID NO: 172 having 1-30 alterations, *e.g.,* substitutions, deletions and/or insertions at one or more positions, *e.g.,* 1 or 2 or 3 or 4 or 5 or 6 or 7 or 8 or 9 or 10 or 11 or 12 or 13 or 14 or 15 or 16 or 17 or 18 or 19 or 20 or 21 or 22 or 23 or 24 or 25 or 26 or 27 alterations, in particular substitutions;
(d) a polypeptide derived from the polypeptide of (a), (b), or (c), wherein the N- and/or C-terminal end of the polypeptide fragment has been extended by addition of one or more amino acids; and
(e) a fragment of the polypeptide of (a), (b), (c), or (d).

197. The polypeptide according to any of paragraphs 186 to 196, wherein the polypeptide is selected from the group consisting of:

(a) a polypeptide having at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% sequence identity to SEQ ID NO: 80;
(b) a polypeptide derived from the polypeptide of (a), the N- and/or C-terminal end has been extended by addition of one or more amino acids, said polypeptide having protease activity; and
(c) a fragment of the polypeptide of (a), or (b);

wherein the polypeptide has protease activity.

198. The polypeptide of any one of paragraphs 186 to 197, which is a variant of SEQ ID NO: 80 or 16, a variant of a mature polypeptide of SEQ ID NO: 80 or 16, or a variant of SEQ ID NO: 80 or 16 comprising a substitution, deletion, and/or insertion at one or more positions.

199. The polypeptide of any one of paragraphs 186 to 198, wherein the signal peptide comprises an N-terminal extension and/or C-terminal extension of 1-10 amino acids, e.g., 1, 2, 3, 4, 5, 6, 7, 8, 9, or 10 amino acids, preferably and extension of 1-5 amino acid residues in the N- terminus and/or 1-30 amino acids in the C-terminus, such as 1-15.

200. The polypeptide according to paragraph 199, wherein the extended polypeptide has protease activity, phytase activity, or endoglucanase activity.

201. The polypeptide of any of paragraphs 186 to 200, comprising, consisting essentially of, or consisting of SEQ ID NO: 80, or SEQ ID NO: 16.

202. A fusion polypeptide comprising the polypeptide of any one of paragraphs 186 to 201 and a second polypeptide of interest.

203. A hybrid polypeptide comprising the polypeptide of any one of paragraphs 186 to 202.

204. The polypeptide of any one of paragraphs 186 to 203, which is isolated.

205. The polypeptide of any one of paragraphs 186 to 204, which is purified.

206. The polypeptide of any of paragraphs 186 to 205, wherein sequence identity is determined by Sequence Identity Determination Method 1.

207. The polypeptide according to any of paragraphs 186 to 206, wherein the polypeptide is encoded by a nucleic acid construct according to any of paragraphs 137 to 164, and/or the expression vector according to paragraph 165, and/or wherein the polypeptide is expressed by a host cell according to any of paragraphs 166 to 182.

## Claims

1. A nucleic acid construct comprising or consisting of:

   a) a first polynucleotide encoding a signal peptide (SP),
   b) a second polynucleotide located downstream of the first polynucleotide and encoding a SP-linker consisting of 2 to 15 amino acids, wherein the SP-linker amino acid sequence consists of the N-terminal amino acid sequence of a second donor polypeptide; and
   c) at least one third polynucleotide located downstream of the second polynucleotide and encoding a polypeptide of interest;
   wherein the first polynucleotide and the second polynucleotide are heterologous to the third polynucleotide, and wherein the first polynucleotide, the second polynucleotide, and the third polynucleotide are operably linked in translational fusion.

2. The nucleic acid construct according to claim 1, wherein the SP-linker consists of 2 to 10 amino acids, preferably consists of 2 to 9 amino acids, 2 to 8 amino acids, 3 to 8 amino acids, 3 to 9 amino acids, 2 to 7 amino acids, 3 to 7 amino acids, 2 to 6 amino acids, or 3 to 6 amino acids, 4 to 10 amino acids, 5 to 10 amino acids, 4 to 9 amino acids, 5 to 9 amino acids, 4 to 8 amino acids, or 5 to 8 amino acids.

3. The nucleic acid construct according to any of claims 1 to 2, wherein the first polynucleotide encoding the signal peptide is endogenous to the second polynucleotide encoding the SP-linker, and/or endogenous to the polynucleotide encoding the second donor polypeptide.

4. The nucleic acid construct according to any preceding claim, wherein the second donor polypeptide is selected from the list consisting of a structural polypeptide, a receptor, a secreted polypeptide, a hormone, or a secreted enzyme, such as a hydrolase, isomerase, ligase, lyase, lysozyme, oxidoreductase, or transferase, e.g., an aminopeptidase, amylase, carbohydrase, carboxypeptidase, catalase, cellobiohydrolase, cellulase, chitinase, cutinase, cyclodextrin glycosyltransferase, deoxyribonuclease, endoglucanase, esterase, alpha-galactosidase, beta-galactosidase, glucoamylase, alpha-glucosidase, beta-glucosidase, invertase, laccase, lipase, mannosidase, mutanase, nuclease, oxidase, pectinolytic enzyme, peroxidase, phosphodiesterase, phytase, polyphenoloxidase, proteolytic enzyme, ribonuclease, transglutaminase, xylanase, aspartate phosphatase, or beta-xylosidase.

5. The nucleic acid construct according to any preceding claim, wherein the SP-linker comprises or consists of an amino acid sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the amino acid sequence of SEQ ID NO: 24 - 48, SEQ ID NO: 52 - 76, SEQ ID NO: 79, SEQ ID NO: 83 - 97, SEQ ID NO: 101- 126, or SEQ ID NO: 179 - 186.

6. The nucleic acid construct according to any preceding claim, wherein the first and the second polynucleotides encode a SP - SP-linker construct comprising or consisting of an amino acid sequence having a sequence identity of at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% or 100% to the amino acid sequence of SEQ ID NO: 11, SEQ ID NO: 12, SEQ ID NO: 153 - 167, SEQ ID NO: 170, SEQ ID NO: 173, or SEQ ID NO: 174.

7. An expression vector comprising a nucleic acid construct according to any of claims 1 to 6.

8. A host cell producing a polypeptide of interest and comprising in its genome:

   a) a nucleic acid construct according to any of claims 1 to 6; and/or
   b) an expression vector according to claim 7.

9. The host cell according to claim 8, wherein the host cell is a bacterial host cell or a eukaryotic host cell, such as a fungal host cell, a mammalian host cell, or a plant cell, a *Bacillus* host cell, a *Trichoderma* host cell, an *Aspergillus* host cell, a *Pichia* host cell, or a *Saccharomyces* host cell.

10. A method of producing a polypeptide of interest, the method comprising:

    a) cultivating a host cell according to any of claims 8 to 9 under conditions conducive for production of the polypeptide of interest; and optionally
    b) recovering the polypeptide of interest.

11. The method according to claim 10, wherein a yield of the polypeptide of interest is increased by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 100% relative to a yield of the polypeptide of interest expressed by a parent host cell lacking the second polynucleotide encoding the SP-linker when cultivated under identical conditions, the parent host cell otherwise being isogenic to the host cell according to any of claims 8 to 9.

12. A method for increasing secretion of a polypeptide of interest by a host cell, the method comprising the steps of:

    a) **providing** a plurality of first polynucleotide sequences, each first polynucleotide encoding a signal peptide;
    b) **expressing** a first polypeptide with a host cell, wherein the polynucleotide encoding the first polypeptide is linked in translational fusion with a first polynucleotide;
    c) **measuring** the amount of first polypeptide for each first polynucleotide sequence;
    d) **ranking** each first polynucleotide based on the amount of secreted first polypeptide;
    e) **selecting** one or more of the ranked first polynucleotides;
    f) **fusing** the selected one or more first polynucleotide with a second polynucleotide encoding a SP-linker comprising or consisting of 2-15 N-terminal amino acids from the secreted first polypeptide expressed in step b), wherein the second polynucleotide is located downstream of the first polynucleotide, and
    g) **expressing,** in a host cell, the polypeptide of interest in translational fusion with the fusion construct generated in step f), wherein the SP-linker is located upstream of the polypeptide of interest.

**13.** A fusion polypeptide, comprising a polypeptide of interest and a SP-linker consisting of 2 to 15 amino acids, wherein the SP-linker is located at the N-terminal end of the polypeptide of interest and is heterologous to the polypeptide of interest.

**14.** The fusion polypeptide of claim 14, wherein the SP-linker consists of the N-terminal sequence of a second donor polypeptide.

**15.** The fusion polypeptide according to any of claims 13 to 14, wherein the SP-linker consists of 2 to 10 amino acids, preferably consists of 2 to 9 amino acids, 3 to 9 amino acids, 2 to 8 amino acids, 3 to 8 amino acids, 2 to 7 amino acids, 3 to 7 amino acids, 2 to 6 amino acids, or 3 to 6 amino acids, 4 to 10 amino acids, 5 to 10 amino acids, 4 to 9 amino acids, 5 to 9 amino acids, 4 to 8 amino acids, or 5 to 8 amino acids.

**16.** The fusion polypeptide according to any of claims 13 to 15, wherein the second donor polypeptide is selected from the list consisting of a structural polypeptide, a receptor, a secreted polypeptide, a hormone, or a secreted enzyme, such as a hydrolase, isomerase, ligase, lyase, lysozyme, oxidoreductase, or transferase, e.g., an aminopeptidase, amylase, carbohydrase, carboxypeptidase, catalase, cellobiohydrolase, cellulase, chitinase, cutinase, cyclodextrin glycosyltransferase, deoxyribonuclease, endoglucanase, esterase, alpha-galactosidase, beta-galactosidase, glucoamylase, alpha-glucosidase, beta-glucosidase, invertase, laccase, lipase, mannosidase, mutanase, nuclease, oxidase, pectinolytic enzyme, peroxidase, phosphodiesterase, phytase, polyphenoloxidase, proteolytic enzyme, ribonuclease, transglutaminase, xylanase, aspartate phosphatase, or beta-xylosidase.

**17.** The fusion polypeptide according to any of claims 13 to 16, wherein the polypeptide of interest is selected from the list of a hydrolase, isomerase, ligase, lyase, lysozyme, oxidoreductase, or transferase, e.g., an aminopeptidase, amylase, carbohydrase, carboxypeptidase, catalase, cellobiohydrolase, cellulase, chitinase, cutinase, cyclodextrin glycosyltransferase, deoxyribonuclease, endoglucanase, esterase, alpha-galactosidase, beta-galactosidase, glucoamylase, alpha-glucosidase, beta-glucosidase, invertase, laccase, lipase, mannosidase, mutanase, nuclease, oxidase, pectinolytic enzyme, peroxidase, phosphodiesterase, phytase, polyphenoloxidase, proteolytic enzyme, ribonuclease, transglutaminase, xylanase, or beta-xylosidase.

**Figure 1**

**Figure 2**

EP 4 273 249 A2

Figure 3

Figure 4

Figure 5

Figure 6

BT11146    SP | Termamyl

BT11211    SP | Termamyl

BT11212    SP | 7AA | Termamyl

SP — Std Amylase SP

SP — yckD SP (*B. pumilus*)

7AA — 7 amino acids from the Protease 2 linker

+8%

+39%

Time (hr)

**Figure 7**

**Figure 8**

EP 4 273 249 A2

## Construct ⎯⎯⎯⎯⎯⎯ Strain name

| Construct | Strain name |
|---|---|
| SP (*A. oryzae* tglA) — Phytase | AT6552 |
| SP-linker<br>SP (*A. oryzae* tglA) — 7aa from xylanase (*T. lanuginosus*) N-term — Phytase | AT6502 |
| SP (*A. oryzae* peptidase) — Phytase | AT6553 |
| SP-linker<br>SP (*A. oryzae* peptidase) — 7aa from endoglucanase (*T. terrestris*) N-term — Phytase | AT6503 |

**Figure 9**

Figure 10

EP 4 273 249 A2

**Figure 11**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9425612 A
- WO 9533836 A
- EP 238023 A
- WO 9114772 A
- US 6395966 B
- US 7151204 B
- WO 9517413 A
- WO 9522625 A
- US 5223409 A

- WO 9206204 A
- US 20190185847 A1
- WO 2018077796 A1
- WO 2013119302 A
- WO 9943835 A

**Non-patent literature cited in the description**

- **MARTIN et al.** *J. Ind. Microbiol. Biotechnol.,* 2003, vol. 3, 568-576
- **SVETINA et al.** *J. Biotechnol.,* 2000, vol. 76, 245-251
- **RASMUSSEN-WILSON et al.** *Appl. Environ. Microbiol.,* 1997, vol. 63, 3488-3493
- **WARD et al.** *Biotechnology,* 1995, vol. 13, 498-503
- **CONTRERAS et al.** *Biotechnology,* 1991, vol. 9, 378-381
- **EATON et al.** *Biochemistry,* 1986, vol. 25, 505-512
- **COLLINS-RACIE et al.** *Biotechnology,* 1995, vol. 13, 982-987
- **CARTER et al.** *Proteins: Structure, Function, and Genetics,* 1989, vol. 6, 240-248
- **STEVENS.** *Drug Discovery World,* 2003, vol. 4, 35-48
- **COOPER et al.** *EMBO J.,* 1993, vol. 12, 2575-2583
- **DAWSON et al.** *Science,* 1994, vol. 266, 776-779
- **NEEDLEMAN ; WUNSCH.** *J. Mol. Biol.,* 1970, vol. 48, 443-453
- **RICE et al.** EMBOSS: The European Molecular Biology Open Software Suite. *Trends Genet.,* 2000, vol. 16, 276-277
- **RICE et al.** *EMBOSS: The European Molecular Biology Open Software Suite,* 2000
- **RABOUILLE.** *Trends in Cell Biology,* 2017, vol. 27, 230-240
- **KIM et al.** *Journal of Cell Science,* 2018, vol. 131, jcs213686
- **CAI D. et al.** *Microb Cell Fact,* 2017, vol. 16, 70, https://doi.org/10.1186/s12934-017-0688-7

- **CAI D. et al.** *Journal of Appl Microbiology,* 2016, vol. 121 (3), 704-712
- **FORD et al.** *Protein Expression and Purification,* 1991, vol. 2, 95-107
- **SAMBROOK et al.** Molecular Cloning: A Laboratory Manual. Cold Spring Harbor Lab, 1989
- **SONG et al.** *PLOS One,* 2016, vol. 11 (7), e0158447
- **MUKHERJEE et al.** *Trichoderma: Biology and Applications,* 2013
- **SCHMOLL ; DATTENBÖCK.** Gene Expression Systems in Fungi: Advancements and Applications. *Fungal Biology,* 2016
- Synthetic Biology: Parts, Devices and Applications. **SMOLKE et al.** Constitutive and Regulated Promoters in Yeast: How to Design and Make Use of Promoters in S. cerevisiae. 2018
- *Nature,* 2014, vol. 507, 462-470, https://doi.org/10.1038/nature13182
- **ROMANOVA N ; NOLL T.** *Biotechnol J.,* 07 December 2017, vol. 13 (3), e1700232
- **ROMANOS et al.** *Yeast,* 1992, vol. 8, 423-488
- **HUE et al.** *J. Bacteriol.,* 1995, vol. 177, 3465-3471
- **GEISBERG et al.** *Cell,* 2014, vol. 156 (4), 812-824
- **MOROZOV et al.** *Eukaryotic Cell,* 2006, vol. 5 (11), 1838-1846
- **HAMBRAEUS et al.** *Microbiology,* 2000, vol. 146 (12), 3051-3059
- **KABERDIN ; BLÄSI.** *FEMS Microbiol. Rev.,* 2006, vol. 30 (6), 967-979
- **GUO ; SHERMAN.** *Mol. Cellular Biol.,* 1995, vol. 15, 5983-5990
- **FREUDL.** *Microbial Cell Factories,* 2018, vol. 17, 52

- **XU et al.** *Biotechnology Letters,* 2018, vol. 40, 949-955
- **KOBER, LARS ; ZEHE, CHRISTOPH ; BODE, JUERGEN.** *Biotechnology and bioengineering,* 2013
- **ATTALLAH, CAROLINA et al.** *Protein Expression and Purification,* 2017, vol. 132, 27-33
- **STERN, BEATE et al.** *Trends Cell Mol Biol,* 2007, vol. 2, 1-17
- **PATEL ; GUPTA.** *Int. J. Syst. Evol. Microbiol.,* 2020, vol. 70, 406-438
- **HEINZE et al.** *BMC Microbiology,* 2018, vol. 18, 56
- **BURKE et al.** *Proc. Natl. Acad. Sci. USA,* 2001, vol. 98, 6289-6294
- **CHOI et al.** *J. Microbiol. Methods,* 2006, vol. 64, 391-397
- **DONALD et al.** *J. Bacteriol.,* 2013, vol. 195 (11), 2612-2620
- **MADIHA et al.** *Biotechnology and bioengineering,* 2004, vol. 87 (4), 537-545
- **PHAM, PHUONG LAN ; KAMEN, AMINE ; DUROCHER, YVES.** *Molecular biotechnology,* 2006, vol. 34 (2), 225-237
- Ainsworth and Bisby's Dictionary of The Fungi. **HAWKSWORTH et al.** CAB International. University Press, 1995
- **LI et al.** *Microbial Cell Factories,* 2017, vol. 16, 168
- **YELTON et al.** *Proc. Natl. Acad. Sci. USA,* 1984, vol. 81, 1470-1474
- **CHRISTENSEN et al.** *Bio/Technology,* 1988, vol. 6, 1419-1422
- **LUBERTOZZI ; KEASLING.** *Biotechn. Advances,* 2009, vol. 27, 53-75
- Biology and Activities of Yeast. Soc. App. Bacteriol. Symposium Series. 1980
- **WINGFIELD.** *Current Protocols in Protein Science,* 2015, vol. 80 (1), 6.1.1-6.1.35
- **LABROU.** *Protein Downstream Processing,* 2014, vol. 1129, 3-10
- **STICKLEN.** *Nature Reviews,* 2008, vol. 9, 433-443
- **TAGUE et al.** *Plant Physiology,* 1988, vol. 86, 506
- **FRANCK et al.** *Cell,* 1980, vol. 21, 285-294
- **CHRISTENSEN et al.** *Plant Mol. Biol.,* 1992, vol. 18, 675-689
- **ZHANG et al.** *Plant Cell,* 1991, vol. 3, 1155-1165
- **EDWARDS ; CORUZZI.** *Ann. Rev. Genet.,* 1990, vol. 24, 275-303
- **ITO et al.** *Plant Mol. Biol.,* 1994, vol. 24, 863-878
- **WU et al.** *Plant Cell Physiol.,* 1998, vol. 39, 885-889
- **CONRAD et al.** *J. Plant Physiol.,* 1998, vol. 152, 708-711
- **CHEN et al.** *Plant Cell Physiol.,* 1998, vol. 39, 935-941
- **KYOZUKA et al.** *Plant Physiol.,* 1993, vol. 102, 991-1000
- **MITRA ; HIGGINS.** *Plant Mol. Biol.,* 1994, vol. 26, 85-93
- **KAGAYA et al.** *Mol. Gen. Genet.,* 1995, vol. 248, 668-674
- **XU et al.** *Plant Mol. Biol.,* 1993, vol. 22, 573-588
- **GASSER et al.** *Science,* 1990, vol. 244, 1293
- **POTRYKUS.** *BiolTechnology,* 1990, vol. 8, 535
- **SHIMAMOTO et al.** *Nature,* 1989, vol. 338, 274
- **HOOYKAS ; SCHILPEROORT.** *Plant Mol. Biol.,* 1992, vol. 19, 15-38
- **CHRISTOU.** *Plant J.,* 1992, vol. 2, 275-281
- **SHIMAMOTO.** *Curr. Opin. Biotechnol.,* 1994, vol. 5, 158-162
- **VASIL et al.** *Bio/Technology,* 1992, vol. 10, 667-674
- **OMIRULLEH et al.** *Plant Mol. Biol.,* 1993, vol. 21, 415-428
- **CUNNINGHAM ; WELLS.** *Science,* 1989, vol. 244, 1081-1085
- **HILTON et al.** *J. Biol. Chem.,* 1996, vol. 271, 4699-4708
- **DE VOS et al.** *Science,* 1992, vol. 255, 306-312
- **SMITH et al.** *J. Mol. Biol.,* 1992, vol. 224, 899-904
- **WLODAVER et al.** *FEBS Lett.,* 1992, vol. 309, 59-64
- **JUMPER et al.** Highly accurate protein structure prediction with AlphaFold. *Nature,* 2021, vol. 596, 583-589
- **REIDHAAR-OLSON ; SAUER.** *Science,* 1988, vol. 241, 53-57
- **BOWIE ; SAUER.** *Proc. Natl. Acad. Sci. USA,* 1989, vol. 86, 2152-2156
- **LOWMAN et al.** *Biochemistry,* 1991, vol. 30, 10832-10837
- **DERBYSHIRE et al.** *Gene,* 1986, vol. 46, 145
- **NER et al.** *DNA,* 1988, vol. 7, 127
- **NESS et al.** *Nature Biotechnology,* 1999, vol. 17, 893-896
- **DAVIS et al.** Basic Methods in Molecular Biology. Elsevier, 2012
- **SAMBROOK et al.** Molecular cloning: A laboratory manual. Cold Spring Harbor laboratory, 1989
- Current pro7tocols in Molecular Biology. John Wiley and Sons, 1995
- Molecular Biological Methods for Bacillus. John Wiley and Sons, 1990
- **YOU, C et al.** *Methods Mol. Biol.,* 2017, vol. 116, 183-92